# EUROPEAN PATENT SPECIFICATION

(11) **EP 1 675 957 B1**
(45) Date of publication and mention of the grant of the patent: **07.12.2011**
(21) Application number: 04795252.8
(22) Date of filing: 15.10.2004
(51) Int. Cl.: C12N 15/82, A01H 5/12, C12N 9/04

(54) **CLONING OF CYTOCHROME P450 GENES FROM NICOTIANA**
KLONIERUNG DES CYTOCHROME P450 GENS VON NICOTIANA
CLONAGE DES GENES CYTOCHROMES P450 A PARTIR DE NICOTIANA

(30) Priority: 16.10.2003 US 686947; 29.04.2004 US 566235 P; 03.09.2004 US 934944
(43) Date of publication of application: 05.07.2006
(73) Proprietor: U.S. Smokeless Tobacco Company LLC, Richmond, VA 23230 (US)
(72) Inventor: XU, Dongmei, Winchester, KY 40391 (US)
(74) Representative: Wichmann, Hendrik
(86) International application number: PCT/US2004/034065
(87) International publication number: WO 2005/038033

## Description

The present invention relates to nucleic acid sequences encoding cytochrome p450 enzymes (hereinafter referred to as p450 and p450 enzymes) in *Nicotiana* plants and methods for using those nucleic acid sequences to alter plant phenotypes.

### BACKGROUND

Cytochrome p450s catalyze enzymatic reactions for a diverse range of chemically dissimilar substrates that include the oxidative, peroxidative and reductive metabolism of endogenous and xenobiotic substrates. In plants, p450s participate in biochemical pathways that include the synthesis of plant products such as phenylpropanoids, alkaloids, terpenoids, lipids, cyanogenic glycosides, and glucosinolates (Chappel, Annu. Rev. Plant Physiol. Plant Mol. Biol. 198, 49:311-343). Cytochrome p450s, also known as p450 hemethiolate proteins, usually act as terminal oxidases in multicomponent electron transfer chains, called p450- containing monooxygenase systems. Specific reactions catalyzed include demethylation, hydroxylation, epoxidation, N-oxidation, sulfooxidation, N-, S-, and 0- dealkylations, desulfation, deamination, and reduction of azo, nitro, and N-oxide groups.

The diverse role of *Nicotiana* plant p450 enzymes has been implicated in effecting a variety of plant metabolites such as phenylpropanoids, alkaloids, terpenoids, lipids, cyanogenic glycosides, glucosinolates and a host of other chemical entities. During recent years, it is becoming apparent that some p450 enzymes can impact the composition of plant metabolites in plants. For example, it has been long desired to improve the flavor and aroma of certain plants by altering its profile of selected fatty acids through breeding; however very little is known about mechanisms involved in controlling the levels of these leaf constituents. The down regulation of p450 enzymes associated with the modification of fatty acids may facilitate accumulation of desired fatty acids that provide more preferred leaf phenotypic qualities. The function of p450 enzymes and their broadening roles in plant constituents is still being discovered. For instance, a special class of p450 enzymes was found to catalyze the breakdown of fatty acid into volatile C6- and C9-aldehydes and -alcohols that are major contributors of "fresh green" odor of fruits and vegetables. The level of other novel targeted p450s may be altered to enhance the qualities of leaf constituents by modifying lipid composition and related break down metabolites in Nicotiana leaf. Several of these constituents in leaf are affected by senescence that stimulates the maturation of leaf quality properties. Still other reports have shown that p450s enzymes are play a functional role in altering fatty acids that are involved in plant-pathogen interactions and disease resistance.

In other instances, p450 enzymes have been suggested to be involved in alkaloid biosynthesis. Nornicotine is a minor alkaloid found in *Nicotiana tabaceum.* It has been postulated that it is produced by the p450 mediated demethylation of nicotine followed by acylation and nitrosation at the N position thereby producing a series of N-acylnonicotines and N-nitrosonornicotines. N-demethylation, catalyzed by a putative p450 demethylase, is thought to be a primary source of nornicotine biosyntheses in *Nicotiana*. While the enzyme is believed to be microsomal, thus far a nicotine demethylase enzyme has not been successfully purified, nor have the genes involved been isolated.

Furthermore, it is hypothesized but not proven that the activity of p450 enzymes is genetically controlled and also strongly influenced by environment factors. For example, the demethylation of nicotine in *Nicotiana* is thought to increase substantially when the plants reach a mature stage. Furthermore, it is hypothesized yet not proven that the demethylase gene contains a transposable element that can inhibit translation of RNA when present.

The large multiplicity of p450 enzyme forms, their differing structure and function have made their research on *Nicotiana* p450 enzymes very difficult before the enclosed invention. In addition, cloning of p450 enzymes has been hampered at least in part because these membrane-localized proteins are typically present in low abundance and often unstable to purification. Hence, a need exists for the identification of p450 enzymes in plants and the nucleic acid sequences associated with those p450 enzymes. In particular, only a few cytochrome p450 proteins have been reported in Nicotiana. The inventions described herein entail the discovery of a substantial number of cytochrome p450 fragments that correspond to several groups of p450 species based on their sequence identity.

### SUMMARY

The present invention is directed to an isolated nucleic acid molecule, a transgenic tobacco plant and methods involving berne, as depicted in the claims. The present invention is further directed to plant p450 enzymes from *Nicotiana.* The present invention is also directed to p450 enzymes in plants whose expression is induced by ethylene and/or plant senescence. The present invention is yet further directed to nucleic acid sequences in plants having enzymatic activities, for example, being categorized as oxygenase, demethylase and the like, or other and the use of those sequences to reduce or silence the expression or over-expression of these enzymes. The invention also relates to p450 enzymes found in plants containing higher nornicotine levels than plants exhibiting lower nornicotine levels.

In one aspect, the disclosure is directed to nucleic acid sequences as set forth in SEQ. ID. Nos. 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313 and 315.

In a second related aspect, those fragments containing greater than 75% identity in nucleic acid sequence were placed into groups dependent upon their identity in a region corresponding to the first nucleic acid following the cytochrome p450 motif GXRXCX(G/A) to the stop codon. The representative nucleic acid groups and respective species are shown in Table I.

In a third aspect, the disclosure is directed to amino acid sequences as set forth in SEQ. ID. Nos. 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 96, 98, 100, 102, 104, 106, 108, 110, 112, 114, 116, 118, 120, 122, 124, 126, 128, 130, 132, 134, 136, 138, 140, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314 and 316.

In a fourth related aspect, those fragments containing greater than 71% identity in amino acid sequence were placed into groups dependent upon their identity to each other in a region corresponding to the first amino acid following the cytochrome p450 motif GXRXCX(G/A) to the stop codon. The representative amino acid groups and respective species are shown in Table II.

In a fifth aspect, the disclosure is directed to amino acid sequences of full length genes as set forth in SEQ. ID. Nos. 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314 and 316.

In a sixth related aspect, those full length genes containing 85% or greater identity in amino acid sequence were placed into groups dependent upon the identity to each other. The representative amino acid groups and respective species are shown in Table III.

In a seventh aspect, the disclosure is directed to amino acid sequences of the fragments set forth in SEQ. ID. Nos. 299-357.

In the eighth related aspect, those fragments containing 90% or greater identity in amino acid sequence were placed into groups dependent upon their identity to each other in a region corresponding to the first cytochrome p450 domain, UXXRXXZ, to the third cytochrome domain, GXRXO, where U is E or K, X is any amino acid and Z is R, T, S or M. The representative amino acid groups respective species shown in Table IV.

In a ninth related aspect, the reduction or elimination or over-expression of p450 enzymes in Nicotiana plants may be accomplished transiently using RNA viral systems.

Resulting transformed or infected plants are assessed for phenotypic changes including, but not limited to, analysis of endogenous p450 RNA transcripts, p450 expressed peptide, and concentrations of plant metabolites using techniques commonly available to one having ordinary skill in the art.

In a tenth important aspect, the present disclosure is also directed to generation of trangenic Nicotiana lines that the p450 enzyme activity levels. In accordance with the disclosure, these transgenic lines include nucleic acid sequences that are effective for reducing or silencing or increasing the expression of certain enzyme thus resulting in phenotypic effects within Nicotiana. Such nucleic acid sequences include SEQ. ID. Nos. 1, 3, 5, 7, 9, 11, 13, 15, 17, 19. 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313 and 315.

In a very important eleventh aspect of the disclosure, plant cultivars including nucleic acids of the present invention in a down regulation capacity using either full length genes or fragments thereof or in an over-expression capacity using full length genes will have altered metabolite profiles relative to control plants.

In a twelfth aspect of the disclosure, plant cultivars including nucleic acid of the present invention using either full length genes or fragments thereof in modifying the biosynthesis or breakdown of metabolites derived from the plant or external to the plants, will have use in tolerating certain exogenous chemicals or plant pests. Such nucleic acid sequences include SEQ ID. Nos. 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313 and 315.

In a thirteenth aspect, the present disclosure is directed to the screening of plants, more preferably Nicotiana, that contain genes that have substantial nucleic acid identity to the taught nucleic acid sequence. The use of the disclosure would be advantageous to identify and select plants that contain a nucleic acid sequence with exact or substantial identity where such plants are part of a breeding program for traditional or transgenic varieties, a mutagenesis program, or naturally occurring diverse plant populations. The screening of plants for substantial nucleic acid identity may be accomplished by evaluating plant nucleic acid materials using a nucleic acid probe in conjunction with nucleic acid detection protocols including, but not limited to, nucleic acid hybridization and PCR analysis. The nucleic acid probe may consist of the taught nucleic acid sequence or fragment thereof corresponding to SEQ ID 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313 and 315.

In a fourteenth aspect, the present disclosure is directed to the identification of plant genes, more preferably Nicotiana, that share substantial amino acid identity corresponding to the taught nucleic acid sequence. The identification of plant genes including both cDNA and genomic clones, those cDNAs and genomic clones, more preferably from Nicotiana may be accomplished by screening plant cDNA libraries using a nucleic acid probe in conjunction with nucleic acid detection protocols including, but not limited to, nucleic acid hybridization and PCR analysis. The nucleic acid probe may be comprised of nucleic acid sequence or fragment thereof corresponding to SEQ ID 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145 and 147.

In an alterative fifteenth aspect, cDNA expression libraries that express peptides may be screened using antibodies directed to part or all of the taught amino acid sequence. Such amino acid sequences include SEQ ID 2, 4, 8, 9, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 96, 98, 100, 102, 104, 106, 108, 110, 112, 114, 116, 118, 120, 122, 124, 126, 128, 130, 132, 134, 136, 138, 140, 144, 146, 148.

In a sixteenth important aspect, the present disclosure is also directed to generation of transgenic Nicotiana lines that have over-expression of p450 enzyme activity levels. In accordance with the disclosure, these transgenic lines include all nucleic acid sequences encoding the amino acid sequences of full length genes that are effective for increasing the expression of certain enzyme thus resulting in phenotypic effects within Nicotiana. Such amino acid sequences include SEQ. ID. 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298. 300, 302, 304, 306, 308, 310, 312, 314 and 316.

A tobacco product is also provided that includes tobacco leaf (lamina and/or stem) having reduced amounts of nornicotine. The tobacco product includes tobacco (tobacco leaf including lamina and/or stem) from a plant that includes the sequences described herein or where genes encoding tobacco specific nitrosamines have been eliminated or suppressed. The elimination or suppression of genes encoding tobacco speicific nitrosamines is effective for reducing tobacco specific nitrosamines in the tobacco products from about 5 to about 10%, in another aspect from about 10 to 20%, in another aspect about 20 to 30%, and in another aspect greate than 30%, as compared to tobacco products made from tobacco plants where gnees coding for tobacco specific nitrosamines have not been eliminated or suppressed. As used herein, the tobacco product may include cigarettes, cigars, pipe tobacco, snuff chewing tobacco, products blended with the tobacco product, and mixtures thereof.

### BRIEF DESCRIPTION OF DRAWINGS

Figure 1 shows nucleic acid SEQ. ID. No.:1 and amino acid SEQ. ID. No.:2.
Figure 2 shows nucleic acid SEQ. ID. No.:3 and amino acid SEQ. ID. No.:4.
Figure 3 shows nucleic acid SEQ. ID. No.:5 and amino acid SEQ. ID. No.:6.
Figure 4 shows nucleic acid SEQ. ID. No.:7 and amino acid SEQ. ID. No.:8.
Figure 5 shows nucleic acid SEQ. ID. No.:9 and amino acid SEQ. ID. No.:10.

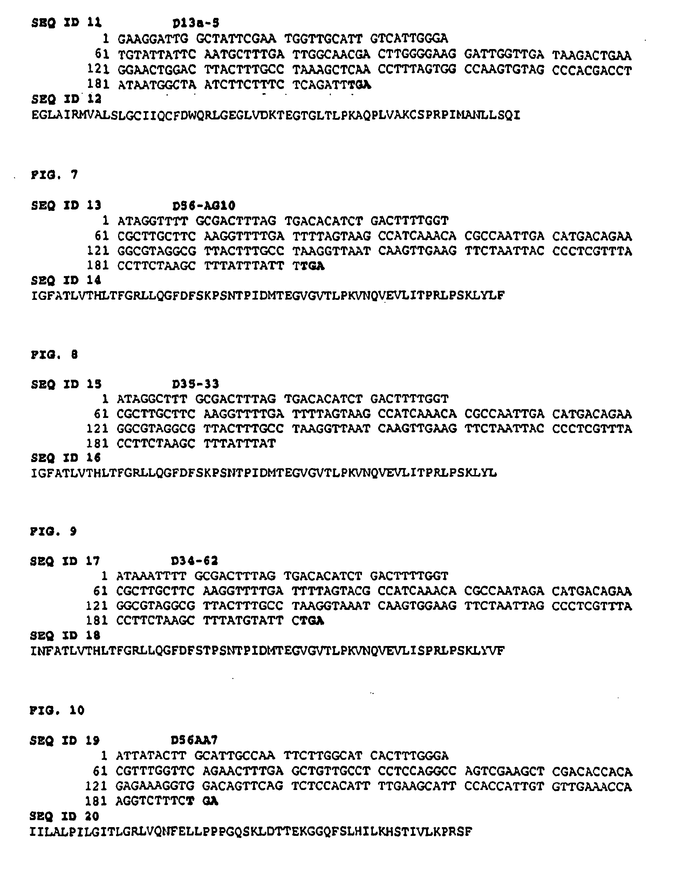

Figure 6 shows nucleic acid SEQ. ID. No.:11 and amino acid SEQ. ID. No.:12.
Figure 7 shows nucleic acid SEQ. ID. No.:13 and amino acid SEQ. ID. No.:14.
Figure 8 shows nucleic acid SEQ. ID. No.:15 and amino acid SEQ. ID. No.:16.
Figure 9 shows nucleic acid SEQ. ID. No.:17 and amino acid SEQ. ID. No.:18.
Figure 10 shows nucleic acid SEQ. ID. No.:19 and amino acid SEQ. ID. No.:20.

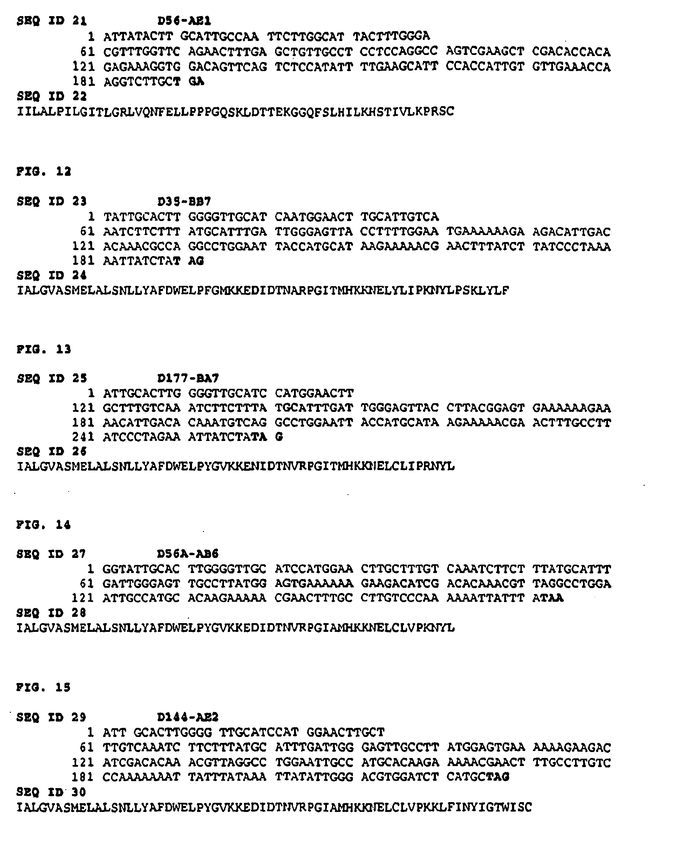

Figure 11 shows nucleic acid SEQ. ID. No.:21 and amino acid SEQ. ID. No.:22.
Figure 12 shows nucleic acid SEQ. ID. No.:23 and amino acid SEQ. ID. No.:24.
Figure 13 shows nucleic acid SEQ. ID. No.:25 and amino acid SEQ. ID. No.:26.
Figure 14 shows nucleic acid SEQ. ID. No.:27 and amino acid SEQ. ID. No.:28.
Figure 15 shows nucleic acid SEQ. ID. No.:29 and amino acid SEQ. ID. No.:30.

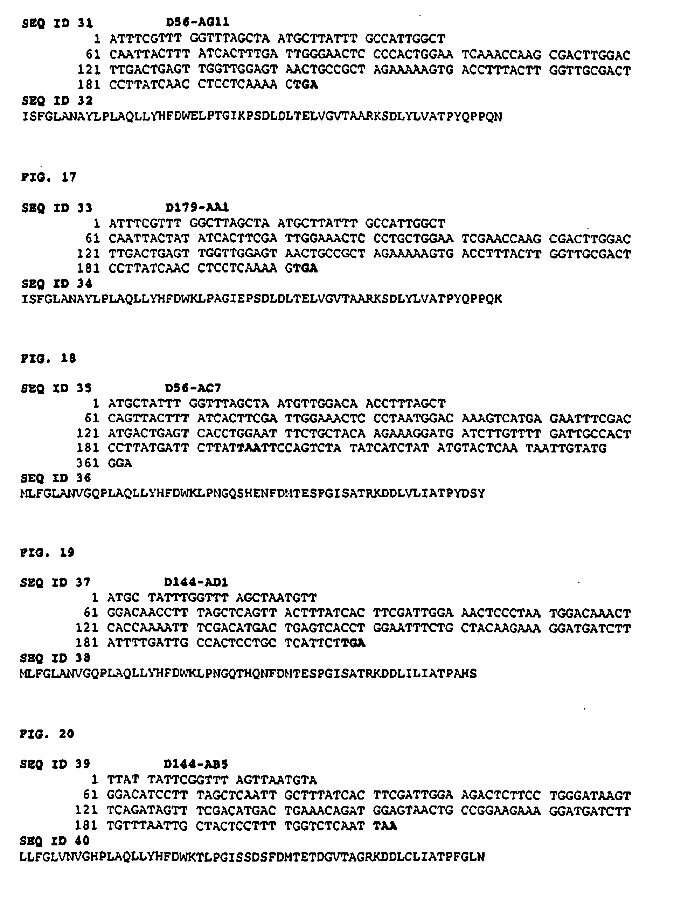

Figure 16 shows nucleic acid SEQ. ID. No.:31 and amino acid SEQ. ID. No.:32.
Figure 17 shows nucleic acid SEQ. ID. No.:33 and amino acid SEQ. ID. No.:34.
Figure 18 shows nucleic acid SEQ. ID. No.:35 and amino acid SEQ. ID. No.:36.
Figure 19 shows nucleic acid SEQ. ID. No.:37 and amino acid SEQ. ID. No.:38.
Figure 20 shows nucleic acid SEQ. ID. No.:39 and amino acid SEQ. ID. No.:40.

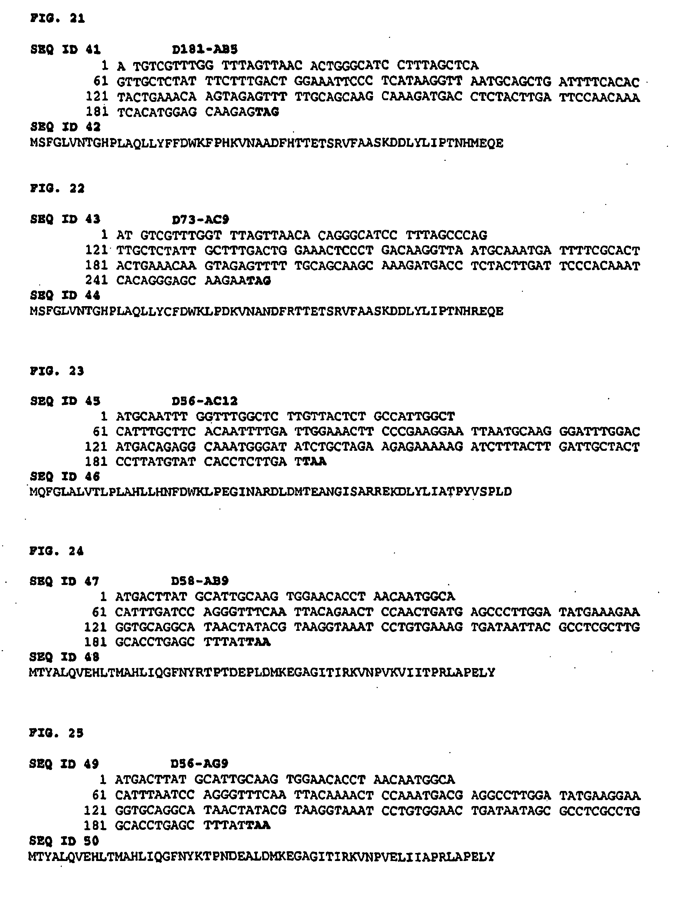

Figure 21 shows nucleic acid SEQ. ID. No.:41 and amino acid SEQ. ID. No.:42.
Figure 22 shows nucleic acid SEQ. ID. No.:43 and amino acid SEQ. ID. No.:44.
Figure 23 shows nucleic acid SEQ. ID. No.:45 and amino acid SEQ. ID. No.:46.
Figure 24 shows nucleic acid SEQ. ID. No.:47 and amino acid SEQ. ID. No.:48.
Figure 25 shows nucleic acid SEQ. ID. No.:49 and amino acid SEQ. ID. No.:50.

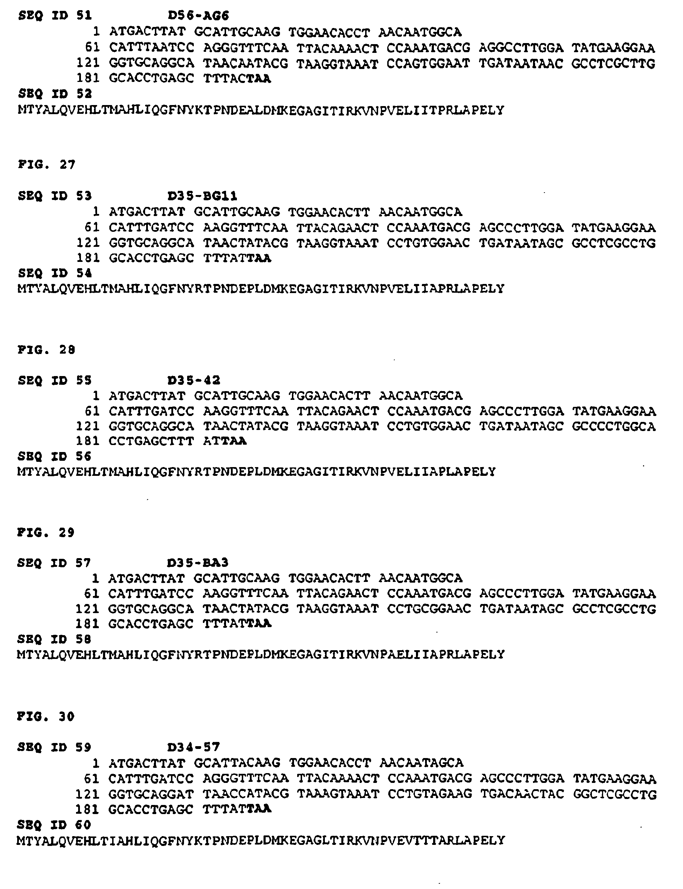

Figure 26 shows nucleic acid SEQ. ID. No.:51 and amino acid SEQ. ID. No.:52.
Figure 27 shows nucleic acid SEQ. ID. No.:53 and amino acid SEQ. ID. No.:54.
Figure 28 shows nucleic acid SEQ. ID. No.:55 and amino acid SEQ. ID. No.:56.
Figure 29 shows nucleic acid SEQ. ID. No.:57 and amino acid SEQ. ID. No.:58.
Figure 30 shows nucleic acid SEQ. ID. No.:59 and amino acid SEQ. ID. No.:60.

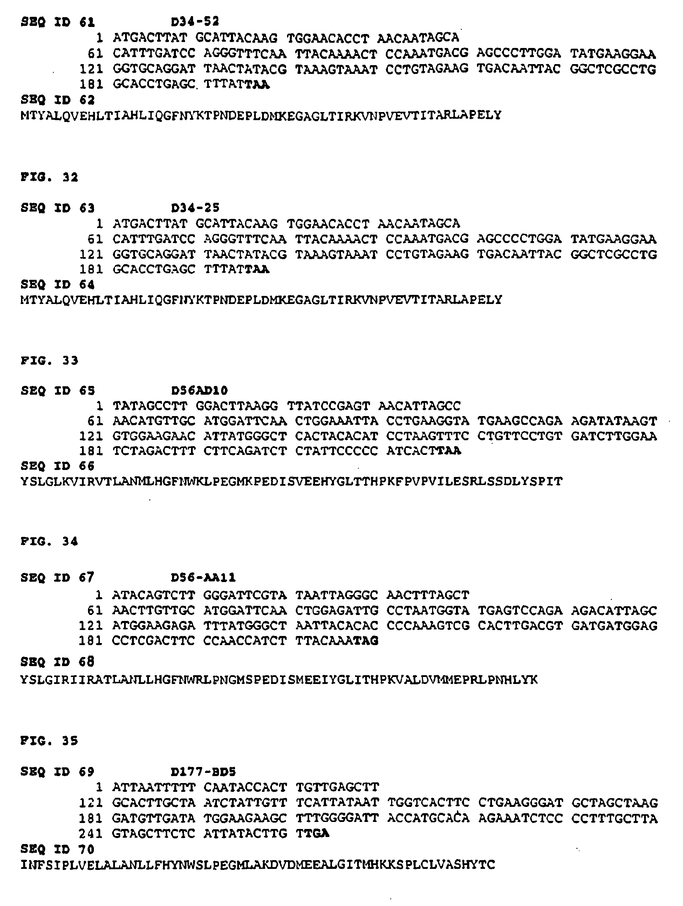

Figure 31 shows nucleic acid SEQ. ID. No.:61 and amino acid SEQ. ID. No.:62.
Figure 32 shows nucleic acid SEQ. ID. No.:63 and amino acid SEQ. ID. No.:64.
Figure 33 shows nucleic acid SEQ. ID. No.:65 and amino acid SEQ. ID. No.:66.
Figure 34 shows nucleic acid SEQ. ID. No.:67 and amino acid SEQ. ID. No.:68.
Figure 35 shows nucleic acid SEQ. ID. No.:69 and amino acid SEQ. ID. No.:70.

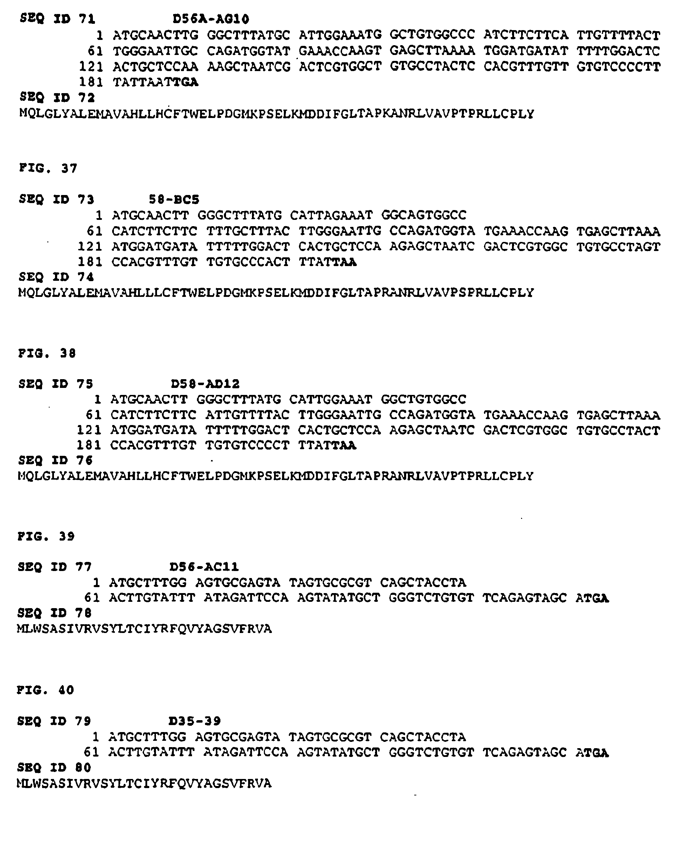

Figure 36 shows nucleic acid SEQ. ID. No.:71 and amino acid SEQ. ID. No.:72.
Figure 37 shows nucleic acid SEQ. ID. No.:73 and amino acid SEQ. ID. No.:74.
Figure 38 shows nucleic acid SEQ. ID. No.:75 and amino acid SEQ. ID. Not.:76.
Figure 39 shows nucleic acid SEQ. ID. No.:77 and amino acid SEQ. ID. No.:78.
Figure 40 shows nucleic acid SEQ. ID. No.:79 and amino acid SEQ. ID. No.:80.

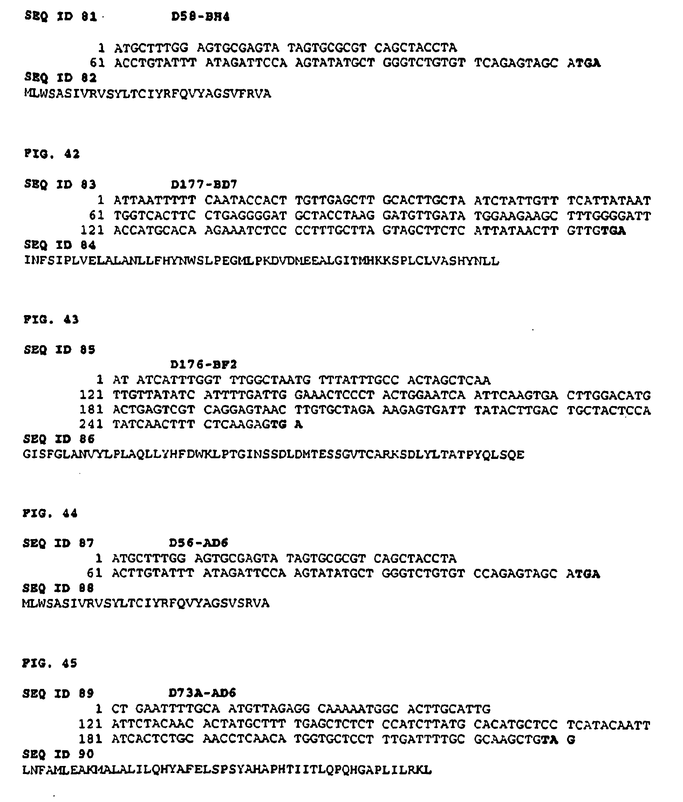

Figure 41 shows nucleic acid SEQ. ID. No.:81 and amino acid SEQ. ID. No.:82.
Figure 42 shows nucleic acid SEQ. ID. No.:83 and amino acid SEQ. ID. No.:84.
Figure 43 shows nucleic acid SEQ. ID. No.:85 and amino acid SEQ. ID. No.:86.
Figure 44 shows nucleic acid SEQ. ID. No.:87 and amino acid SEQ. ID. No.:88.
Figure 45 shows nucleic acid SEQ. ID. No.:89 and amino acid SEQ. ID. No.:90.

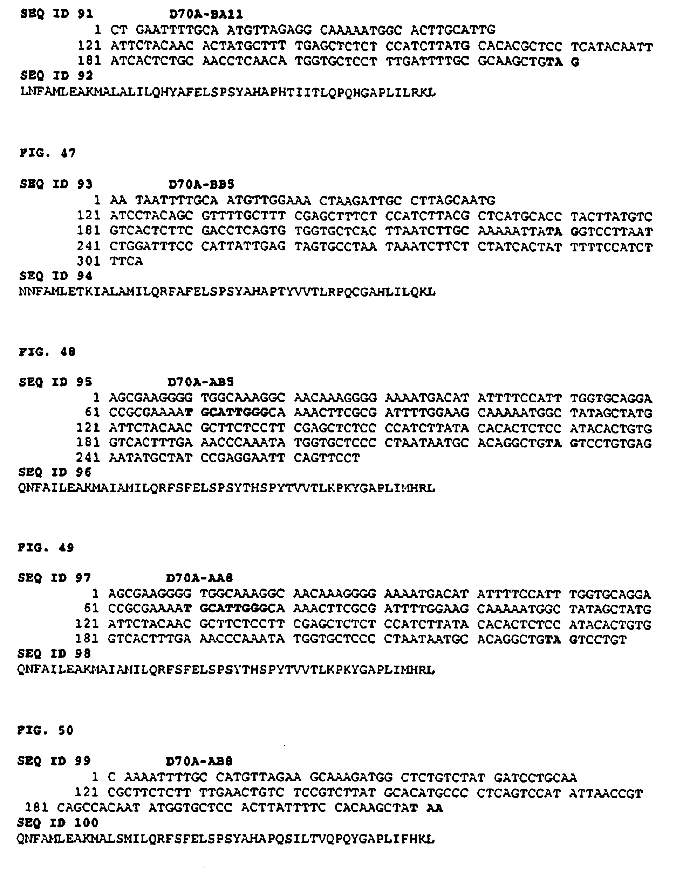

Figure 46 shows nucleic acid SEQ. ID. No.:91 and amino acid SEQ. ID. No.:92.
Figure 48 shows nucleic acid SEQ. ID. No.:95 and amino acid SEQ. ID. No.:96.
Figure 49 shows nucleic acid SEQ. ID. No.:97 and amino acid SEQ. ID. No.:98.
Figure 50 shows nucleic acid SEQ. ID. No.:99 and amino acid SEQ. ID. No.:100.

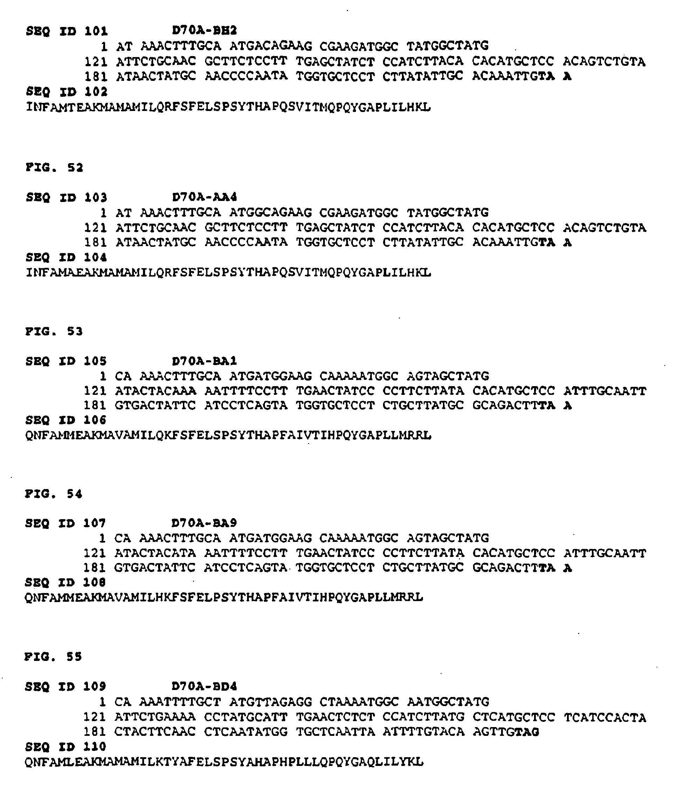

Figure 51 shows nucleic acid SEQ. ID. No.:101 and amino acid SEQ. ID. No.:102.
Figure 52 shows nucleic acid SEQ. ID. No.:103 and amino acid SEQ. ID. No.:104.
Figure 53 shows nucleic acid SEQ. ID. No.:105 and amino acid SEQ. ID. No.:106.
Figure 54 shows nucleic acid SEQ. ID. No.:107 and amino acid SEQ. ID. No.:108.
Figure 55 shows nucleic acid SEQ. ID. No.:109 and amino acid SEQ. ID. No.:110.

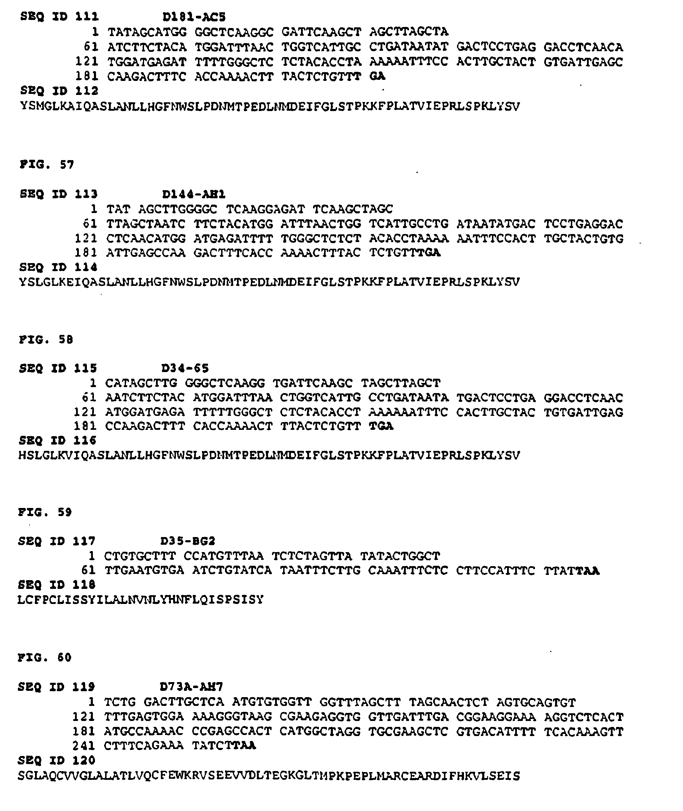

Figure 56 shows nucleic acid SEQ. ID. No.:111 and amino acid SEQ. ID. No.:112.
Figure 57 shows nucleic acid SEQ. ID. No.:113 and amino acid SEQ. ID. No.:114.
Figure 58 shows nucleic acid SEQ. ID. No.:115 and amino acid SEQ. ID. No.:116.
Figure 59 shows nucleic acid SEQ. ID. No.:117 and amino acid SEQ. ID. No.:118.
Figure 60 shows nucleic acid SEQ. ID. No.:119 and amino acid SEQ. ID. No.:120.

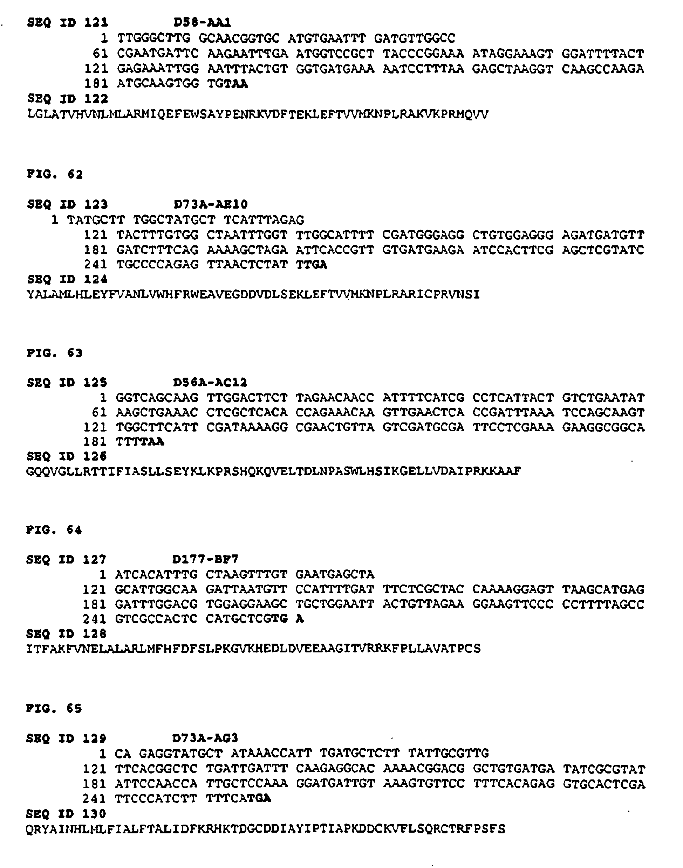

Figure 61 shows nucleic acid SEQ. ID. No.:121 and amino acid SEQ. ID. No.:122.
Figure 62 shows nucleic acid SEQ. ID. No.:123 and amino acid SEQ. ID. No.:124.
Figure 63 shows nucleic acid SEQ. ID. No.:125 and amino acid SEQ. ID. No.:126.
Figure 64 shows nucleic acid SEQ. ID. No.:127 and amino acid SEQ. ID. No.:128.
Figure 65 shows nucleic acid SEQ. ID. No.:129 and amino acid SEQ. ID. No.:130.

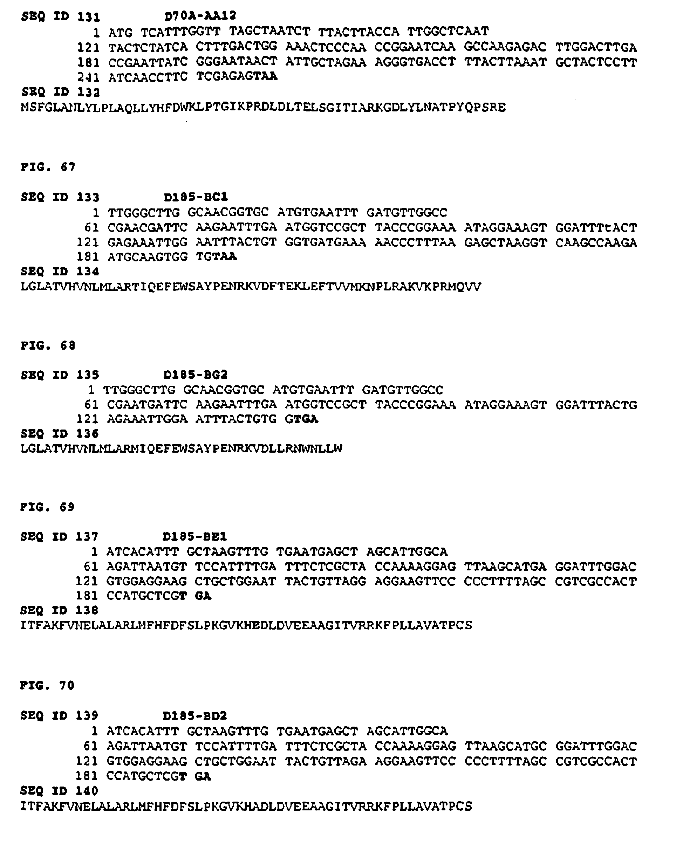

Figure 66 shows nucleic acid SEQ. ID. No.:131 and amino acid SEQ. ID. No.:132.
Figure 67 shows nucleic acid SEQ. ID. No.:133 and amino acid SEQ. ID. No.:134.
Figure 68 shows nucleic acid SEQ. ID. No.:135 and amino acid SEQ. ID. No.:136.
Figure 69 shows nucleic acid SEQ. ID. No.:137 and amino acid SEQ. ID. No.:138.
Figure 70 shows nucleic acid SEQ. ID. No.:139 and amino acid SEQ. ID. No.:140.

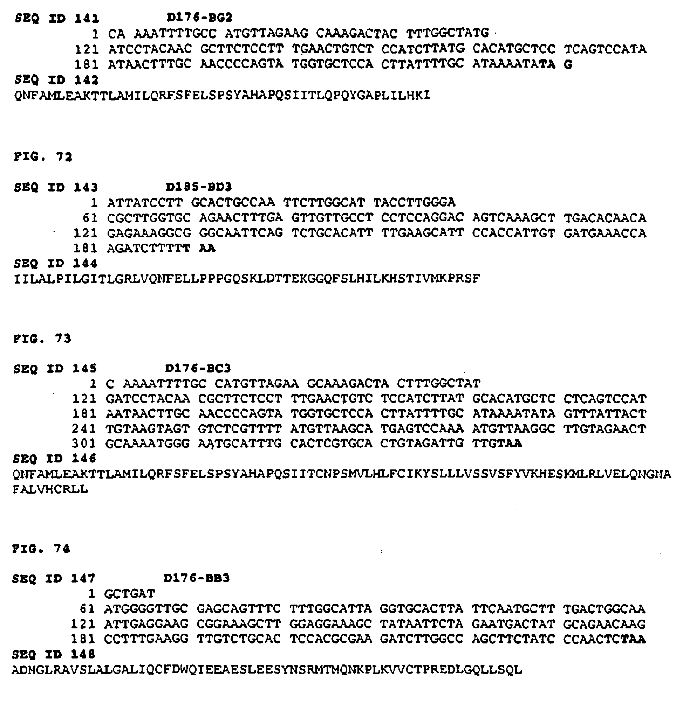

Figure 72 shows nucleic acid SEQ. ID. No.:143 and amino acid SEQ. ID. No.:144.
Figure 73 shows nucleic acid SEQ. ID. No.:145 and amino acid SEQ. ID. No.:146.

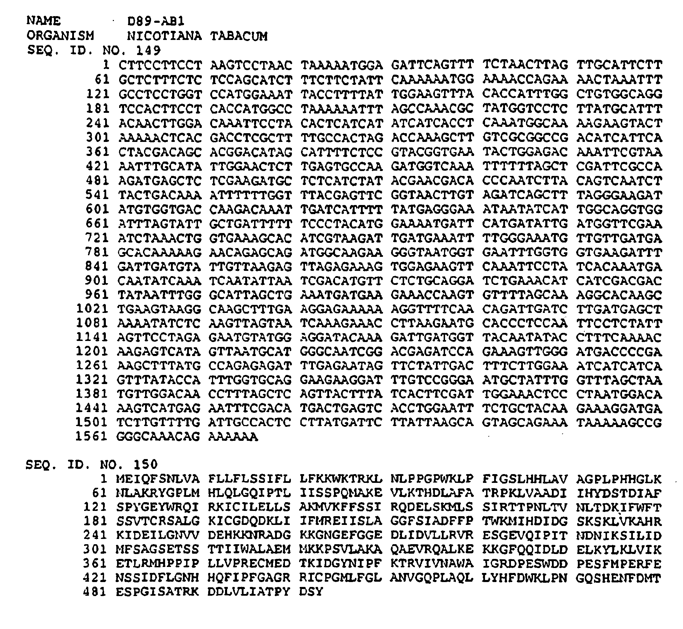

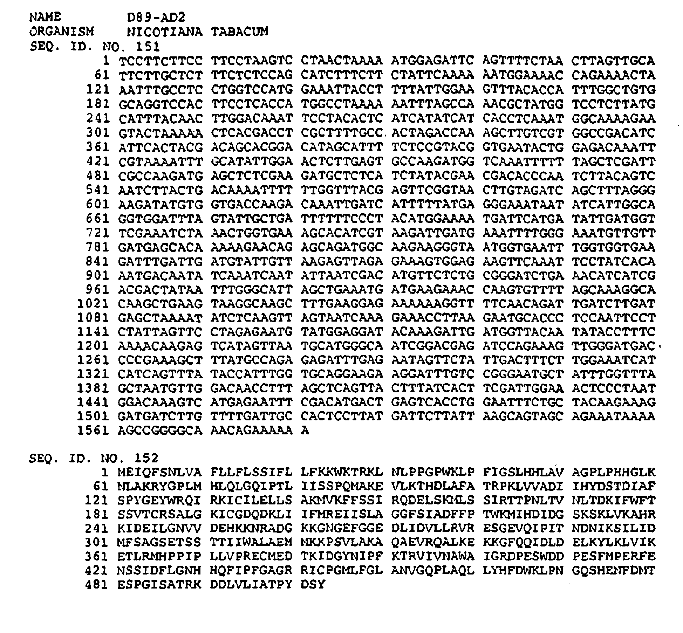

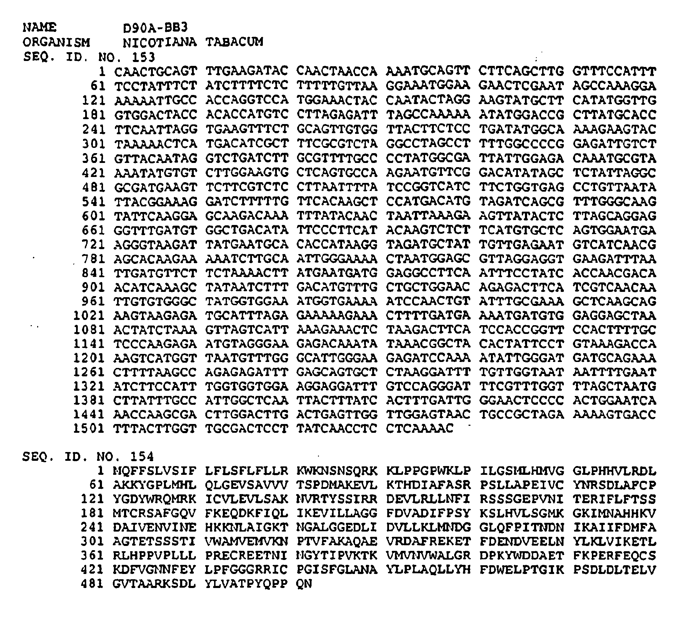

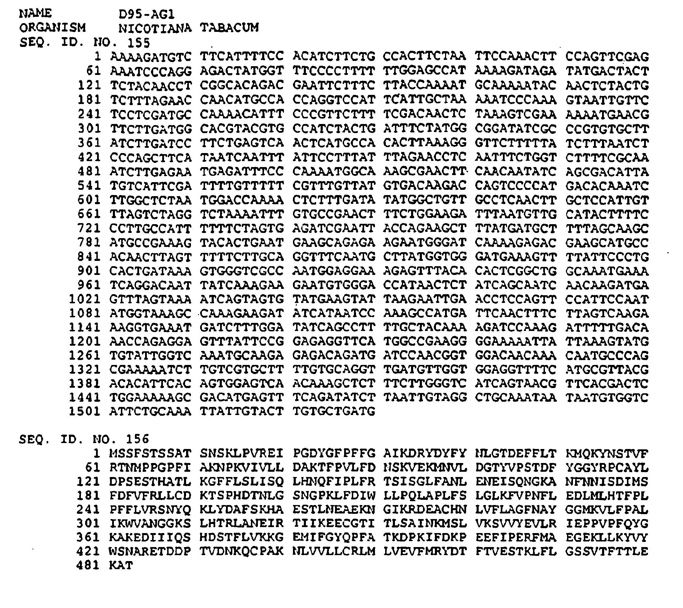

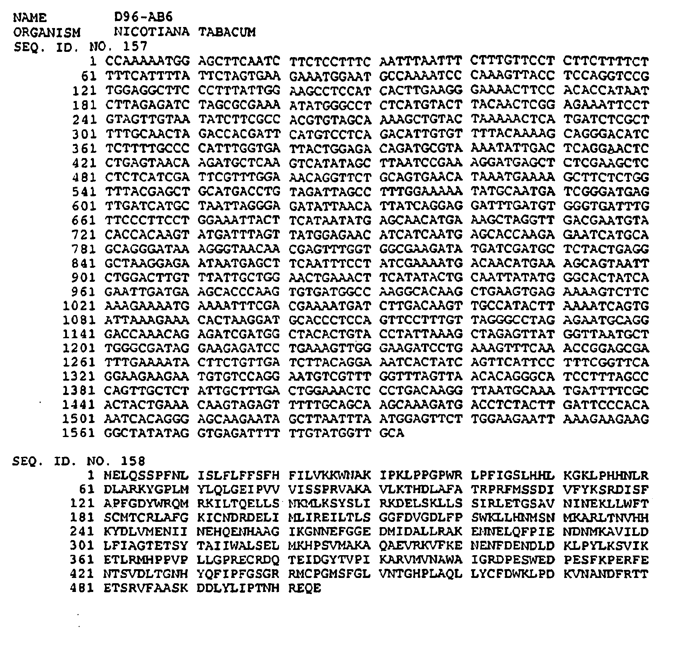

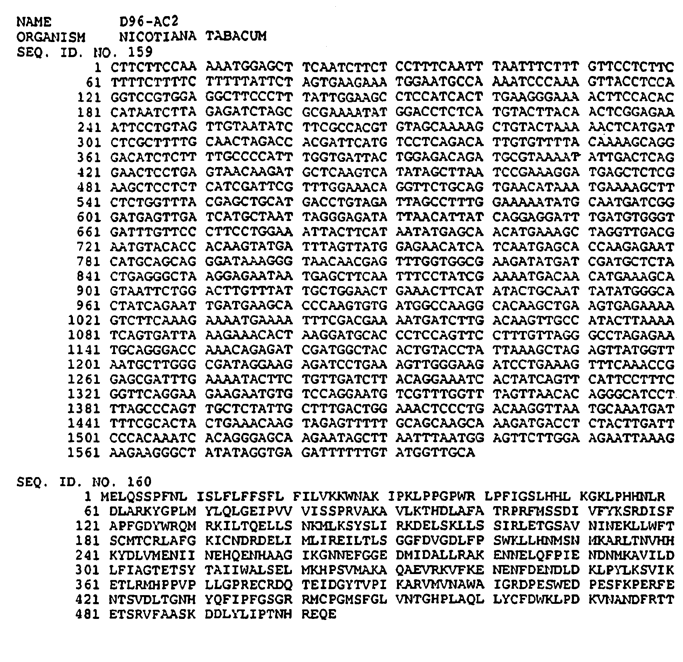

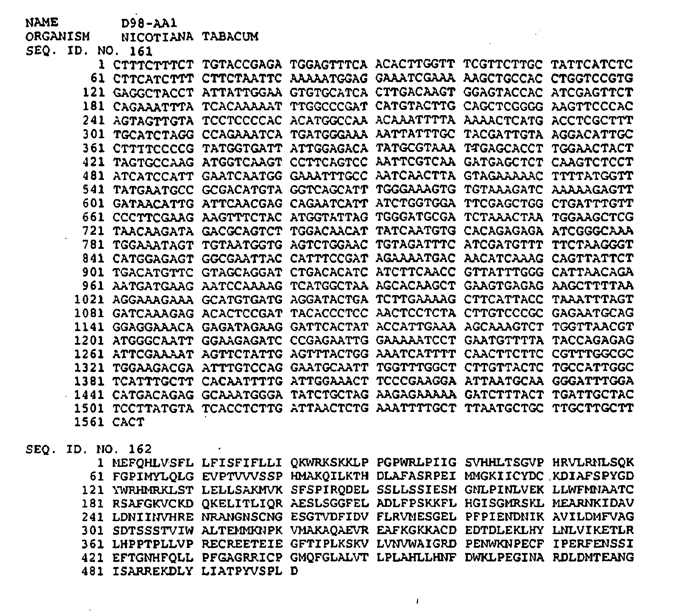

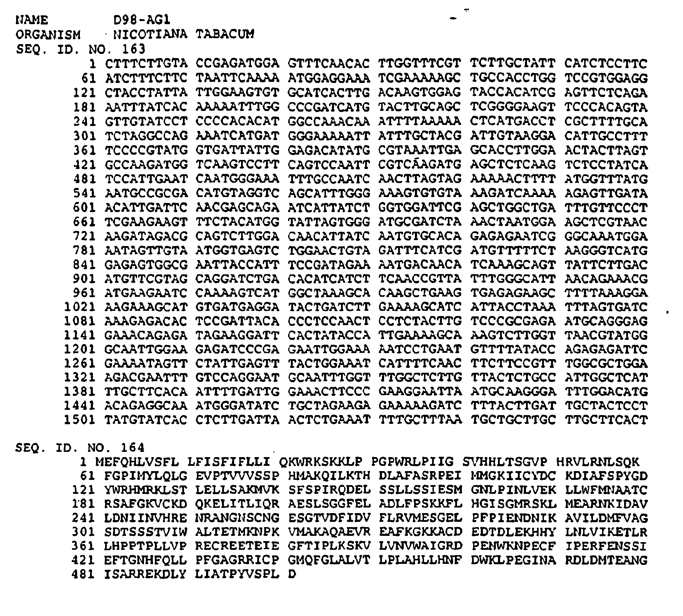

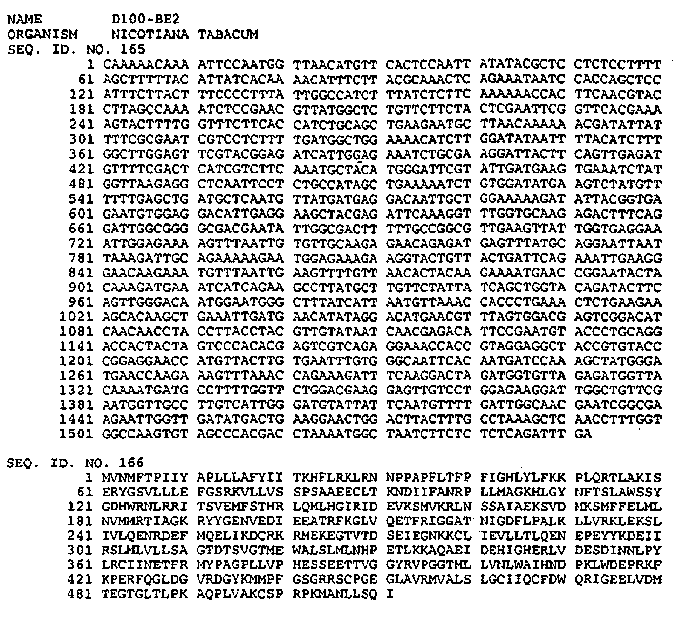

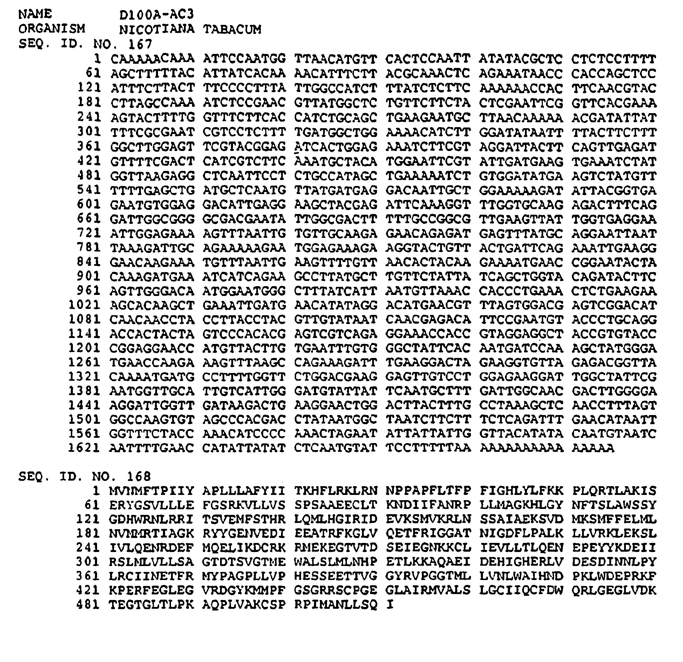

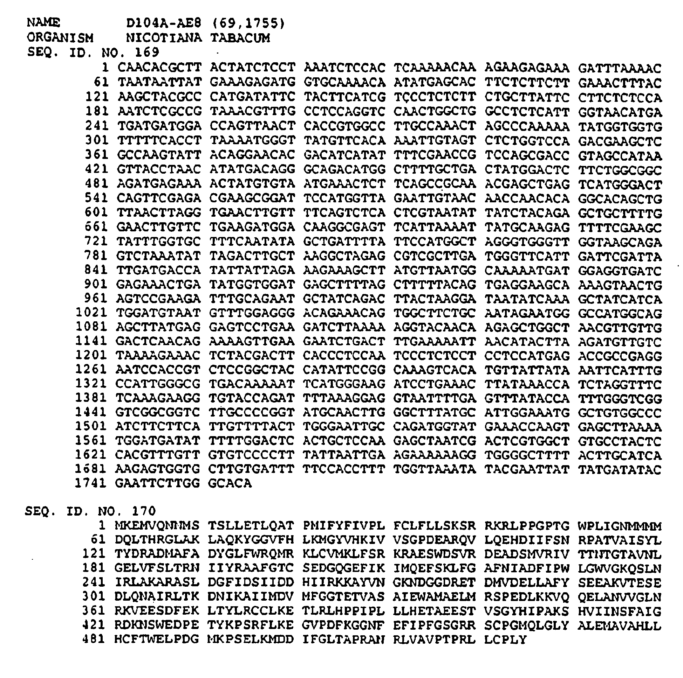

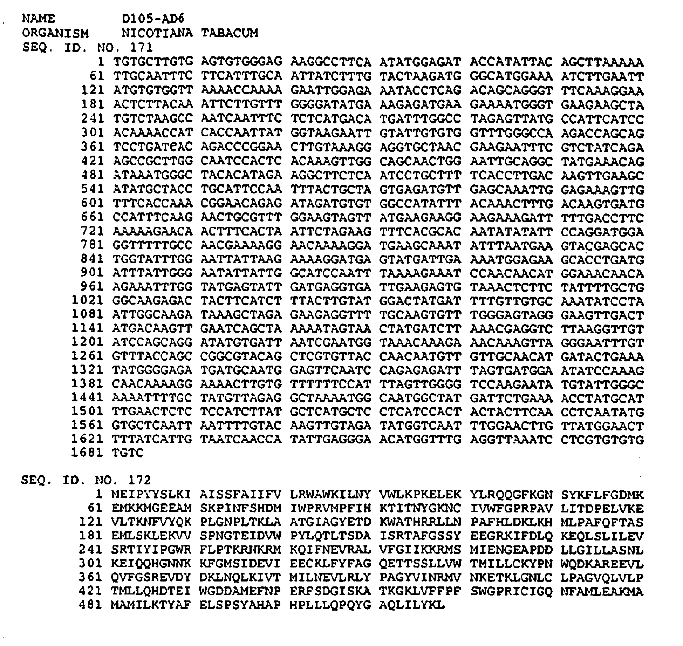

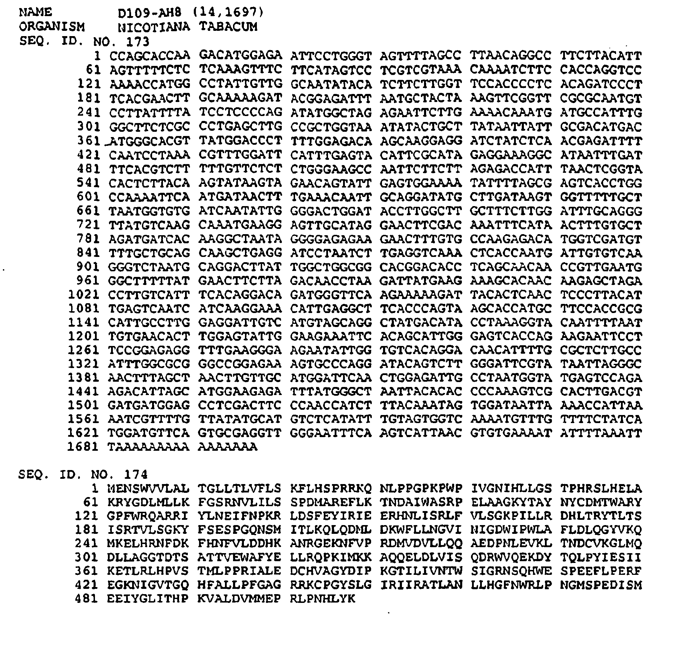

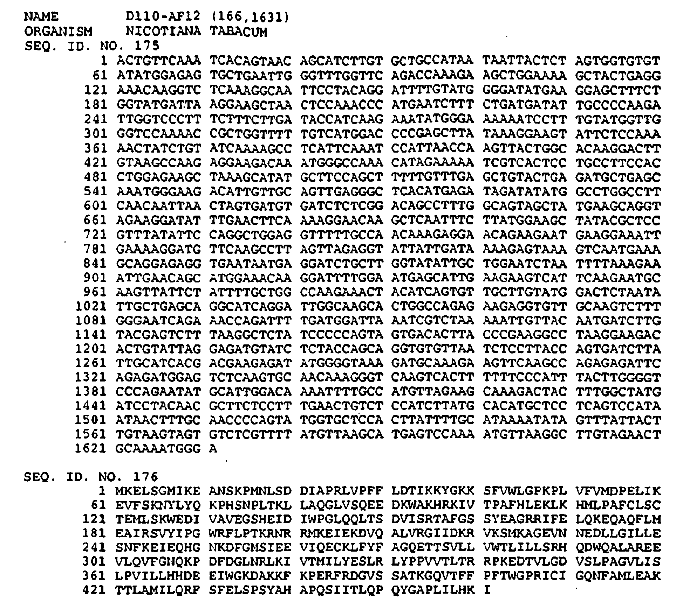

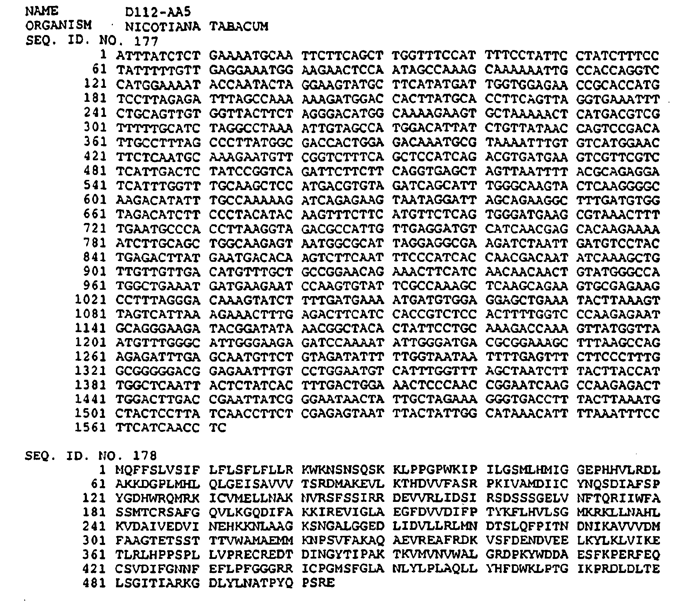

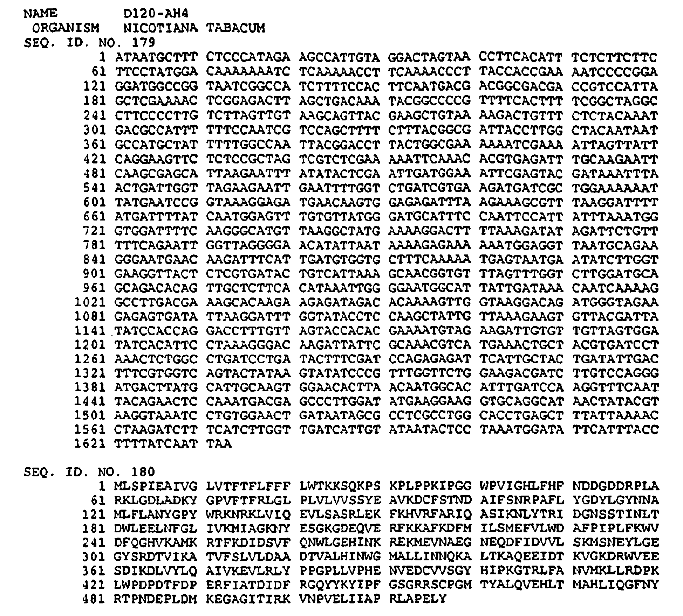

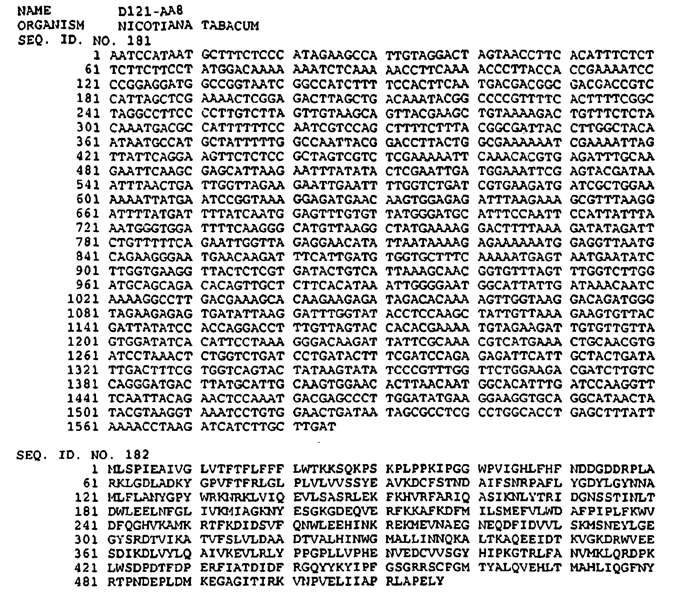

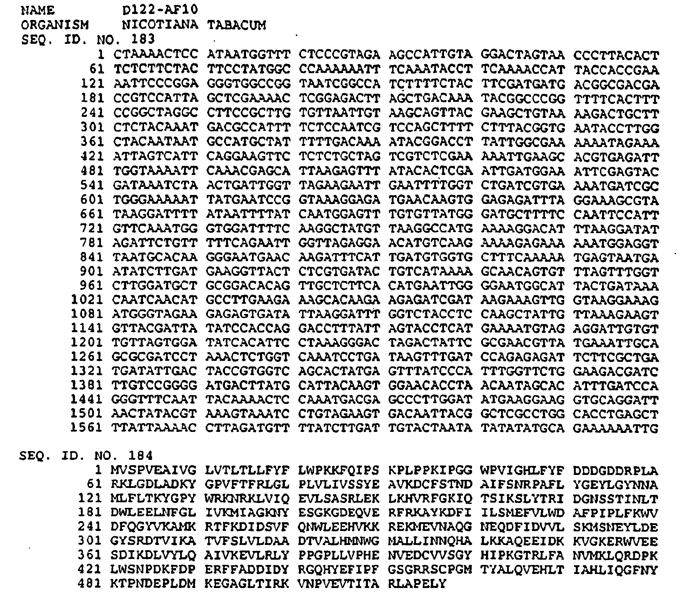

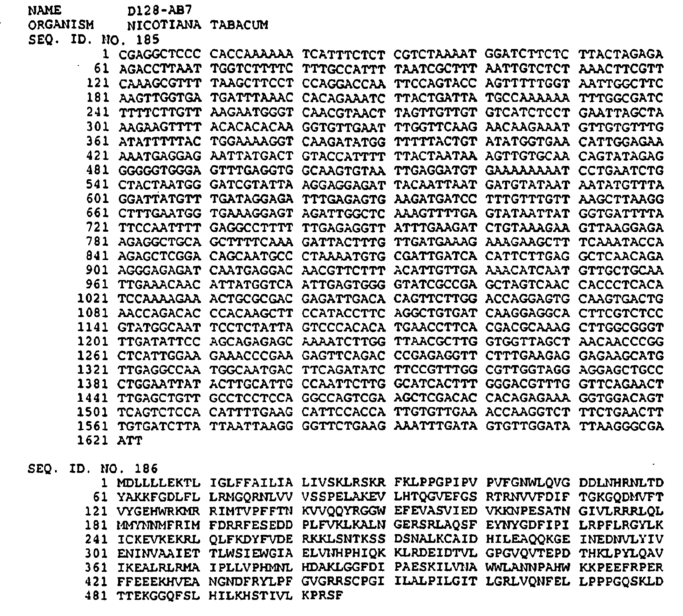

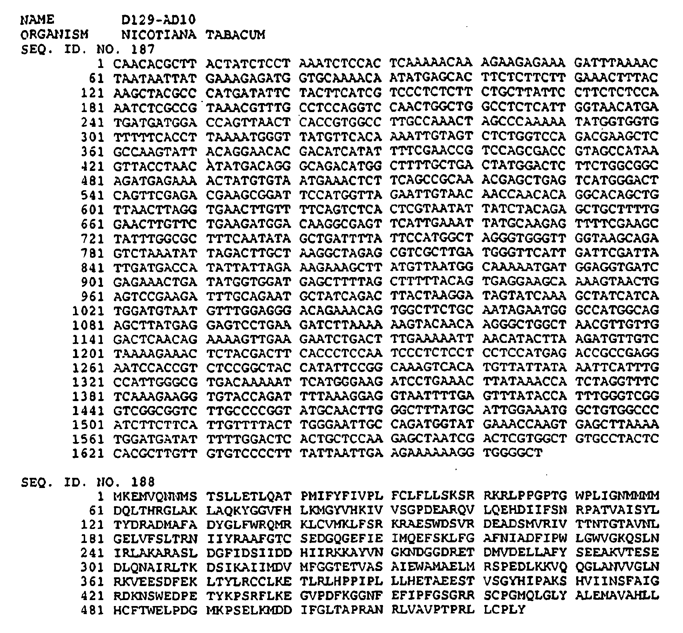

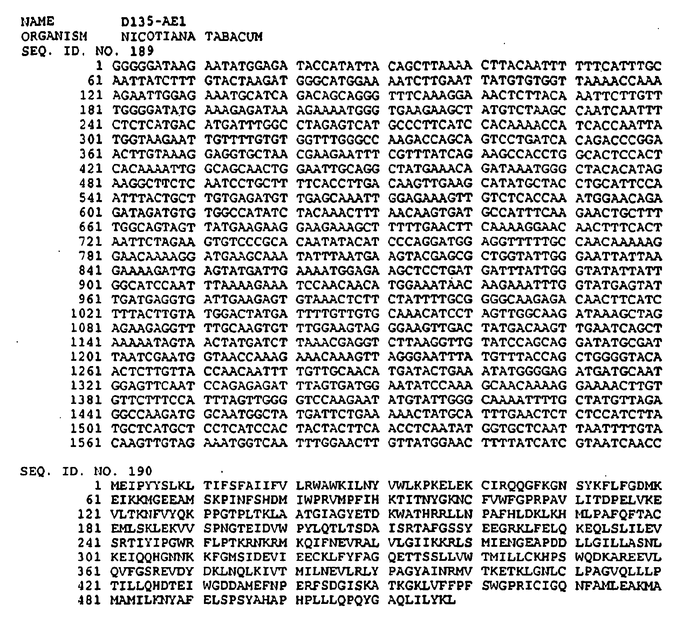

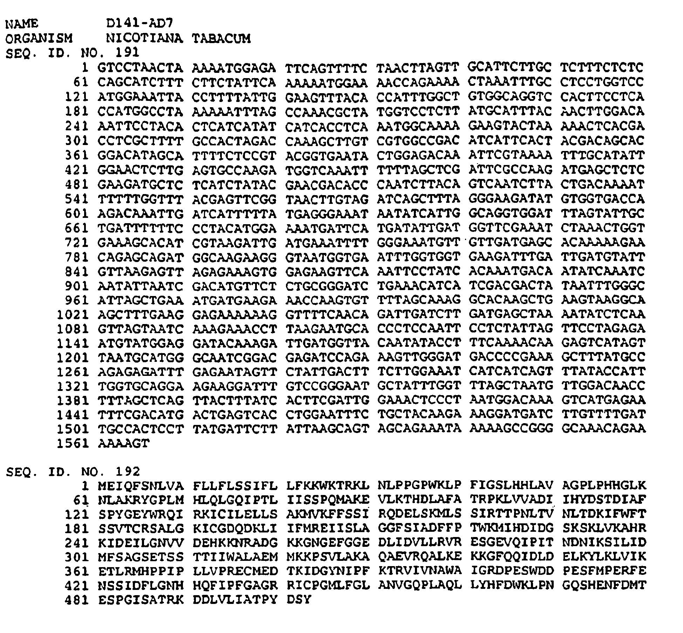

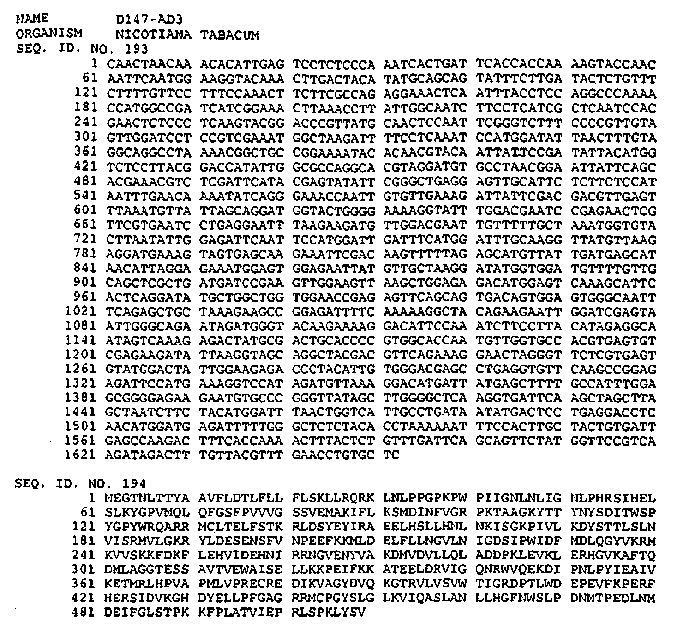

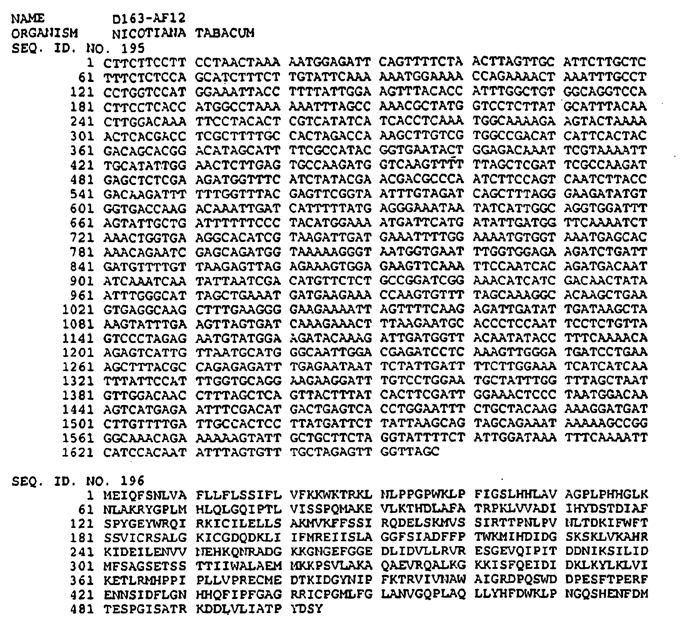

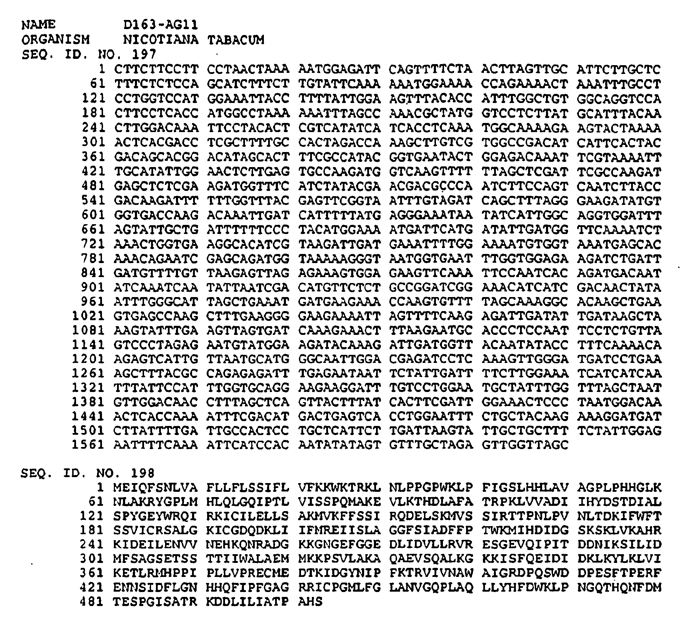

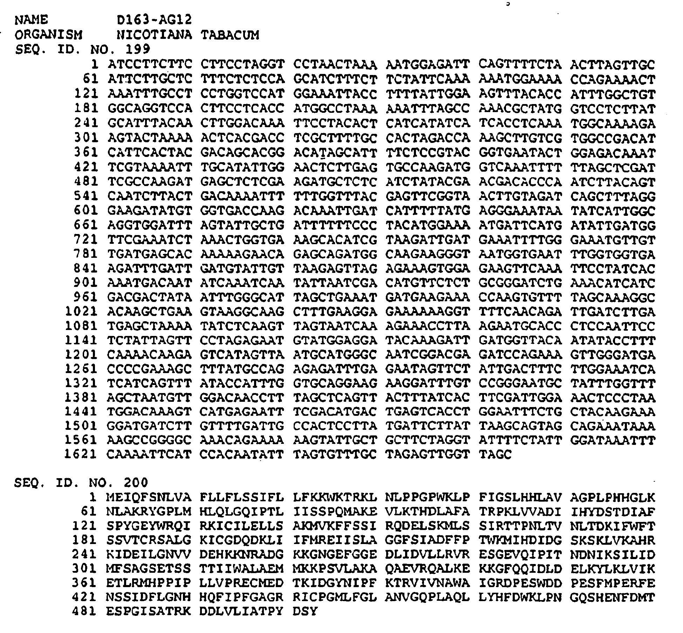

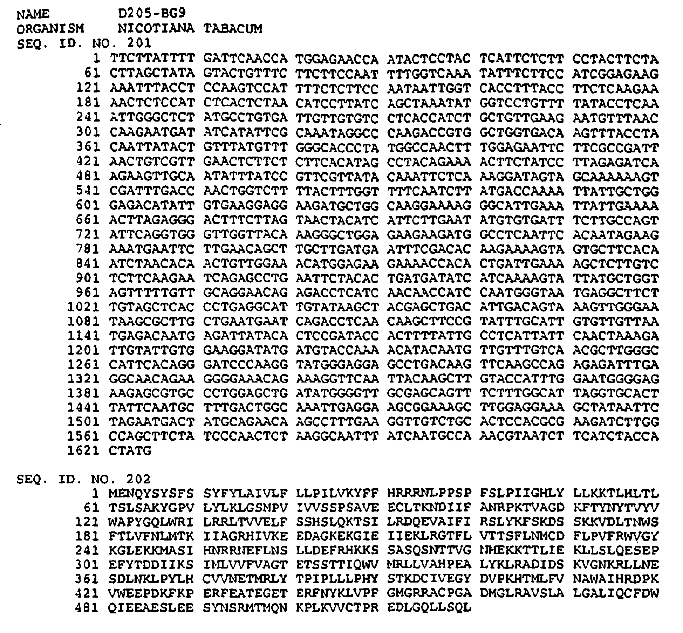

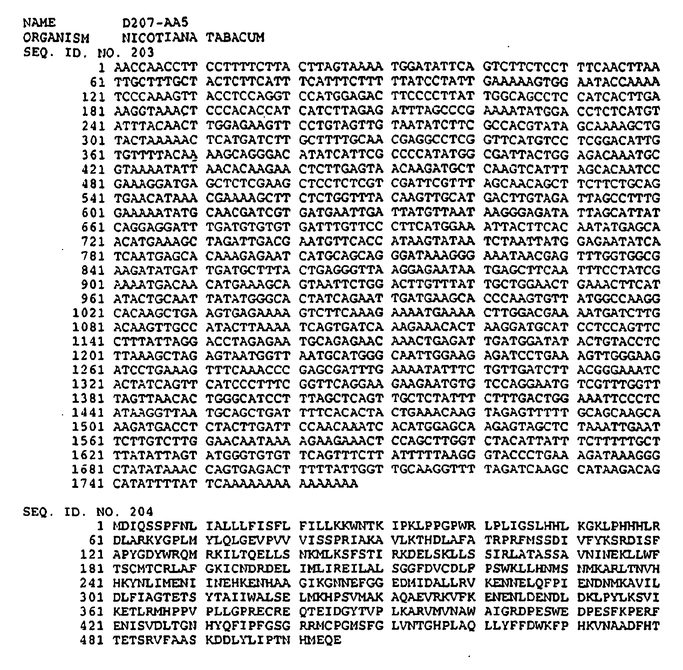

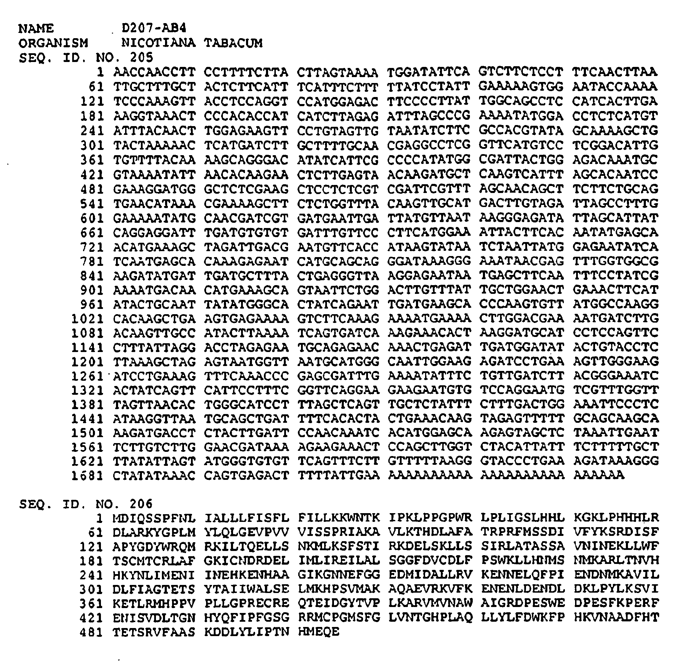

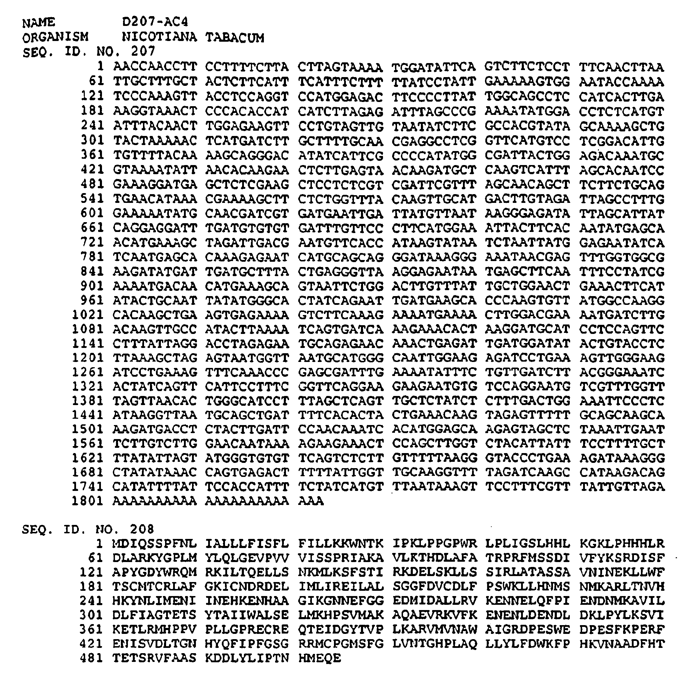

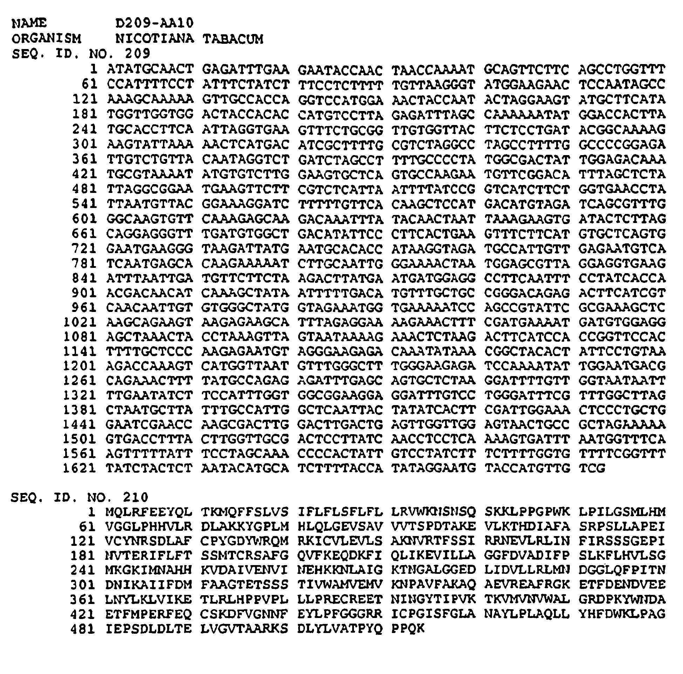

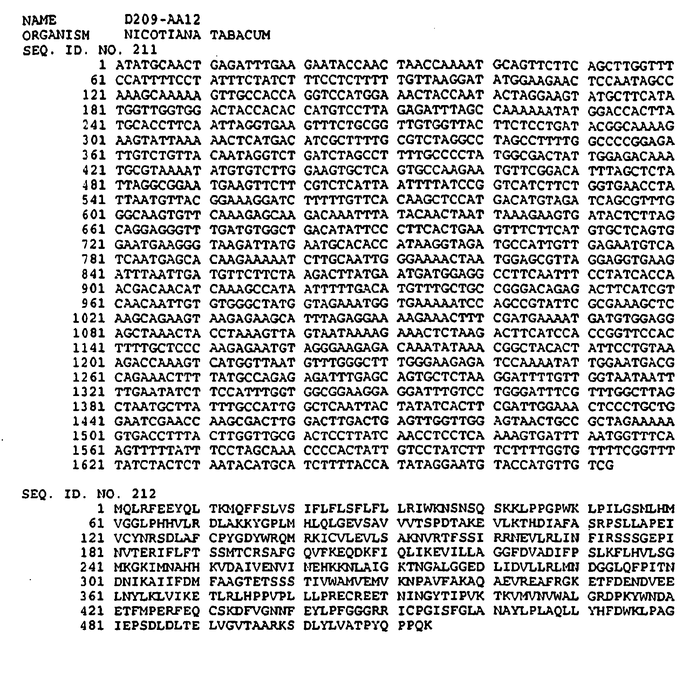

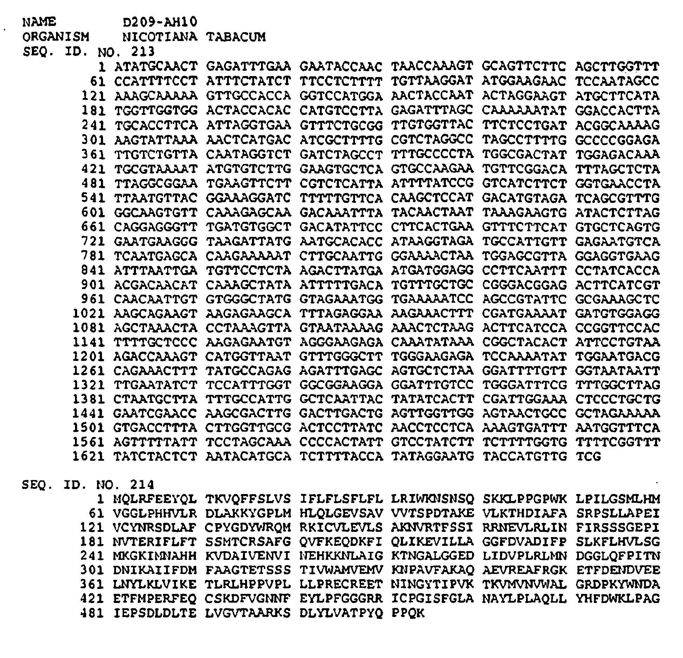

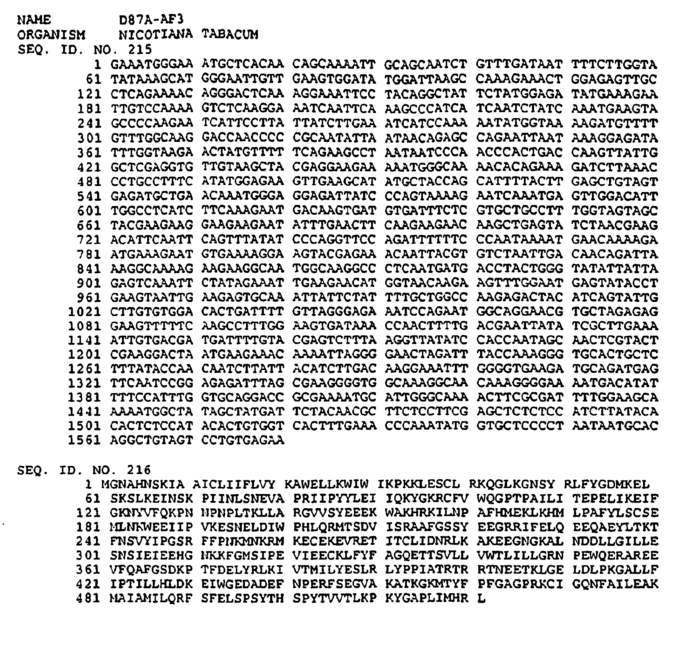

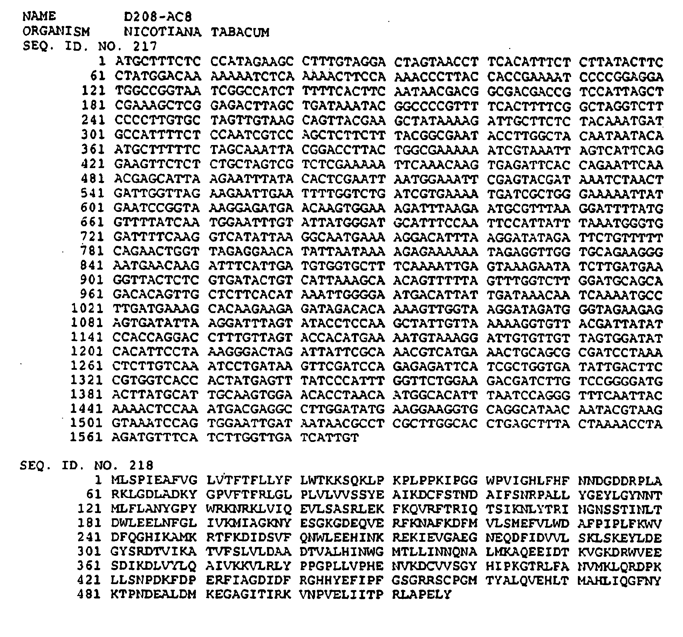

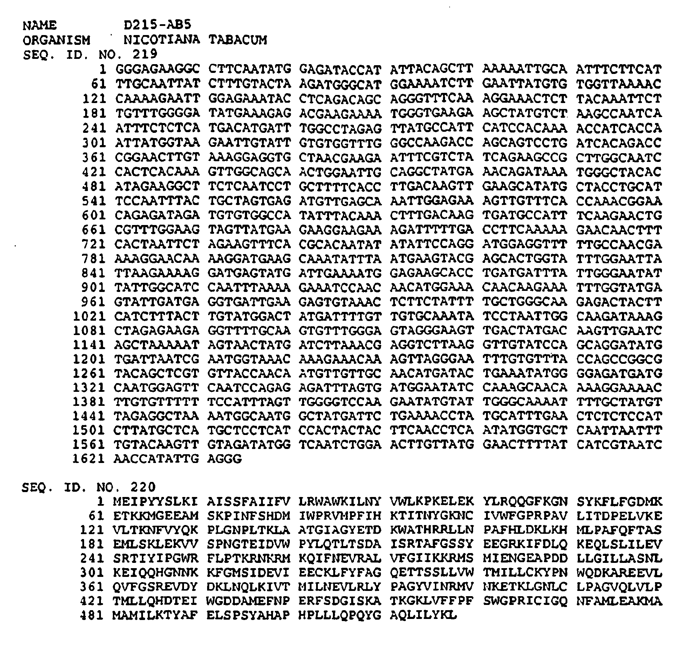

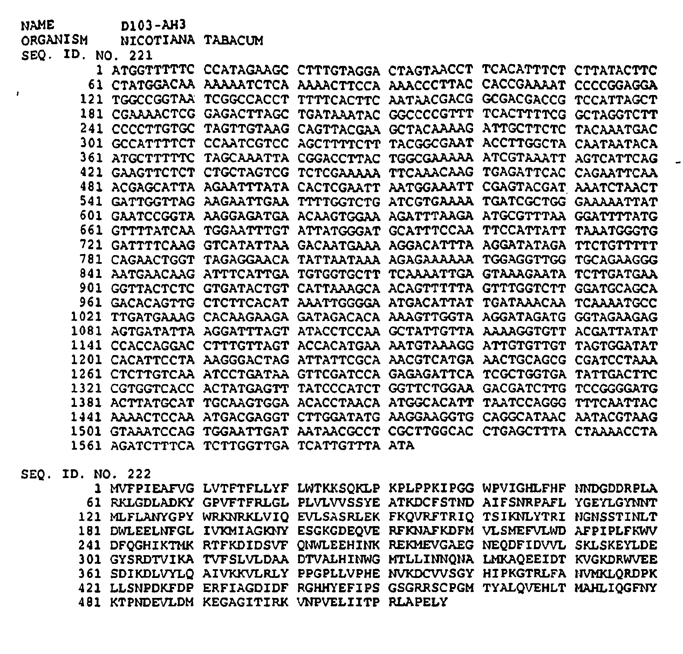

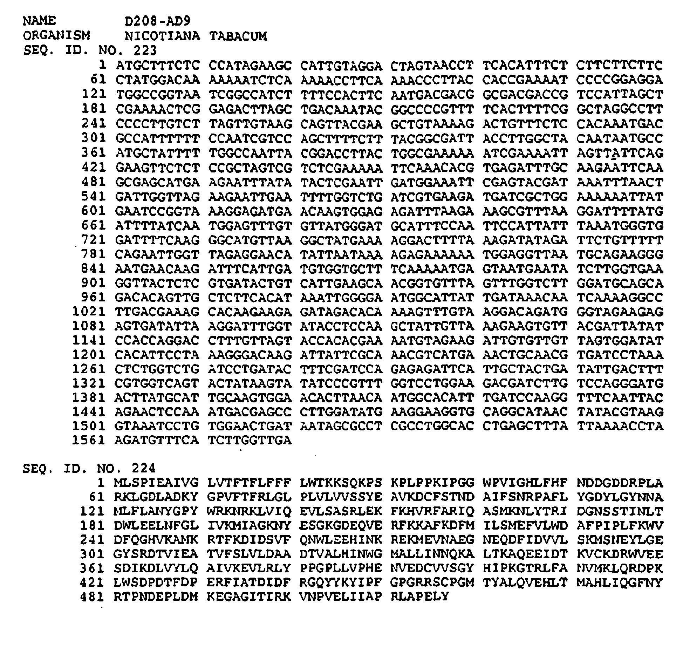

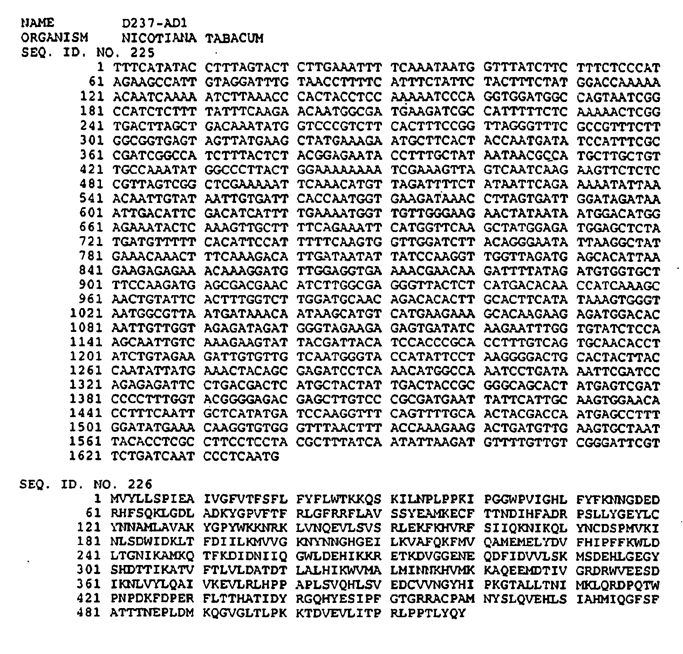

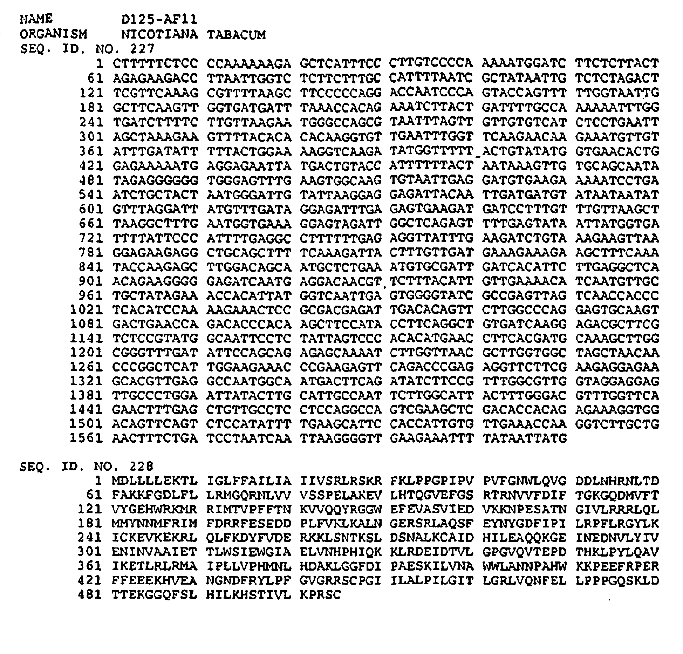

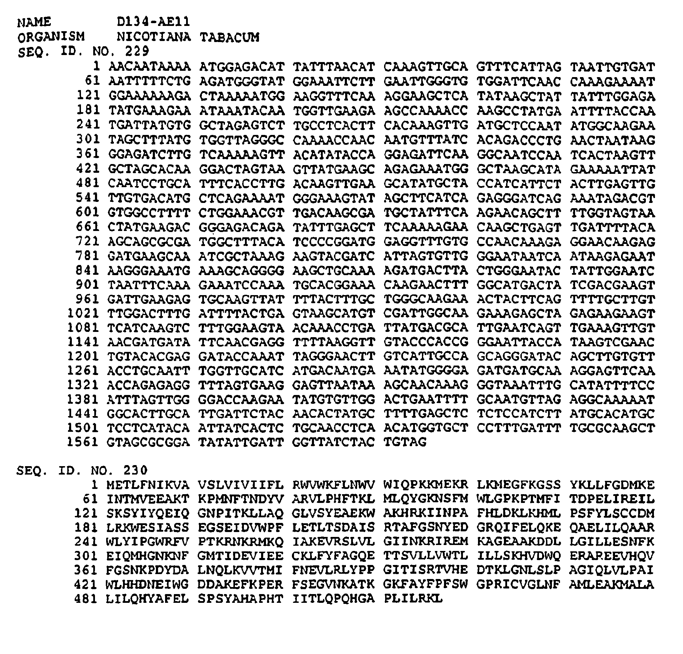

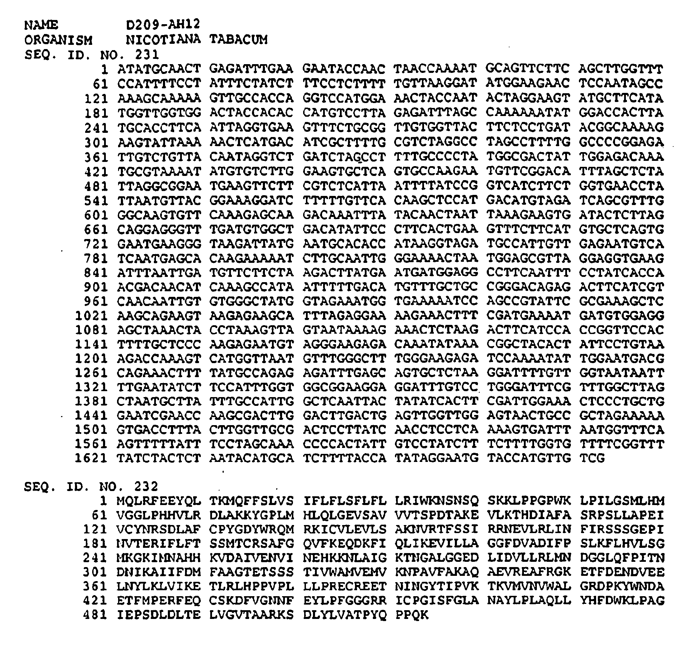

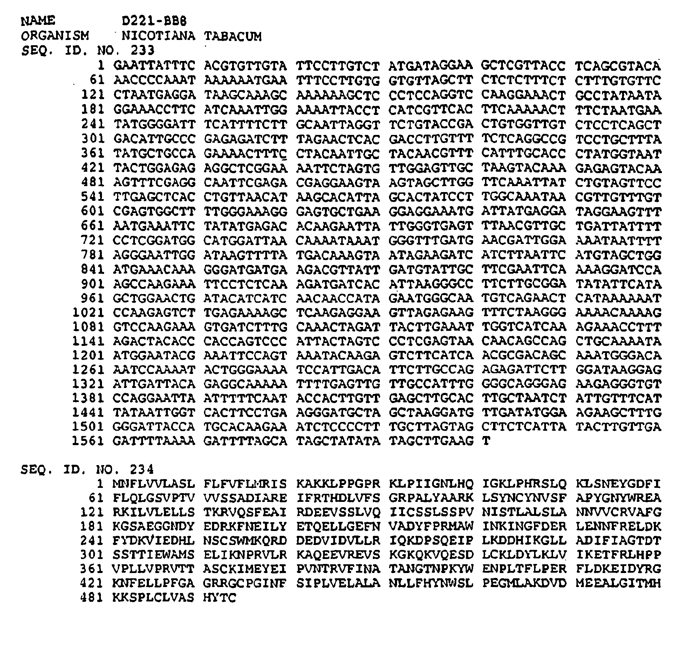

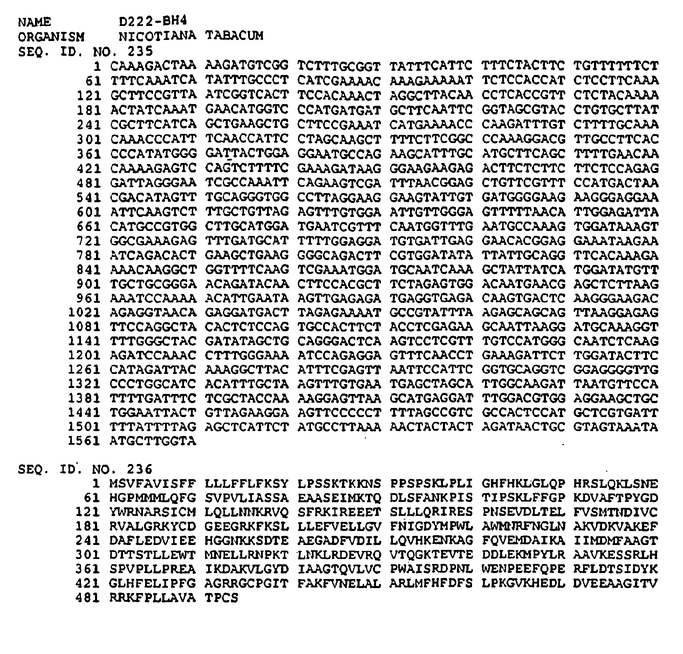

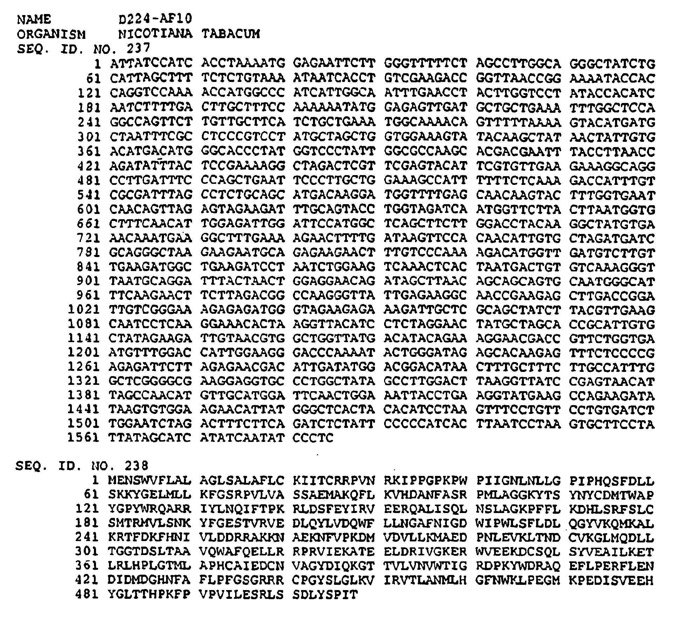

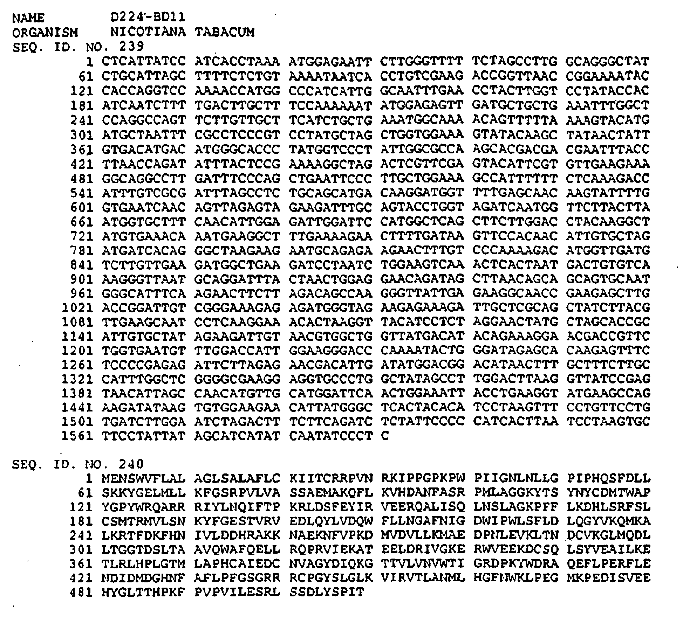

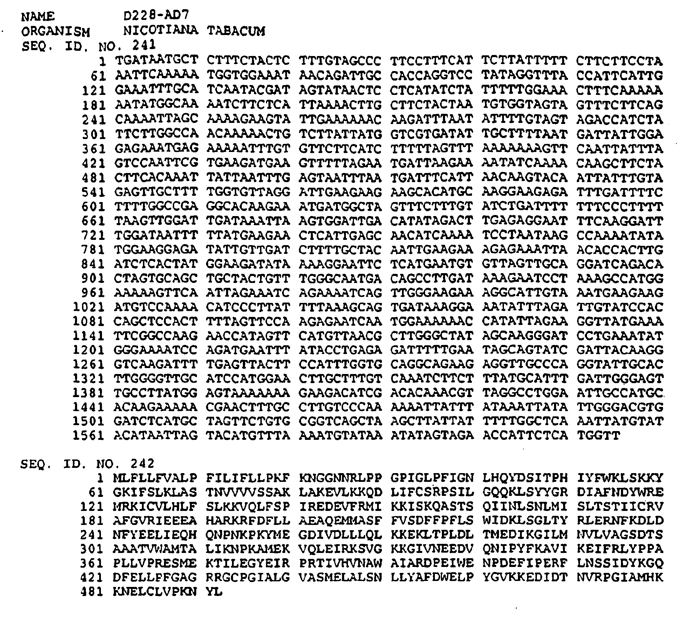

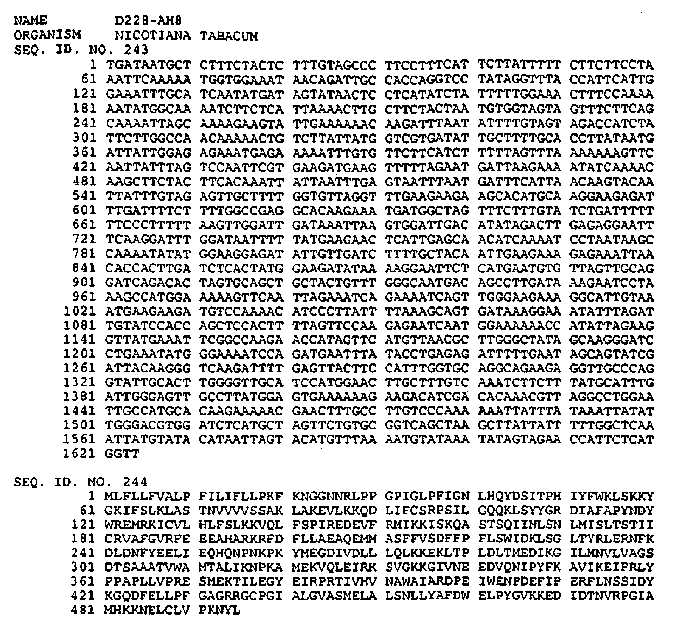

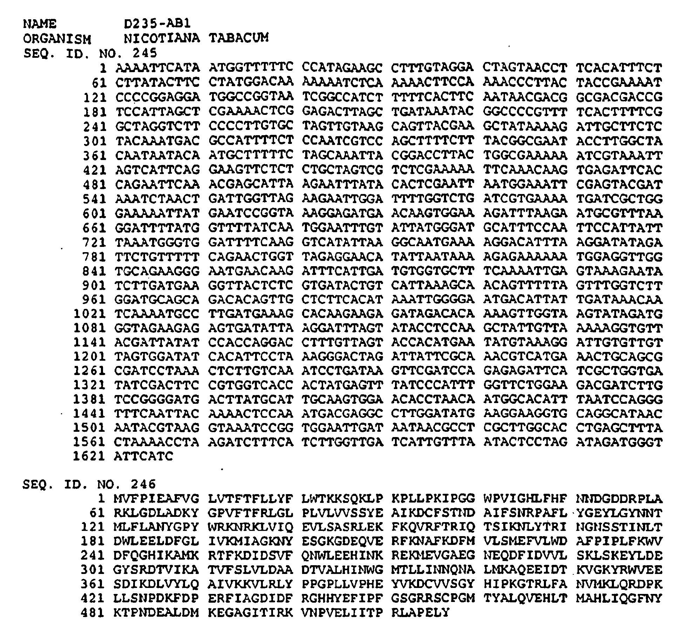

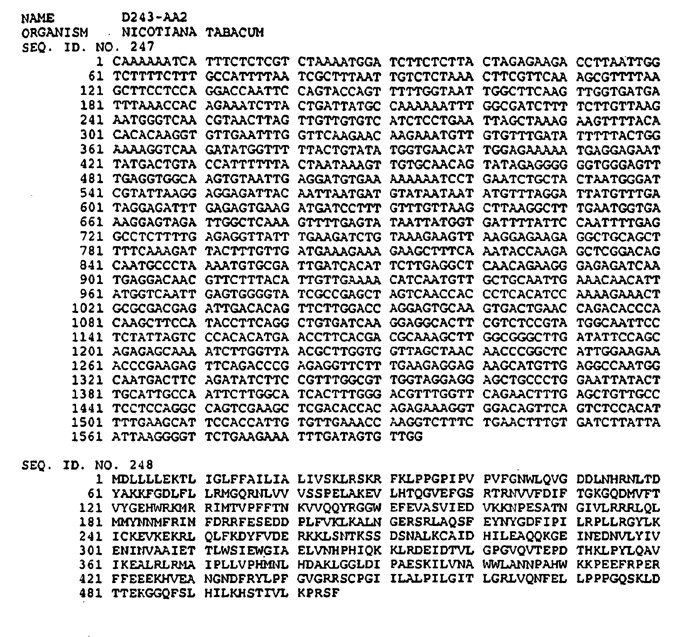

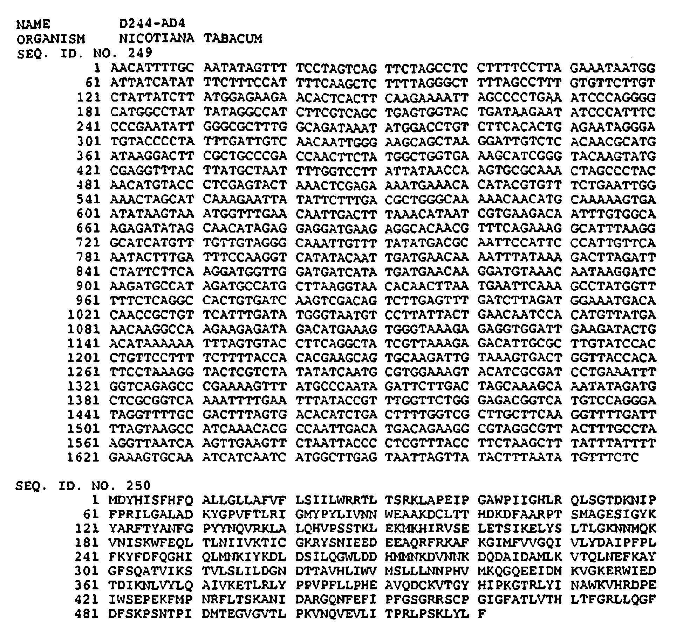

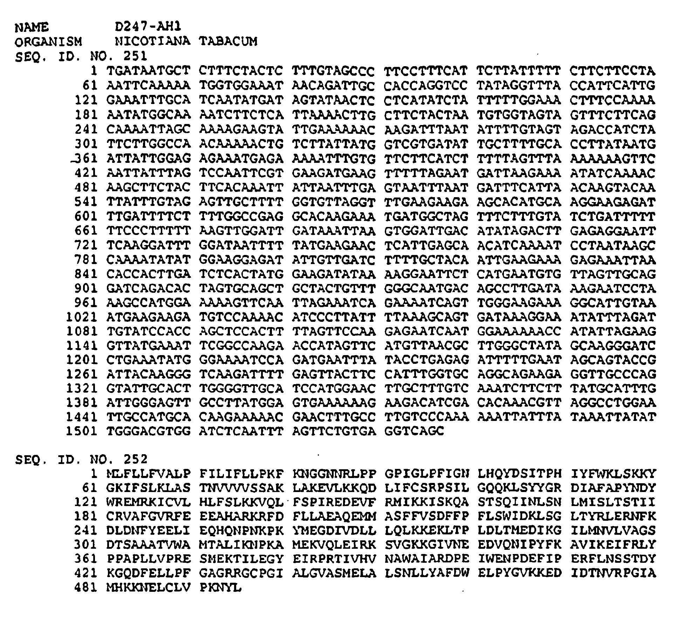

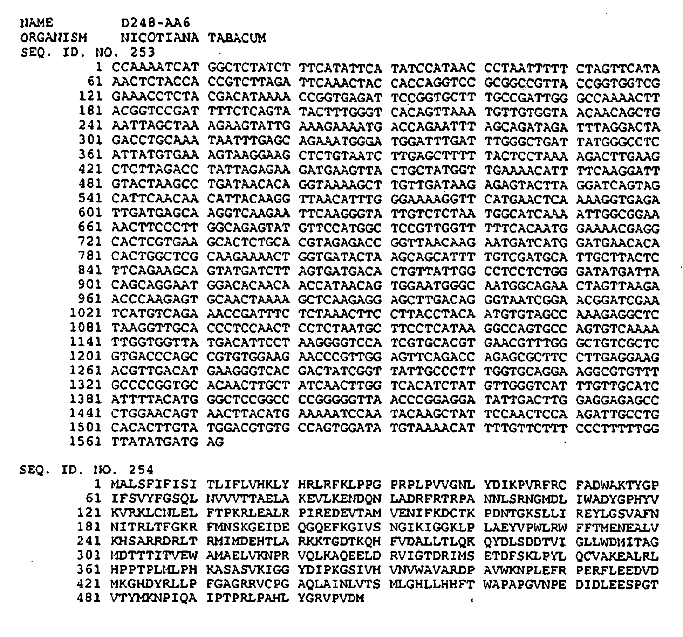

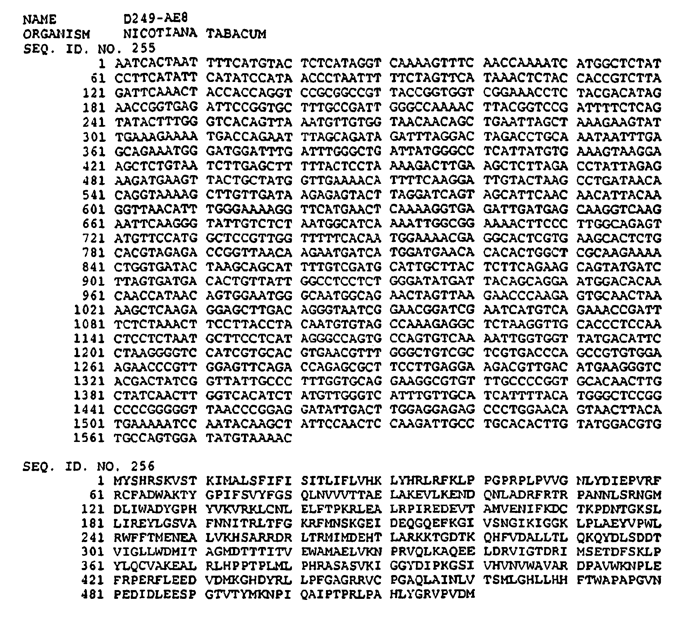

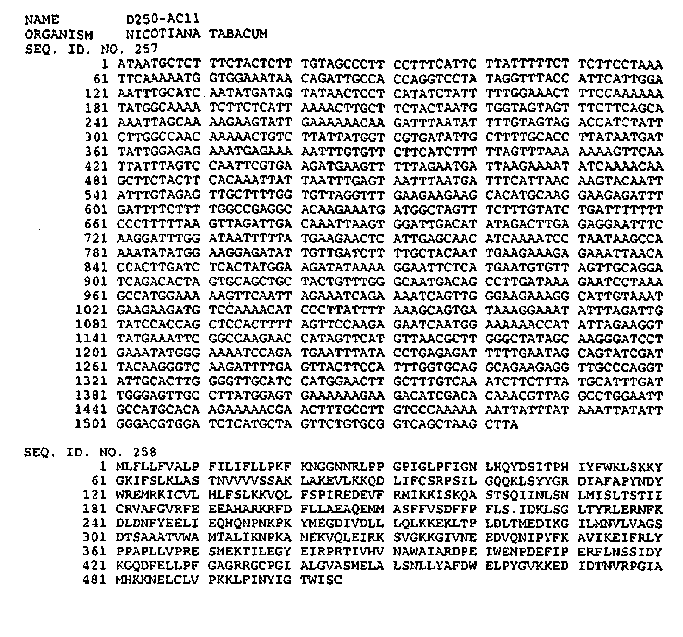

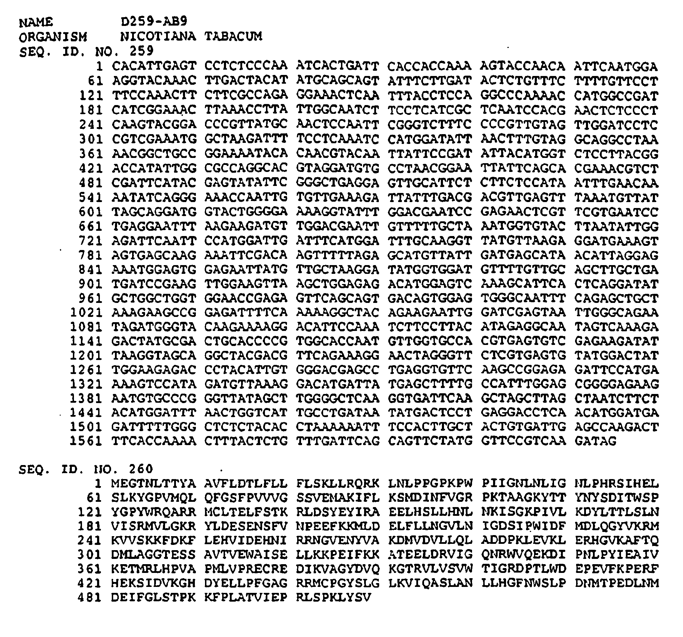

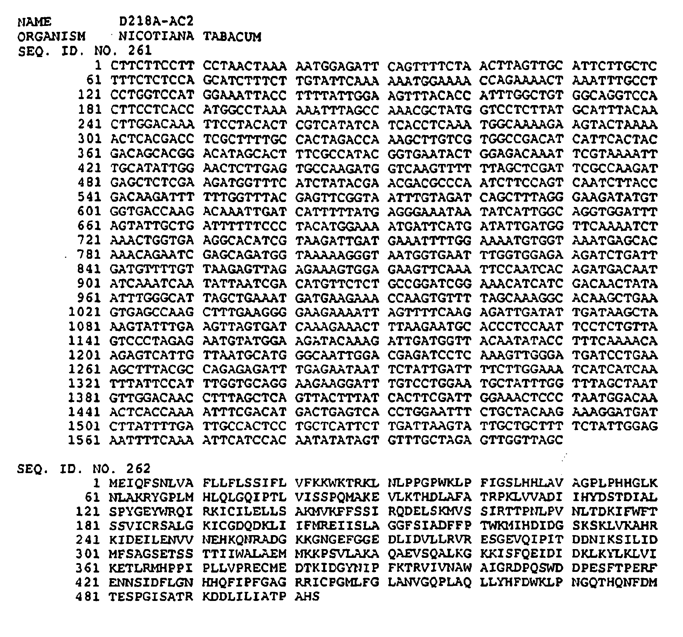

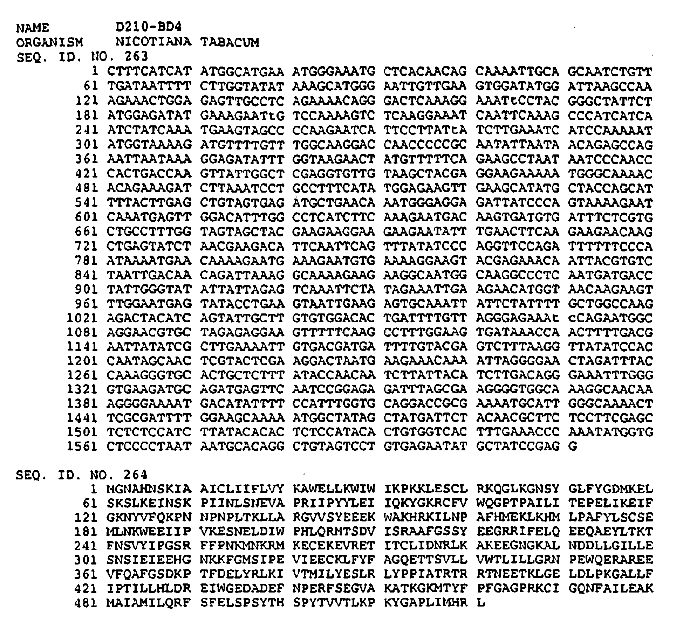

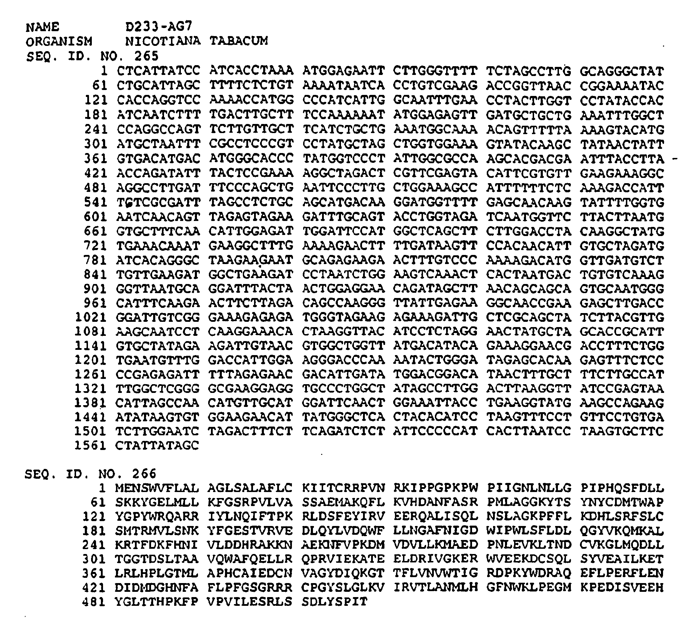

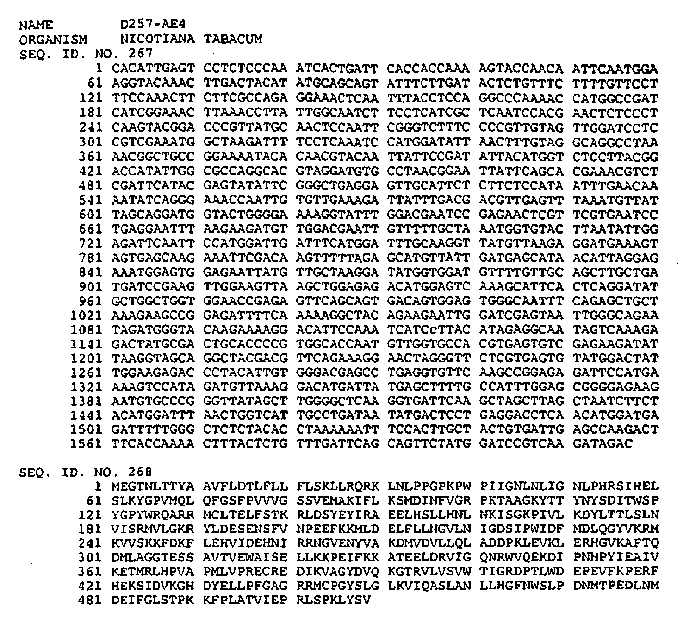

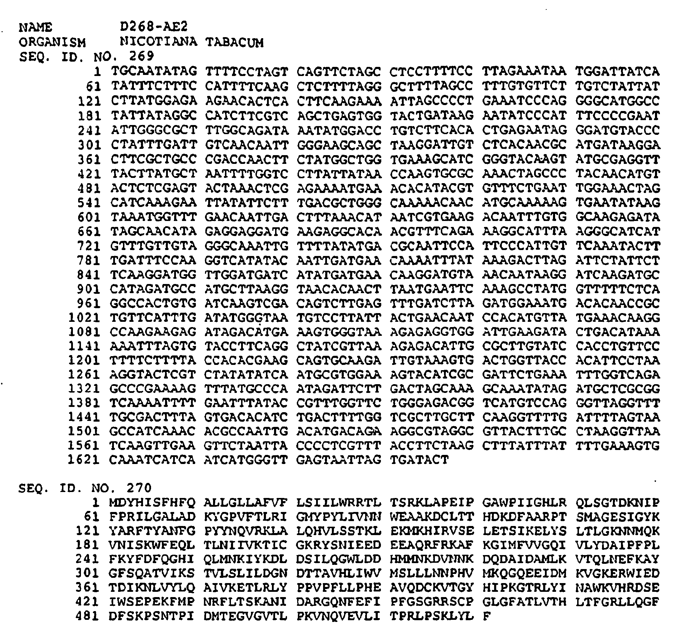

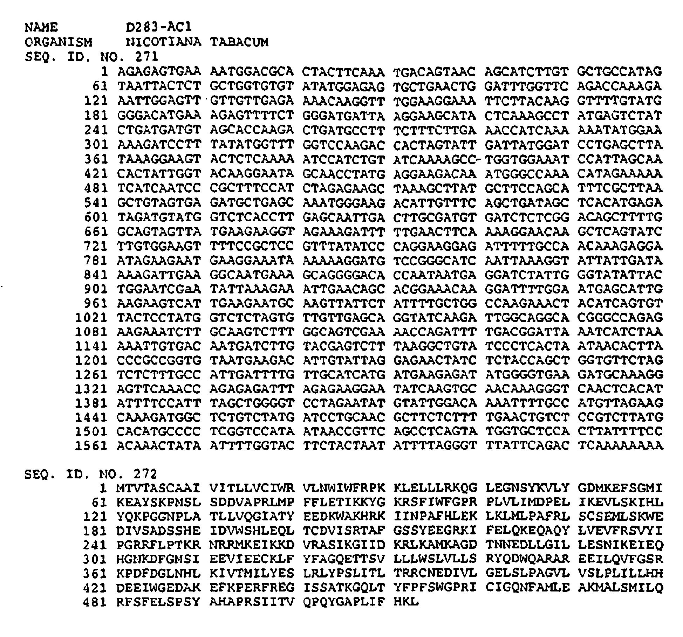

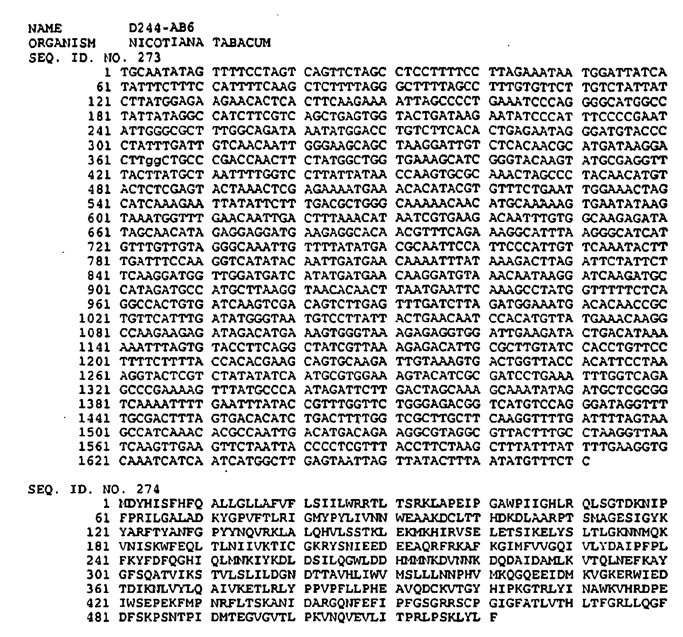

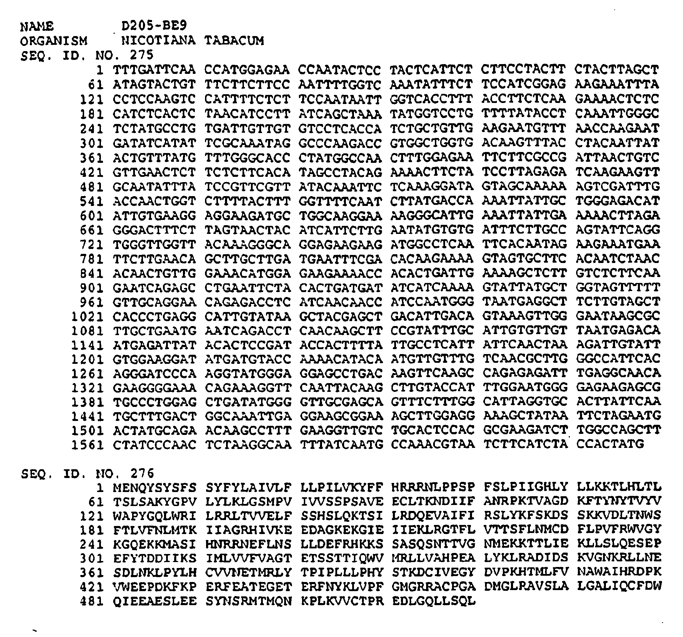

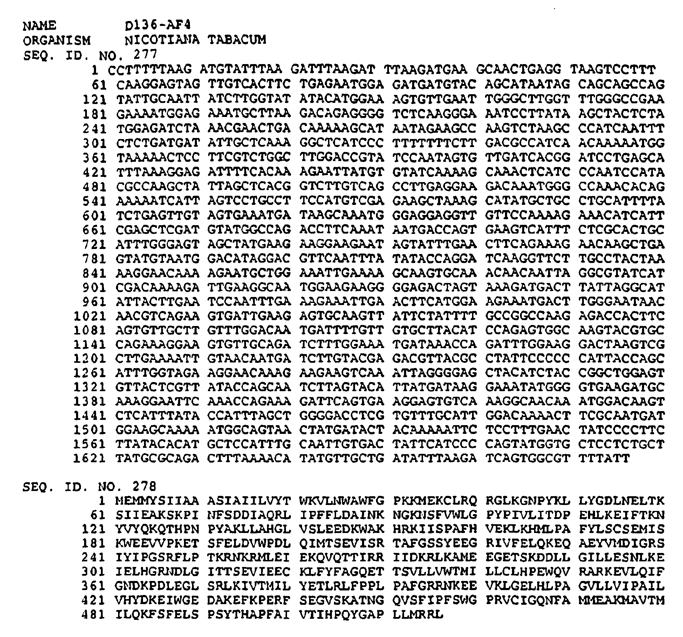

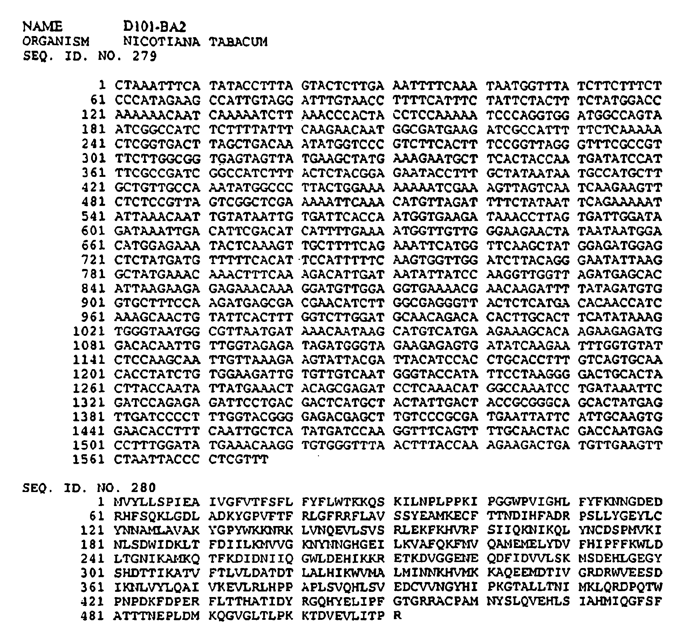

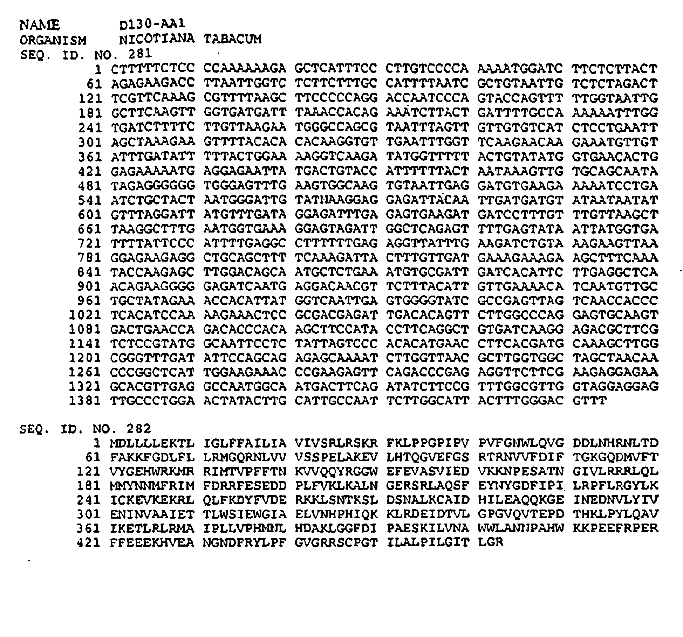

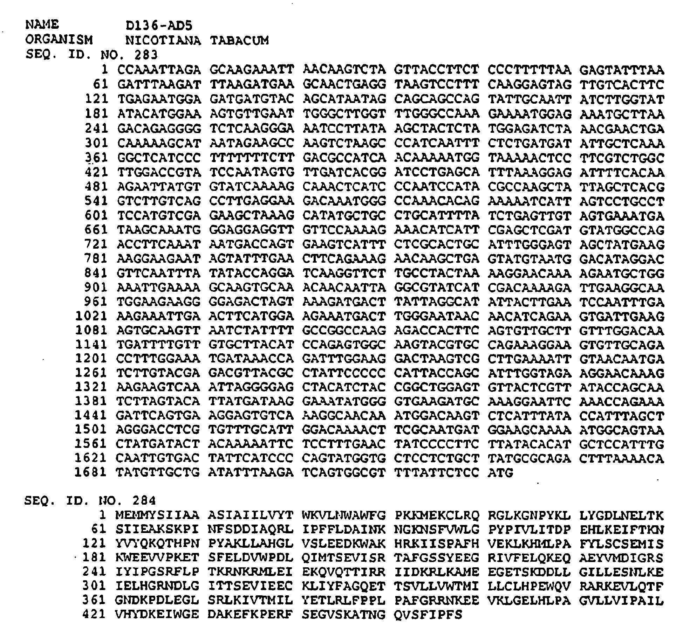

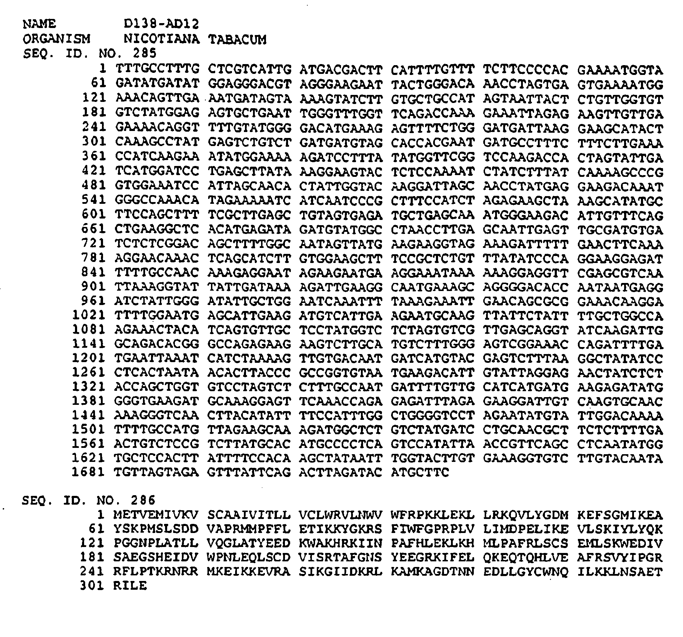

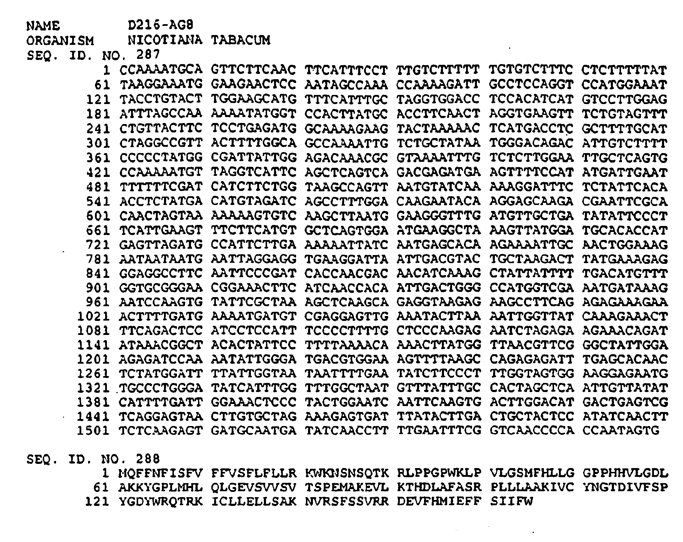

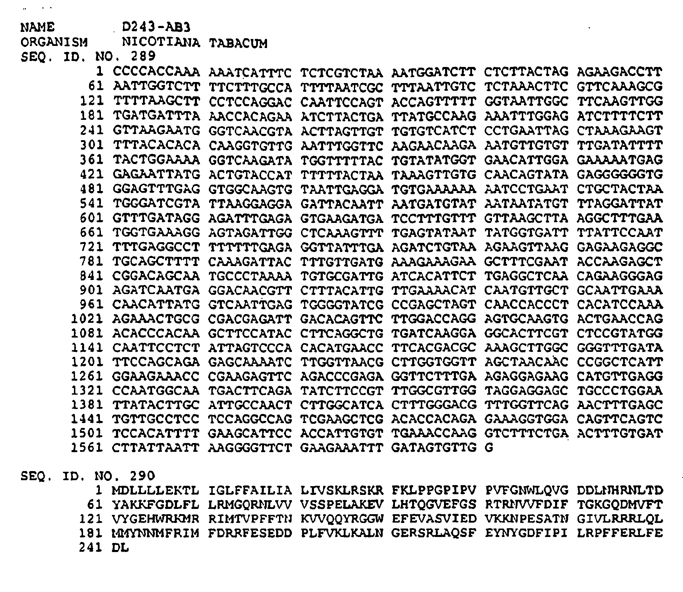

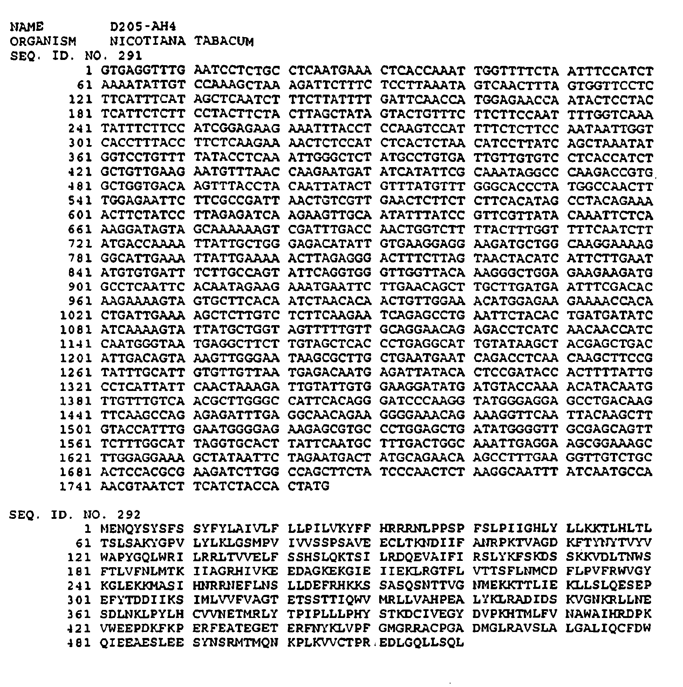

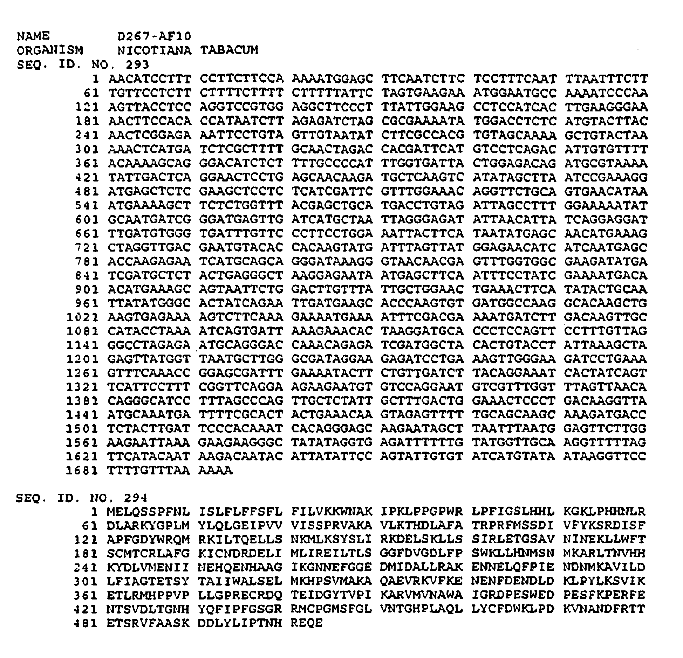

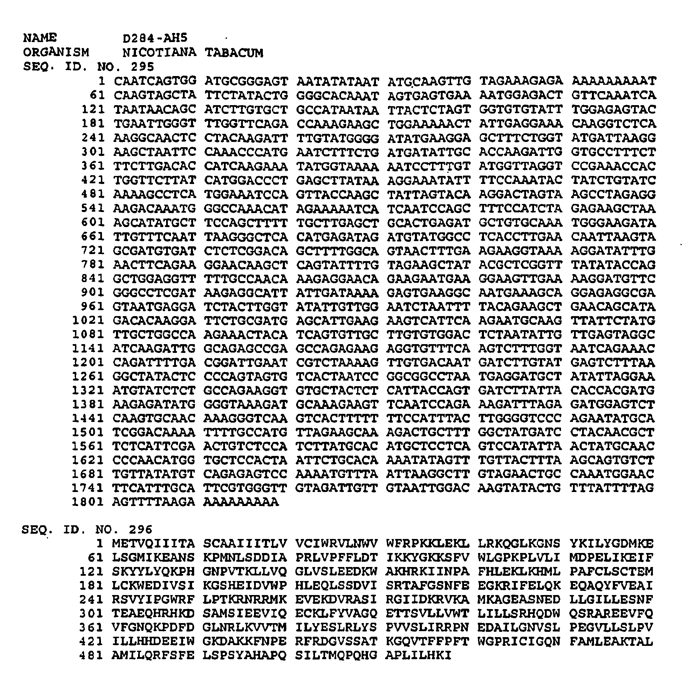

Figure 74 shows nucleic acid SEQ. ID. No.:147 and amino acid SEQ. ID. No.:148.
Figure 75 shows nucleic acid SEQ. ID No.: 149 and amino acid SEQ. ID. No.: 150.
Figure 76 shows nucleic acid SEQ. ID No.: 151 and amino acid SEQ. ID. No.: 152.
Figure 77 shows nucleic acid SEQ. ID No.: 153 and amino acid SEQ. ID. No.: 154.
Figure 78 shows nucleic acid SEQ. ID No.: 155 and amino acid SEQ. ID. No.: 156.
*Figure 79 sh*ows nucleic acid SEQ. ID No.: 157 and amino acid SEQ. ID. No.: 158.
Figure 80 shows nucleic acid SEQ. ID No.: 159 and amino acid SEQ. ID. No.: 160.
Figure 81 shows nucleic acid SEQ. ID No.: 161 and amino acid SEQ. ID. No.: 162.
Figure 82 shows nucleic acid SEQ. ID No.: 163 and amino acid SEQ. ID. No.: 164.
Figure 83 shows nucleic acid SEQ. ID No.: 165 and amino acid SEQ. ID. No.: 166.
Figure 84 shows nucleic acid SEQ. ID No.: 167 and amino acid SEQ. ID. No.: 168.
Figure 85 shows nucleic acid SEQ. ID No.: 169 and amino acid SEQ. ID. No.: 170.
Figure 86 shows nucleic acid SEQ. ID No.: 171 and amino acid SEQ. ID. No.: 172.
Figure 87 shows nucleic acid SEQ. ID No.: 173 and amino acid SEQ. ID. No.: 174.
Figure 88 shows nucleic acid SEQ. ID No.: 175 and amino acid SEQ. ID. No.: 176.
Figure 89 shows nucleic acid SEQ. ID No.: 177 and amino acid SEQ. ID. No.: 178.
Figure 90 shows nucleic acid SEQ. ID No.: 179 and amino acid SEQ. ID. No.: 180.
Figure 91 shows nucleic acid SEQ. ID No.: 181 and amino acid SEQ. ID. No.: 182.
Figure 92 shows nucleic acid SEQ. ID No.: 183 and amino acid SEQ. ID. No.: 184.
Figure 93 shows nucleic acid SEQ. ID No.: 185 and amino acid SEQ. ID. No.: 186.
Figure 94 shows nucleic acid SEQ. ID No.: 187 and amino acid SEQ. ID. No.: 188.
Figure 95 shows nucleic acid SEQ. ID No.: 189 and amino acid SEQ. ID. No.: 190.
*Figure 96* shows nucleic acid SEQ. ID No.: 191 and amino acid SEQ. ID. No.: 192.
Figure 97 shows nucleic acid SEQ. ID No.: 193 and amino acid SEQ. ID. No.: 194.
Figure 98 shows nucleic acid SEQ. ID No.: 195 and amino acid SEQ. ID. No.: 196.
Figure 99 shows nucleic acid SEQ. ID.No.: 197 and amino acid SEQ. ID. No.: 198.
Figure 100 shows nucleic acid SEQ. ID No.: 199 and amino acid SEQ. ID. No.: 200.
Figure 101 shows nucleic acid SEQ. ID No.: 201 and amino acid SEQ. ID. No.: 202.
Figure 102 shows nucleic acid SEQ. ID No.: 203 and amino acid SEQ. ID. No.: 204.
Figure 103 shows nucleic acid SEQ. ID No.: 205 and amino acid SEQ. ID. No.: 206.
Figure 104 shows nucleic acid SEQ. ID No.: 207 and amino acid SEQ. ID. No.: 208.
Figure 105 shows nucleic acid SEQ. ID No.: 209 and amino acid SEQ. ID. No.: 210.
Figure 106 shows nucleic acid SEQ. ID No.: 211 and amino acid SEQ. ID. No.: 212.
Figure 107 shows nucleic acid SEQ. ID No.: 213 and amino acid SEQ. ID. No.: 214.
Figure 108 shows nucleic acid SEQ. ID No.: 215 and amino acid SEQ. ID. No.: 216.
Figure 109 shows nucleic acid SEQ. ID No.: 217 and amino acid SEQ. ID. No.: 218.
Figure 110 shows nucleic acid SEQ. ID No.: 219 and amino acid SEQ. ID. No.: 220.
Figure 111 shows nucleic acid SEQ. ID No.: 221 and amino acid SEQ. ID. No.: 222.
Figure 112 shows nucleic acid SEQ. ID No.: 223 and amino acid SEQ. ID. No.: 224.
Figure 113 shows nucleic acid SEQ. ID No.: 225 and amino acid SEQ. ID. No.: 226.
Figure 114 shows nucleic acid SEQ. ID No.: 227 and amino acid SEQ. ID. No.: 228.
Figure 115 shows nucleic acid SEQ. ID No.: 229 and amino acid SEQ. ID. No.: 230.
Figure 116 shows nucleic acid SEQ. ID No.: 231 and amino acid SEQ. ID. No.: 232.
Figure 117 shows nucleic acid SEQ. ID No.: 233 and amino acid SEQ. ID. No.: 234.
Figure 118 shows nucleic acid SEQ. ID No.: 235 and amino acid SEQ. ID. No.: 236.
Figure 119 shows nucleic acid SEQ. ID No.: 237 and amino acid SEQ. ID. No.: 238.
Figure 120 shows nucleic acid SEQ. ID No.: 239 and amino acid SEQ. ID. No.: 240.
Figure 121 shows nucleic acid SEQ. ID No.: 241 and amino acid SEQ. ID. No.: 242.
Figure 122 shows nucleic acid SEQ. ID No.: 243 and amino acid SEQ. ID. No.: 244.
Figure 123 shows nucleic acid SEQ. ID No.: 245 and amino acid SEQ. ID. No.: 246.
Figure 124 shows nucleic acid SEQ. ID No.: 247 and amino acid SEQ. ID. No.: 248.
Figure 125 shows nucleic acid SEQ. ID No.: 249 any "amino acid SEQ. ID. No.: 250.
Figure 126 shows nucleic acid SEQ. ID No.: 251 and amino acid SEQ. ID. No.: 252.
Figure 127 shows nucleic acid SEQ. ID No.: 253 and amino acid SEQ. ID. No.: 254.
Figure 128 shows nucleic acid SEQ. ID No.: 255 and amino acid SEQ. ID. No.: 256.
Figure 129 shows nucleic acid SEQ. ID No.: 257 and amino acid SEQ. ID. No.: 258.
Figure 130 shows nucleic acid SEQ. ID No.: 259 and amino acid SEQ. ID. No.: 260.
Figure 131 shows nucleic acid SEQ. ID No.: 261 and amino acid SEQ. ID. No.: 262.
Figure 132 shows nucleic acid SEQ. ID No.: 263 and amino acid SEQ. ID. No.: 264.
Figure 133 shows nucleic acid SEQ. ID No.: 265 and amino acid SEQ. ID. No.: 266.
Figure 134 shows nucleic acid SEQ. ID No.: 267 and amino acid SEQ. ID. No.: 268.
Figure 135 shows nucleic acid SEQ. ID No.: 269 and amino acid SEQ. ID. No.: 270.
Figure 136 shows nucleic acid SEQ. ID No.: 271 and amino acid SEQ. ID. No.: 272.
Figure 137 shows nucleic acid SEQ. ID No.: 273 and amino acid SEQ. ID. No.: 274.
Figure 138 shows nucleic acid SEQ. ID No.: 275 and amino acid SEQ. ID. No.: 276.
Figure 139 shows nucleic acid SEQ. ID No.: 277 and amino acid SEQ. ID. No.: 278.
Figure 140 shows nucleic acid SEQ. ID No.: 279 and amino acid SEQ. ID. No.: 280.
Figure 141 shows nucleic acid SEQ. ID No.: 281 and amino acid SEQ. ID. No.: 282.
Figure 142 shows nucleic acid SEQ. ID No.: 283 and amino acid SEQ. ID. No.: 284.
Figure 143 shows nucleic acid SEQ. ID No.: 285 and amino acid SEQ. ID. No.: 286.
Figure 144 shows nucleic acid SEQ. ID No.: 287 and amino acid SEQ. ID. No.: 288.
Figure 145 shows nucleic acid SEQ. ID No.: 289 and amino acid SEQ. ID. No.: 290.
Figure 146 shows nucleic acid SEQ. ID No.: 291 and amino acid SEQ. ID. No.: 292.
Figure 147 shows nucleic acid SEQ. ID No.: 293 and amino acid SEQ. ID. No.: 294.
Figure 148 shows nucleic acid SEQ. ID No.: 295 and amino acid SEQ. ID. No.: 296.

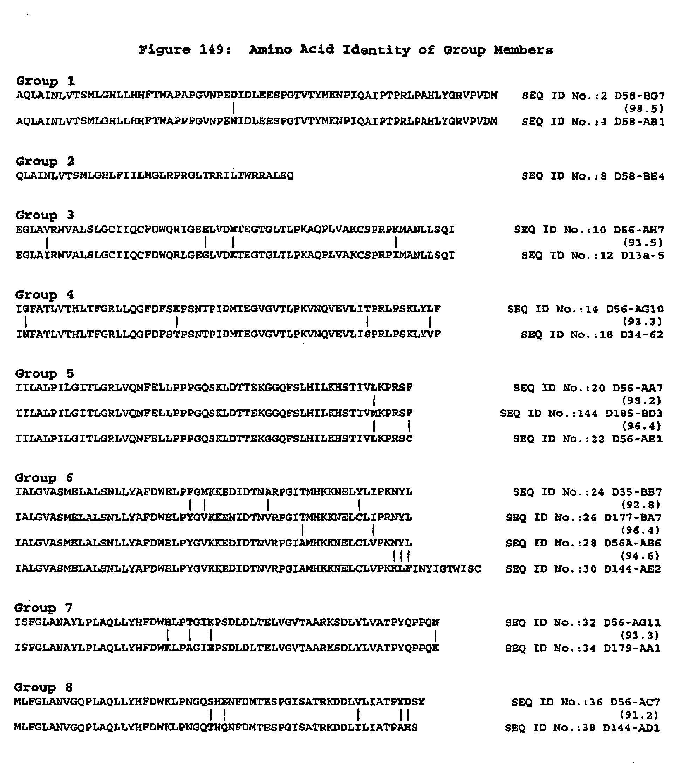

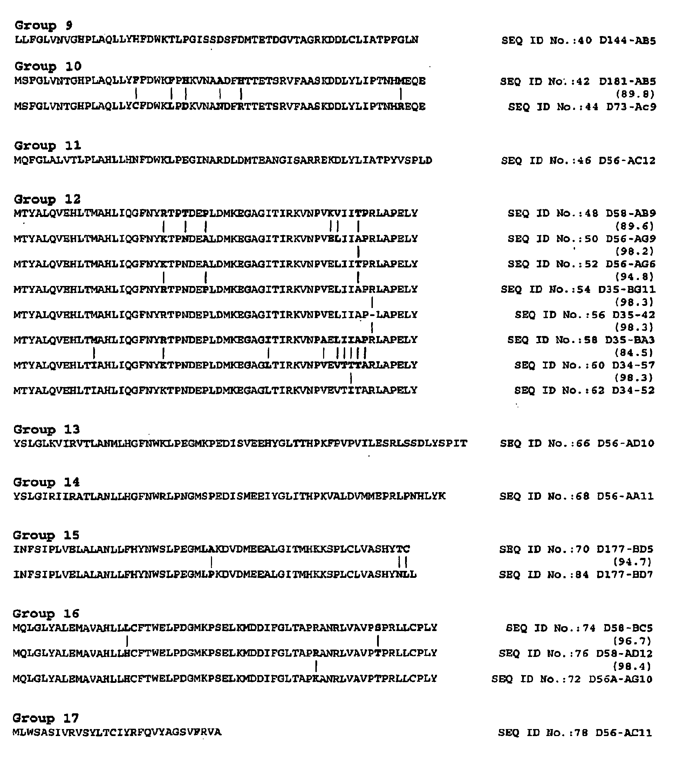

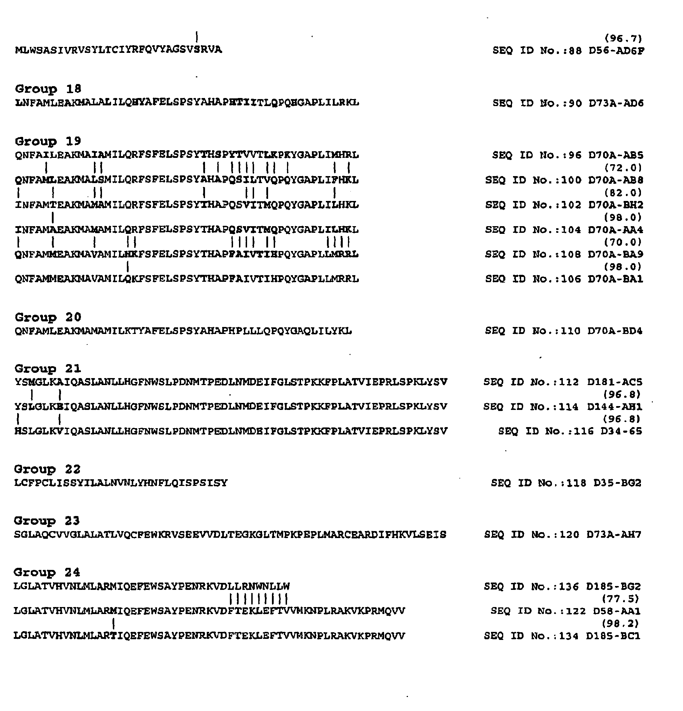

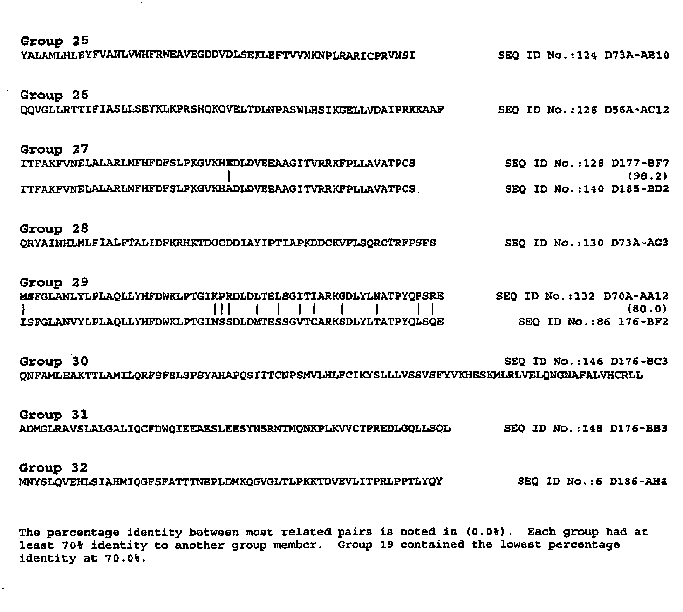

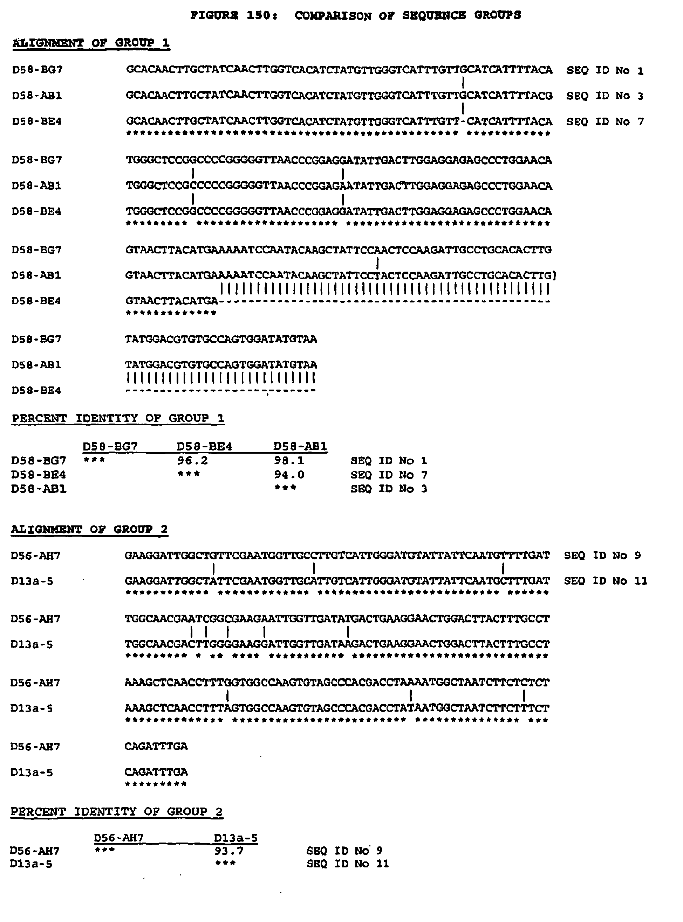

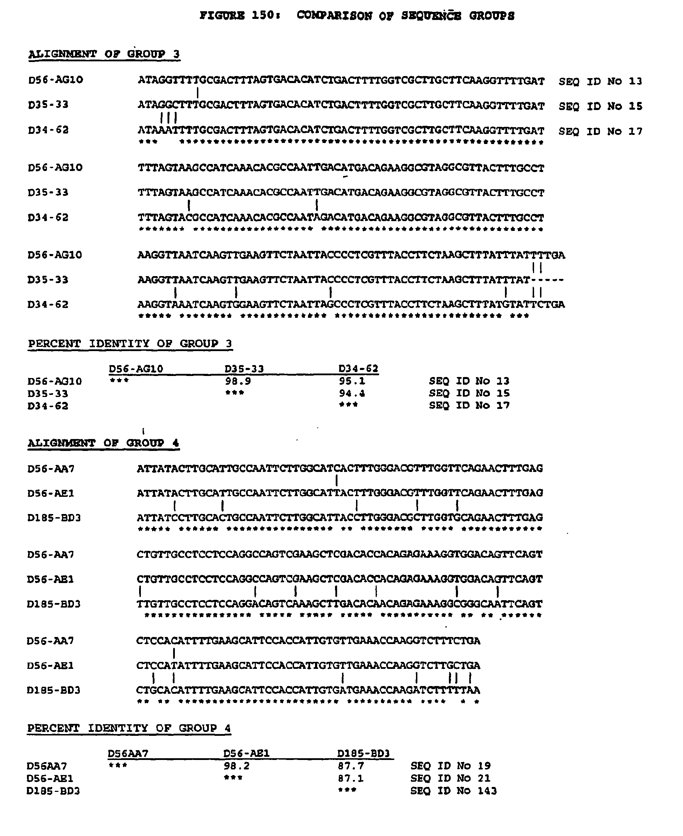

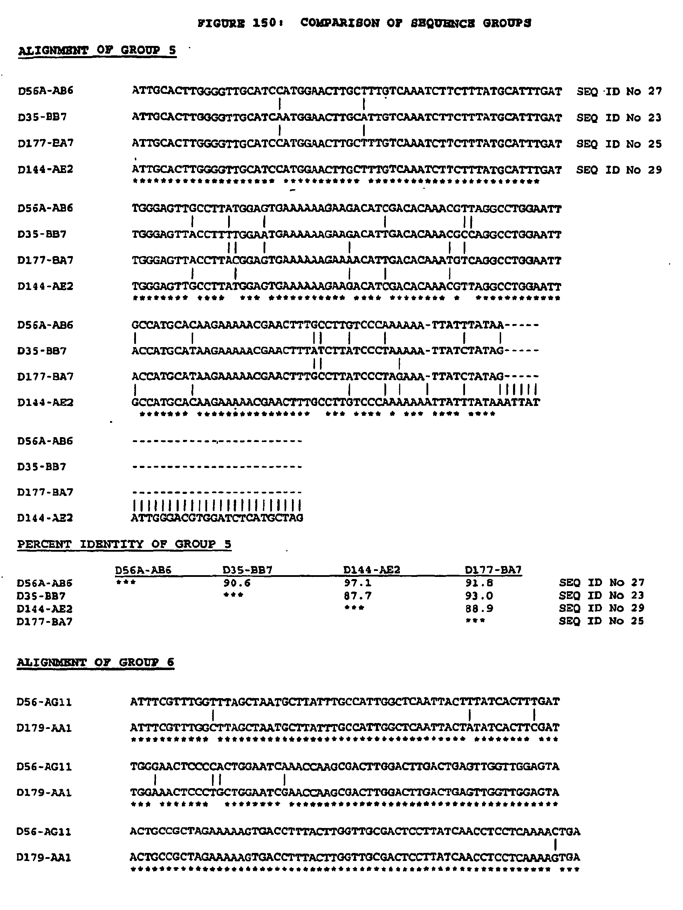

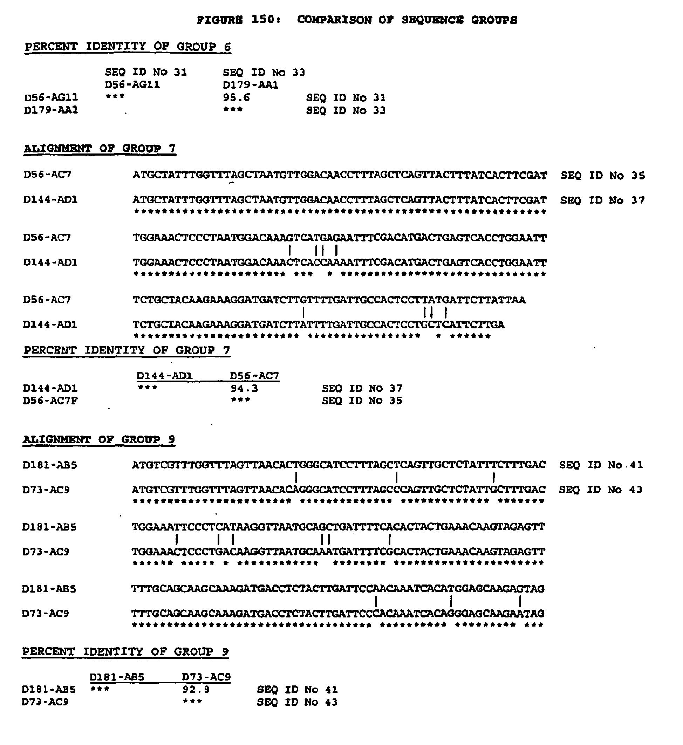

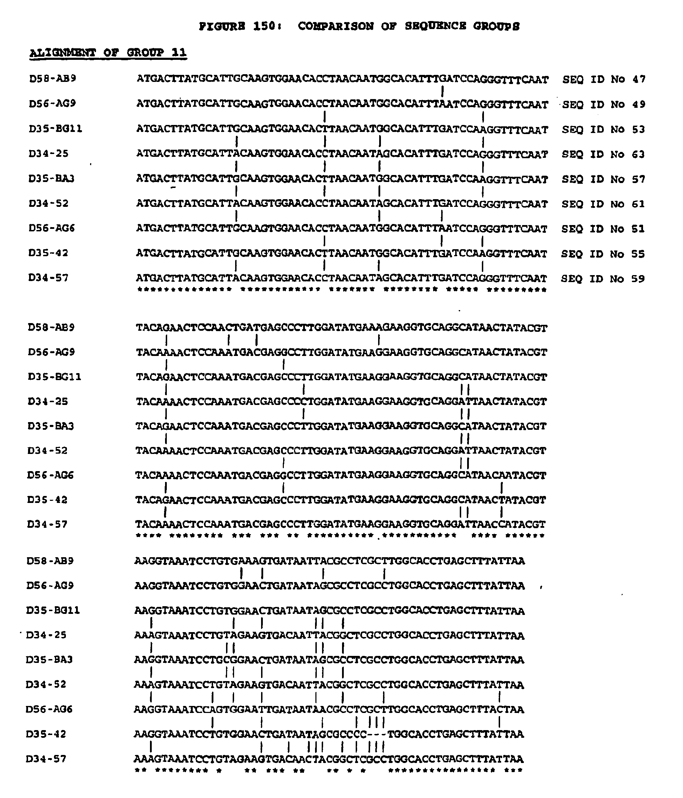

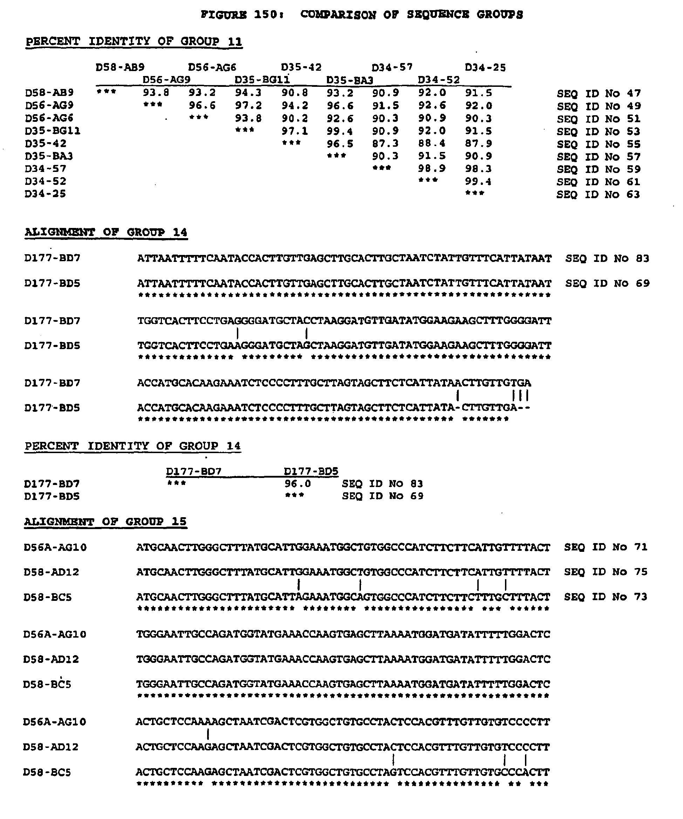

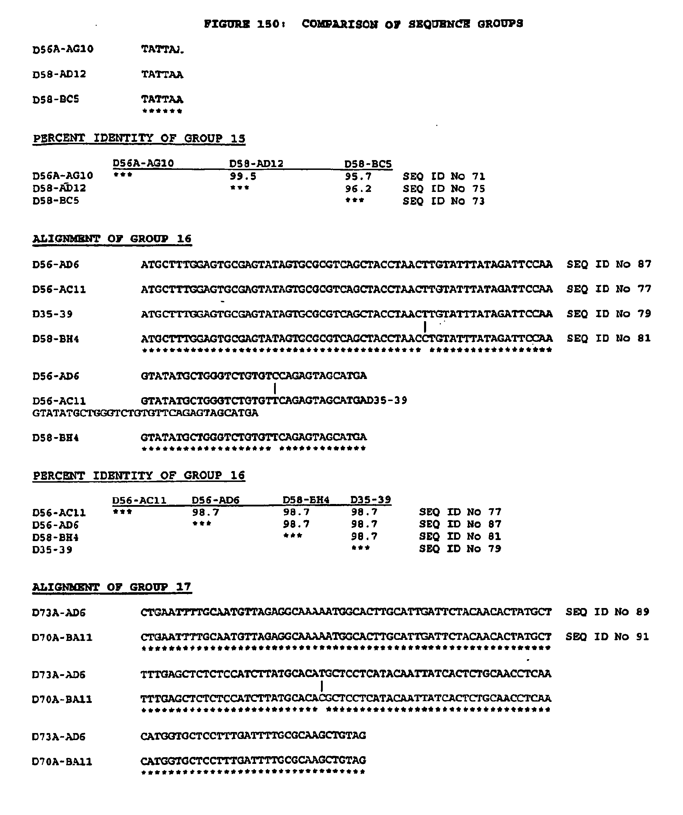

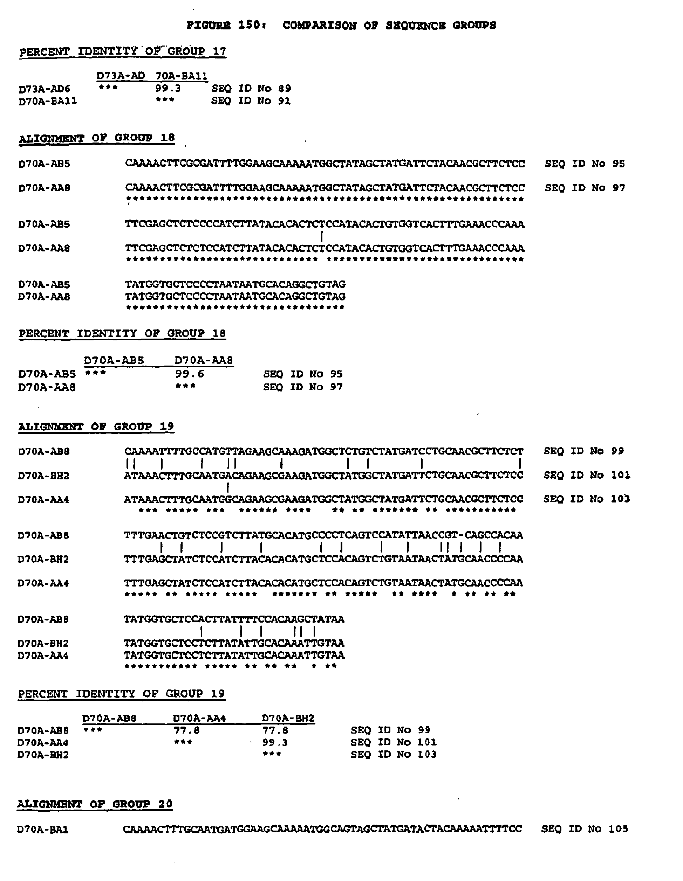

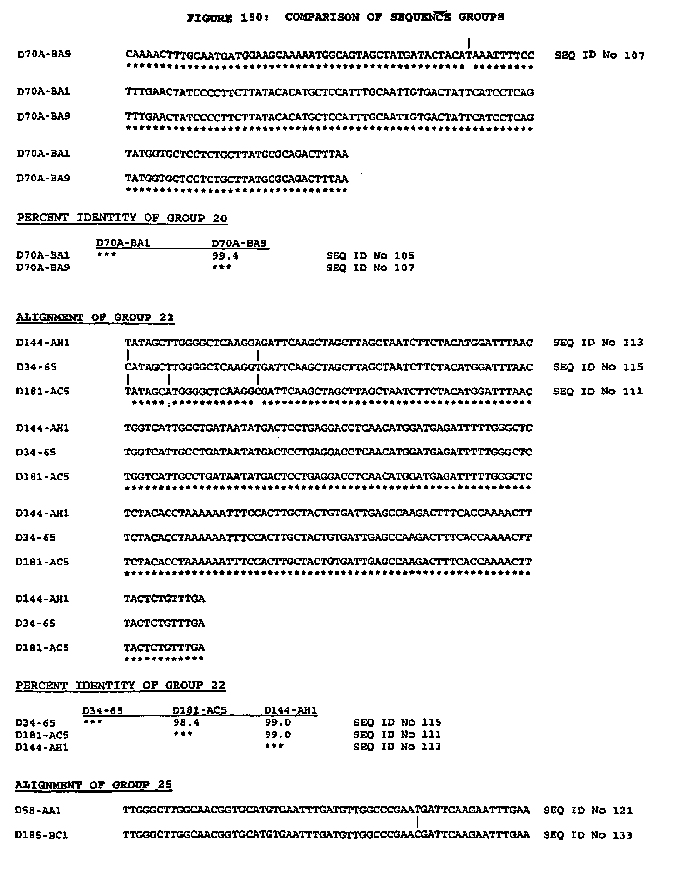

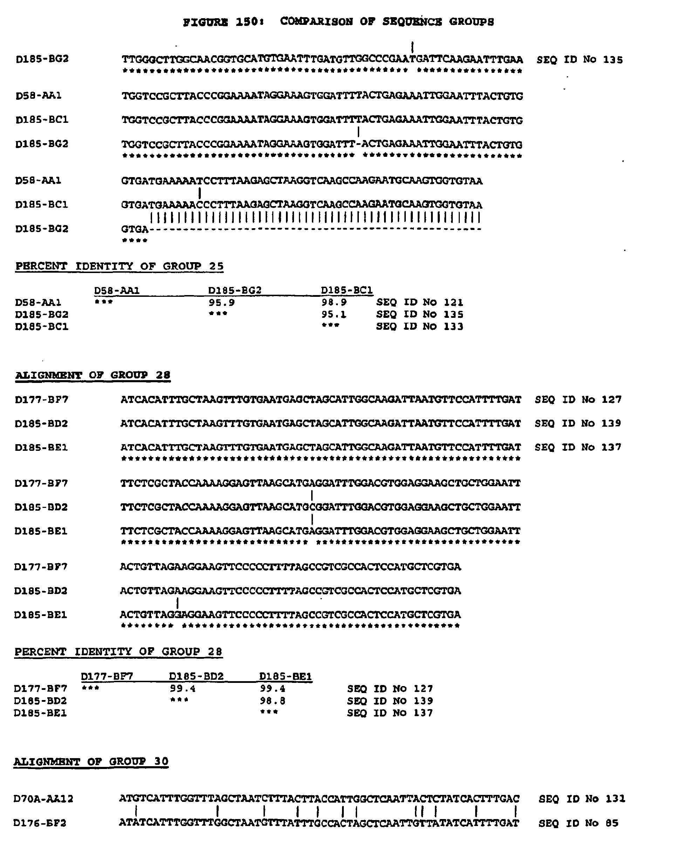

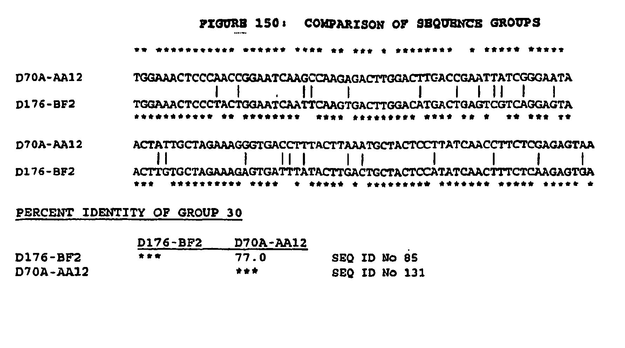

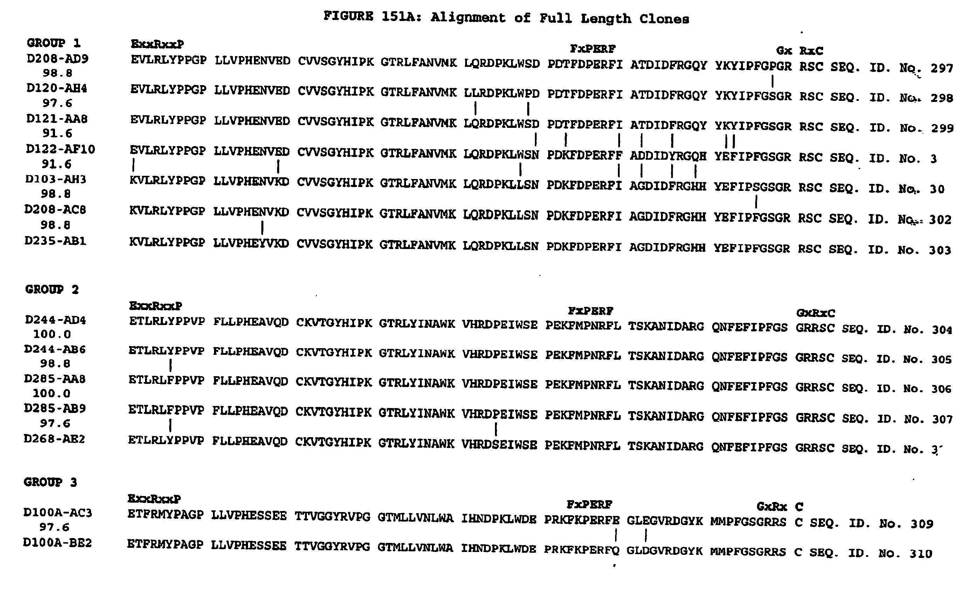

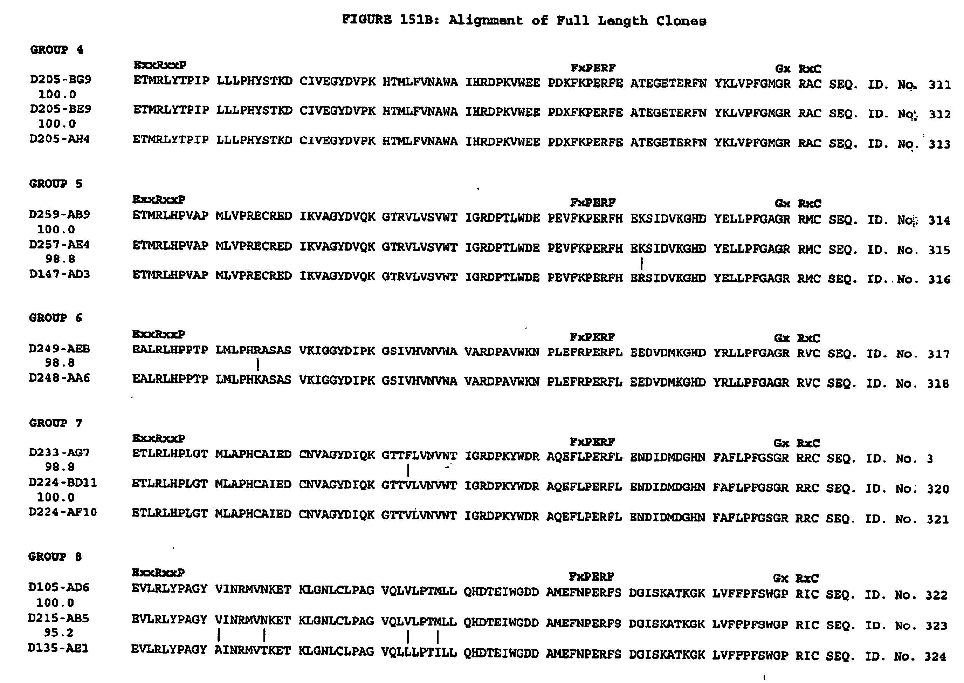

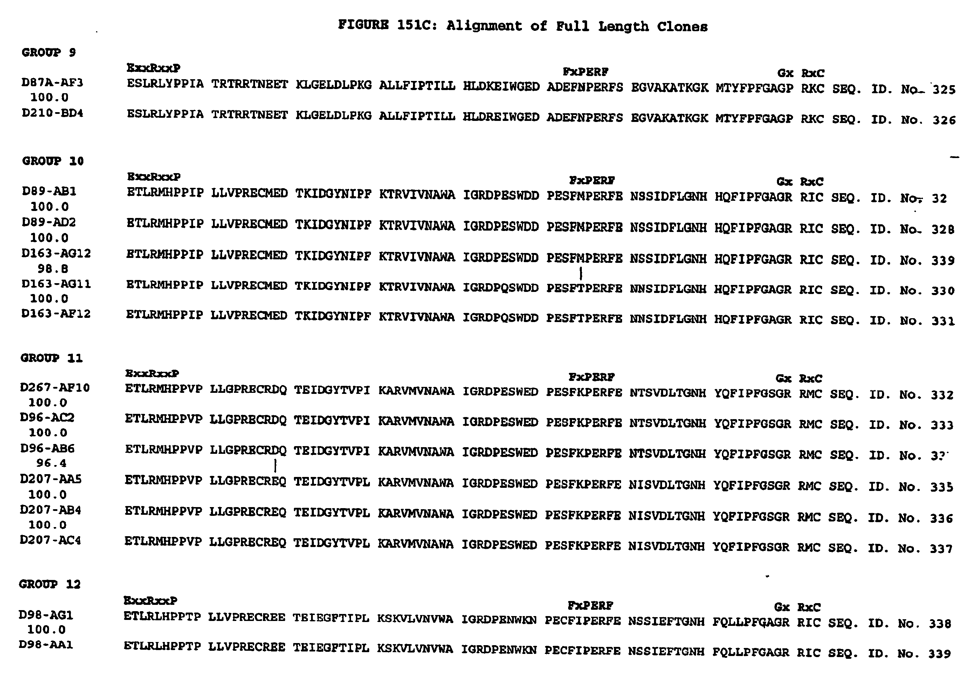

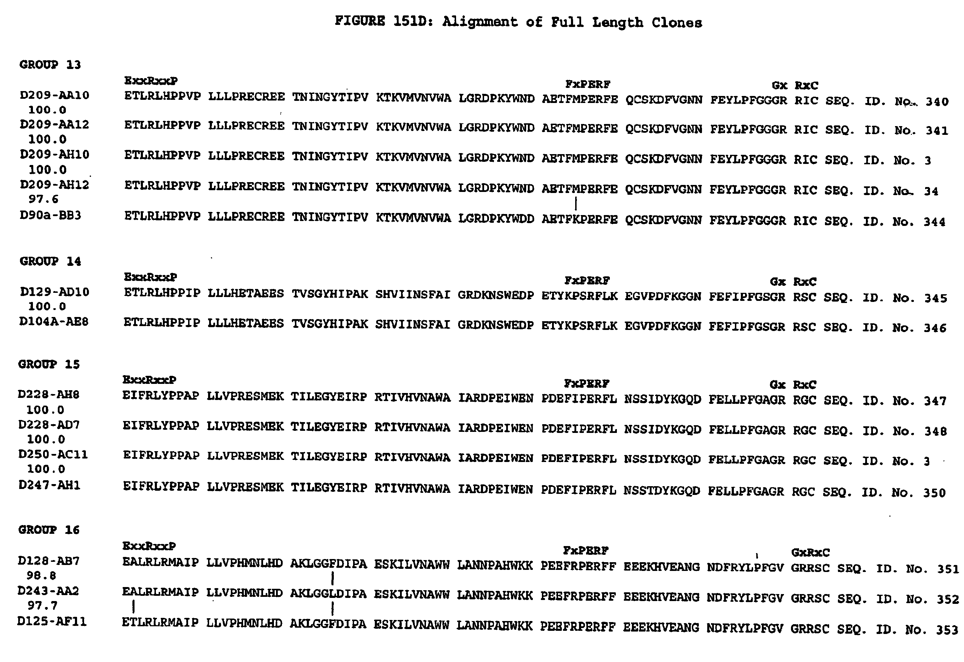

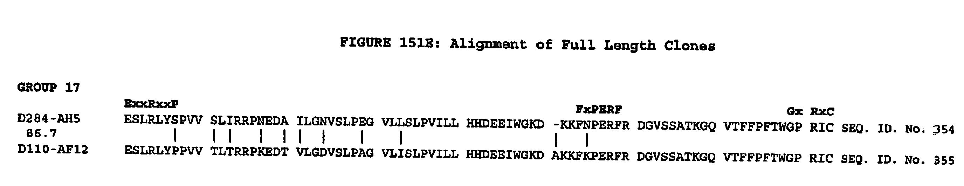

Figure 149 shows nucleic acid SEQ. ID No.: 297 and amino acid SEQ. ID. No.: 298. Figure 151 shows a comparison of Sequence Groups.

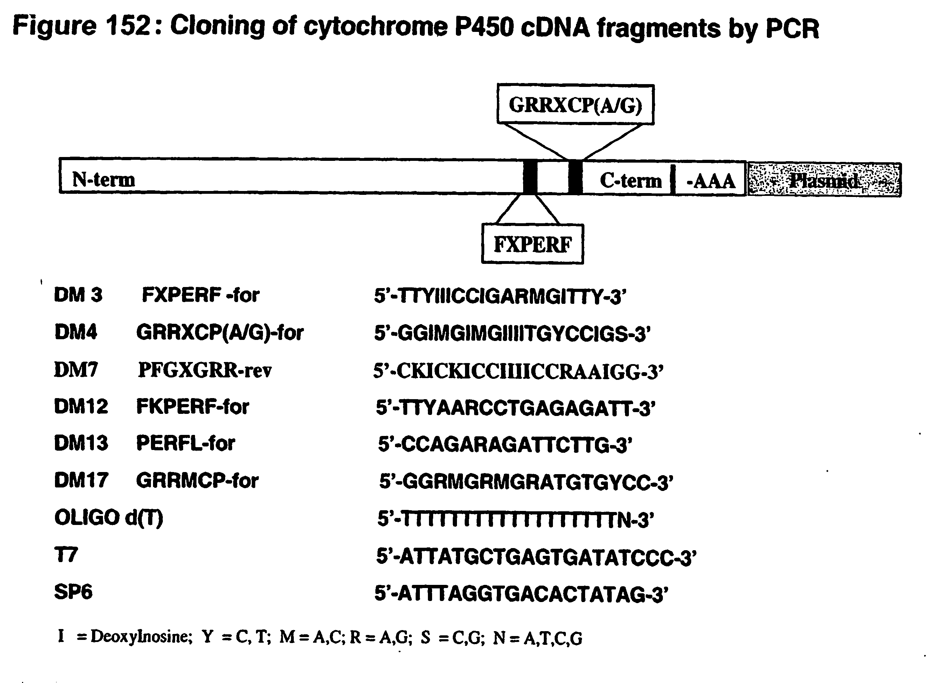

Figure 152 illustrates alignment of full length clones.
Figure 153 shows a procedure used for cloning of cytochrome p450 cDNA fragments by PCR

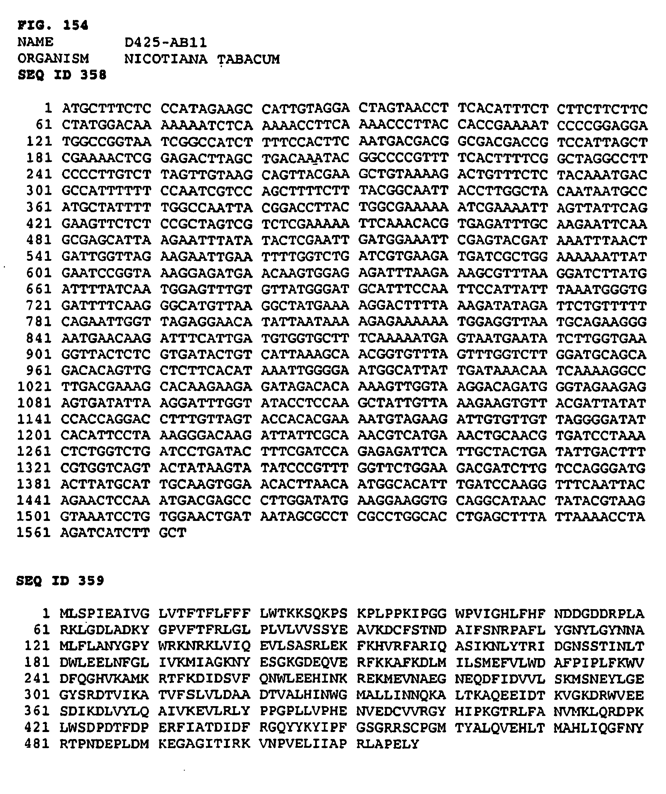

Figure 154 shows nucleic acid SEQ. ID No.: 299 and amino acid SEQ. ID. No.: 300.

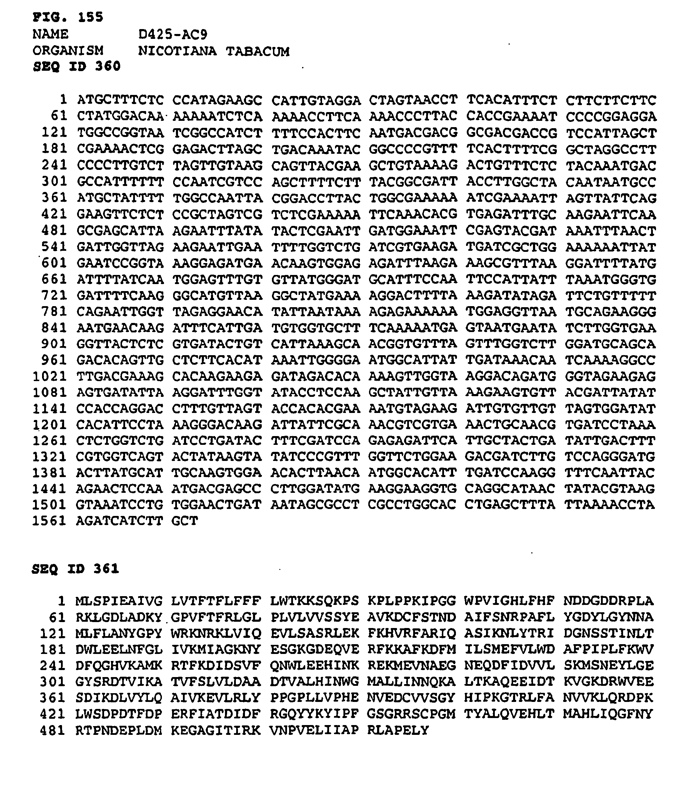

Figure 155 shows nucleic acid SEQ. ID No.: 301 and amino acid SEQ. ID. No.: 302.

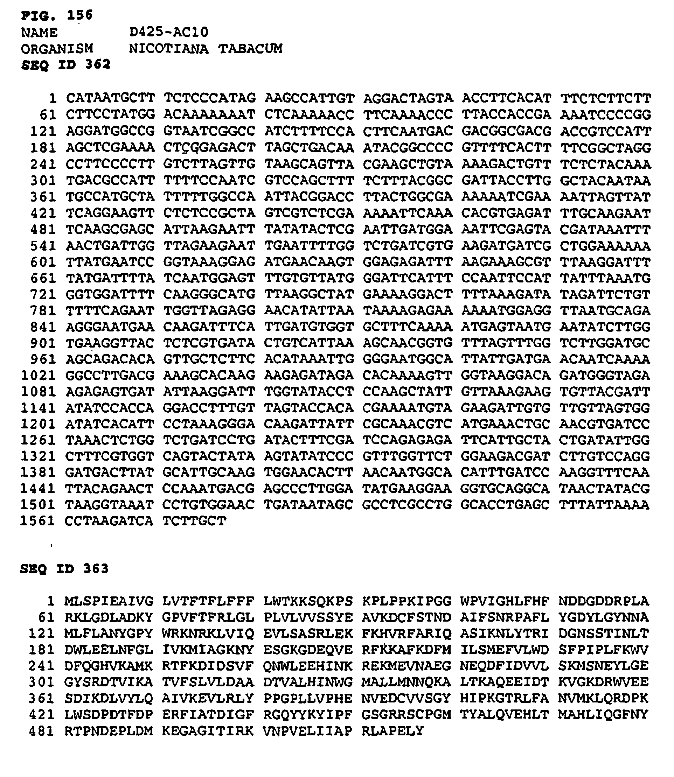

Figure 156 shows nucleic acid SEQ. ID No.: 303 and amino acid SEQ. ID. No.: 304.

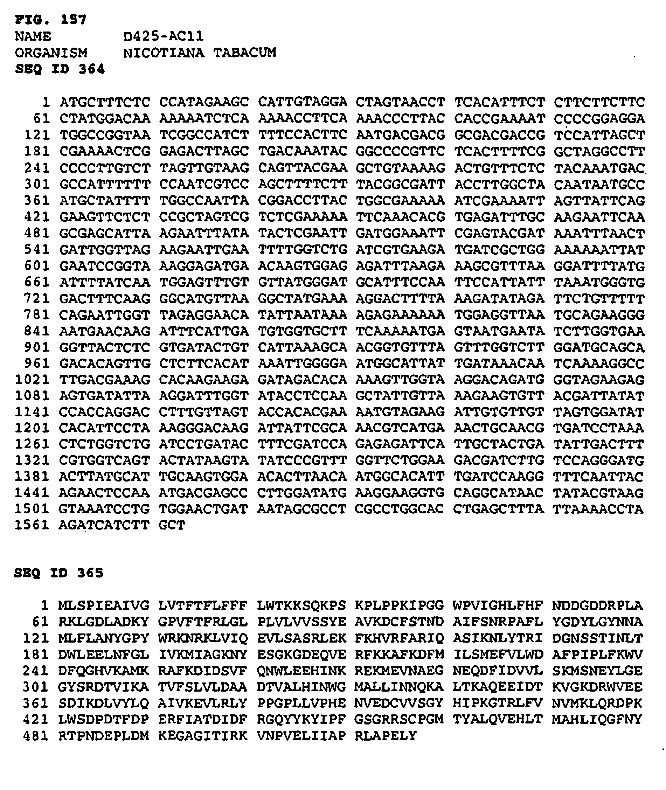

Figure 157 shows nucleic acid SEQ. ID No.: 305 and amino acid SEQ. ID. No.: 306.

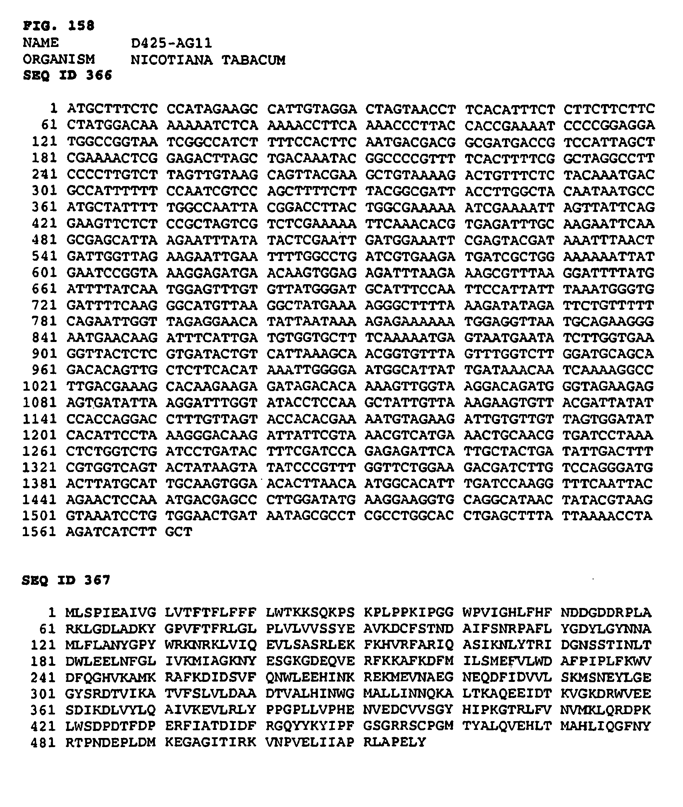

Figure 158 shows nucleic acid SEQ. ID No.: 307 and amino acid SEQ. ID. No.: 308.

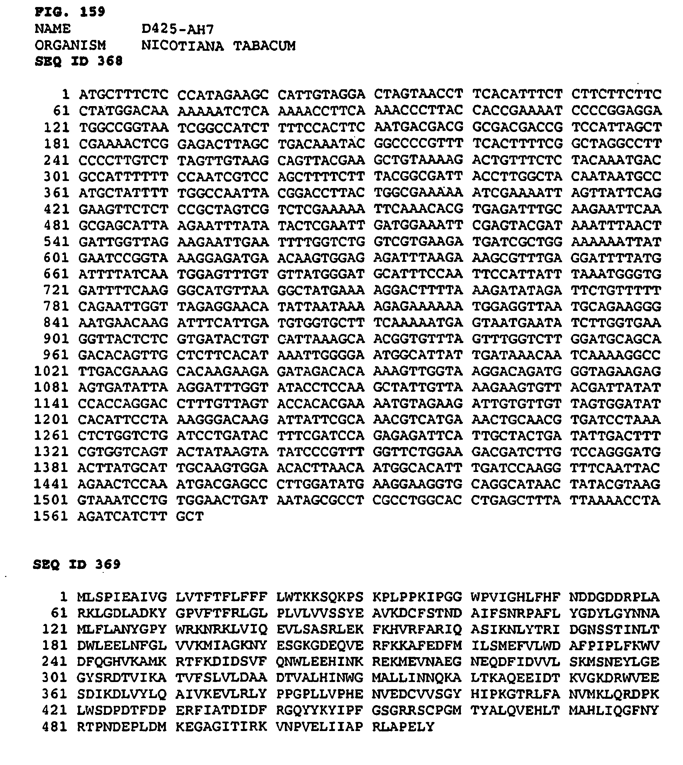

Figure 159 shows nucleic acid SEQ. ID No.: 309 and amino acid SEQ. ID. No.: 310.

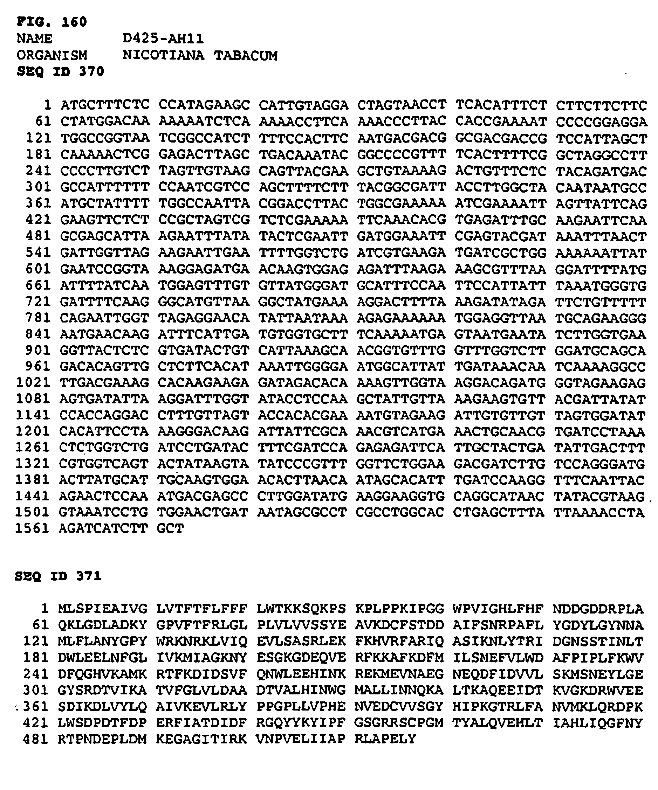

Figure 160 shows nucleic acid SEQ. ID No.: 311 and amino acid SEQ. ID. No.: 312.

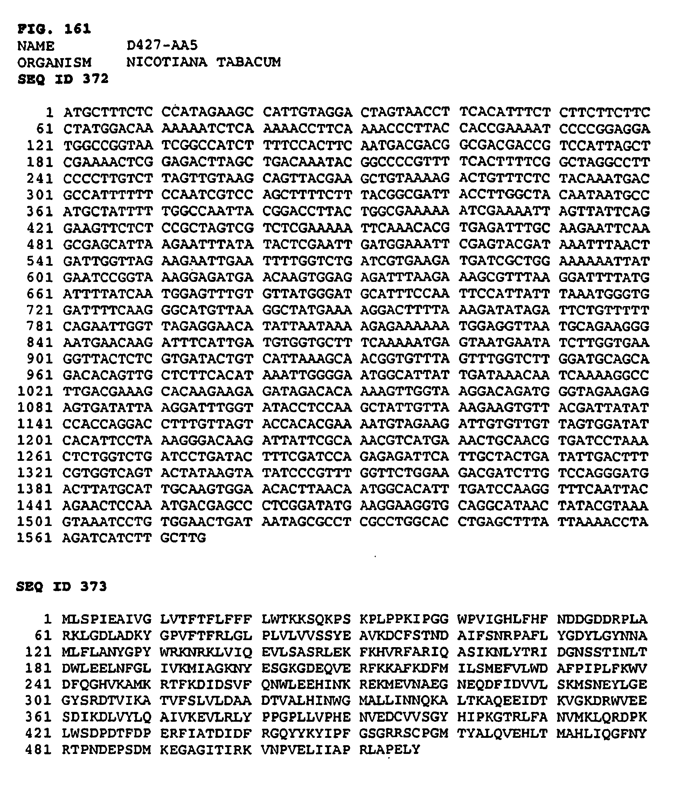

Figure 161 shows nucleic acid SEQ. ID No.: 313 and amino acid SEQ. ID. No.: 314.

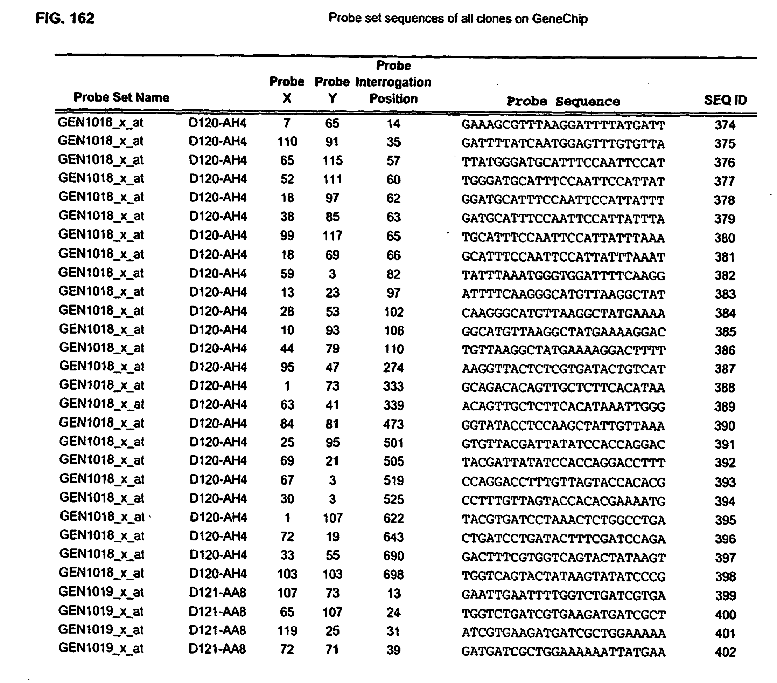

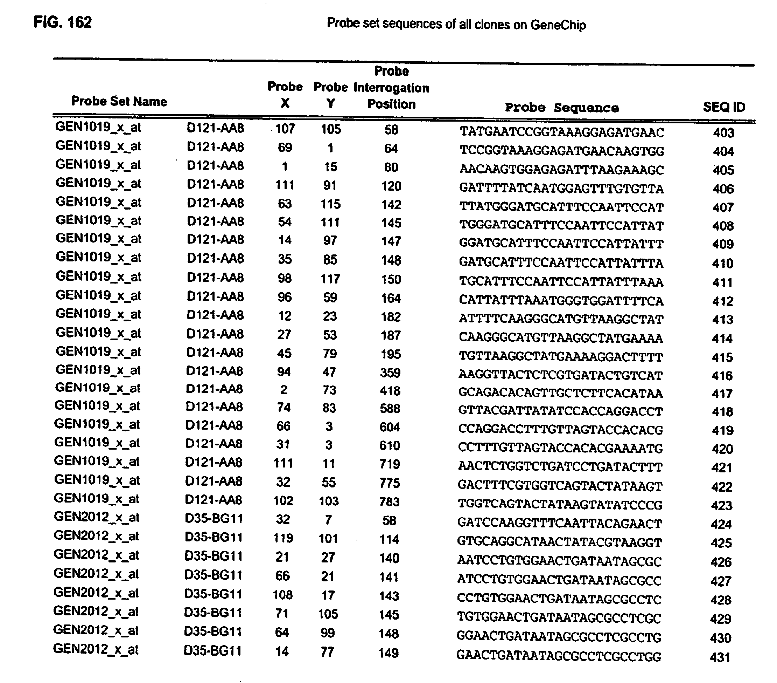

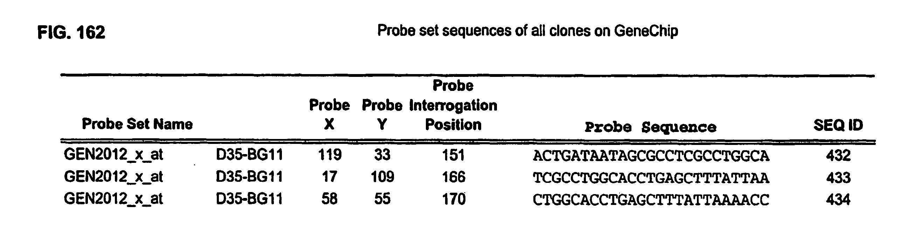

Figure 162 shows nucleic acid SEQ. ID No.: 315 and amino acid SEQ. ID. No.: 316.

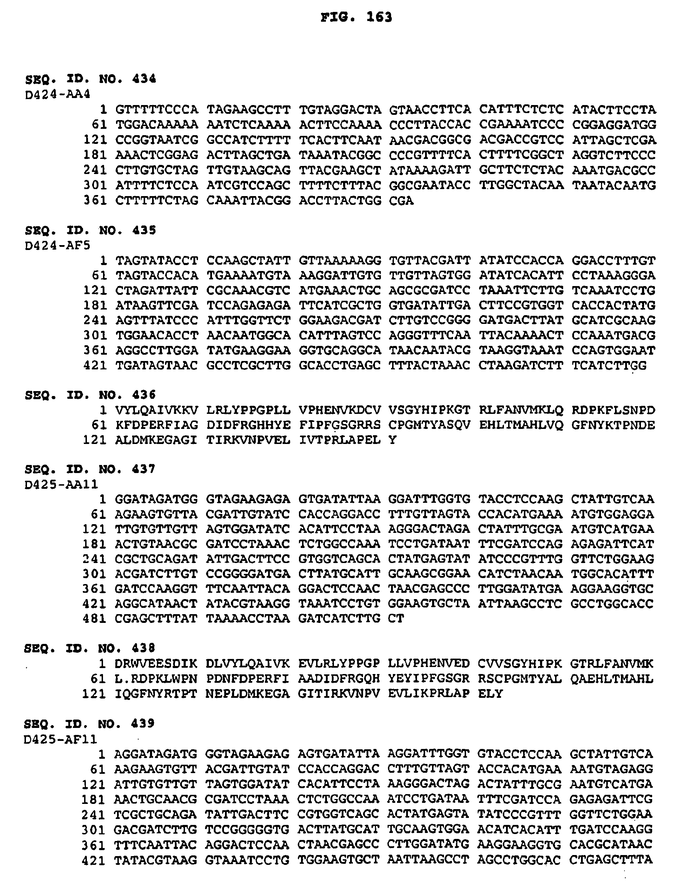

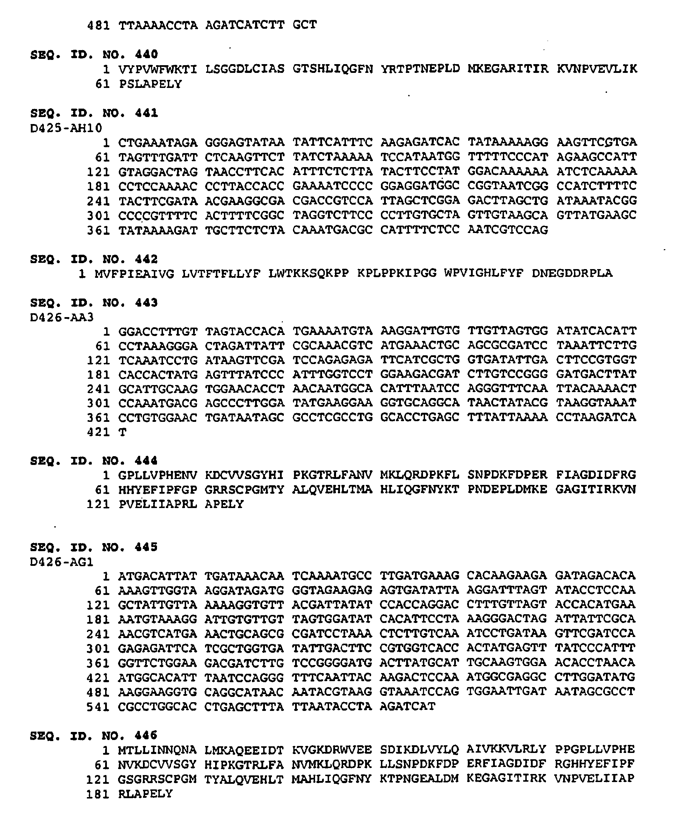

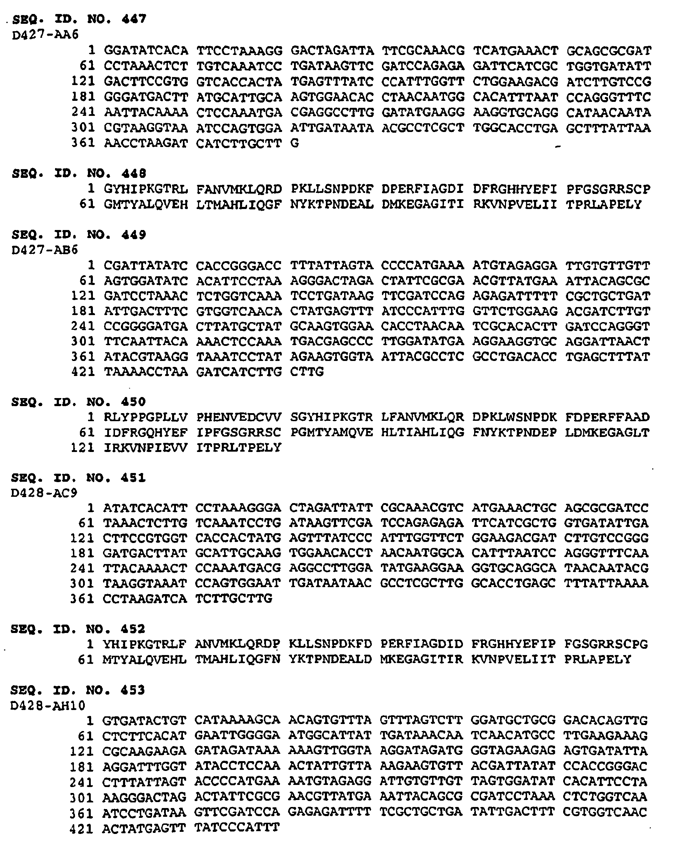

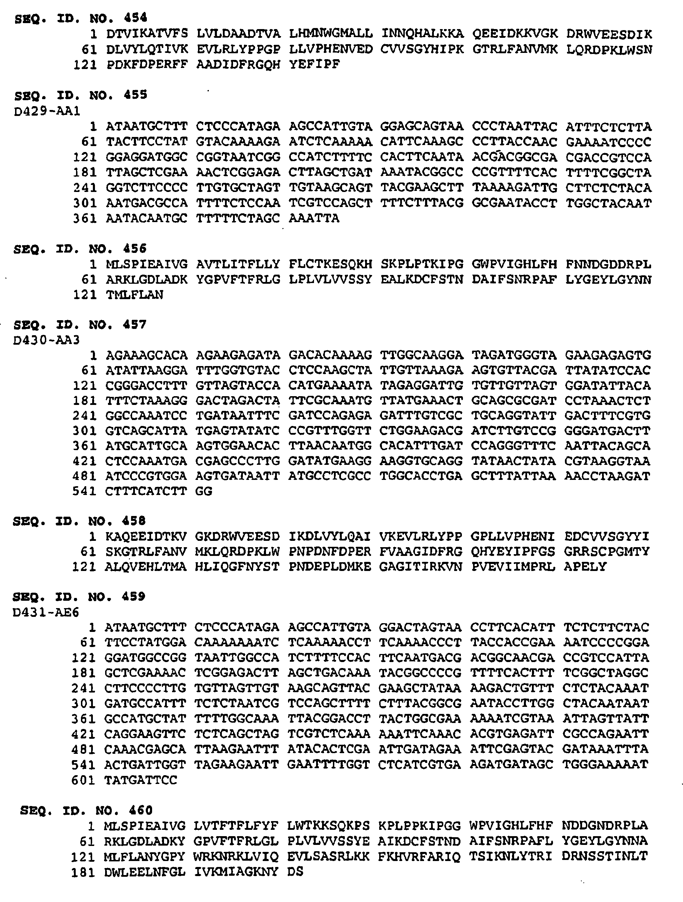

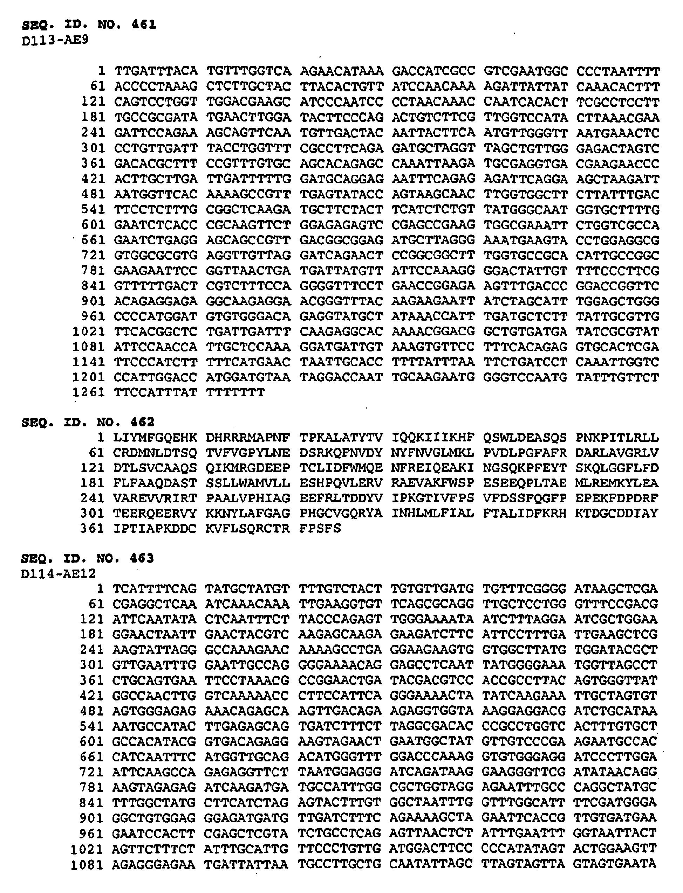

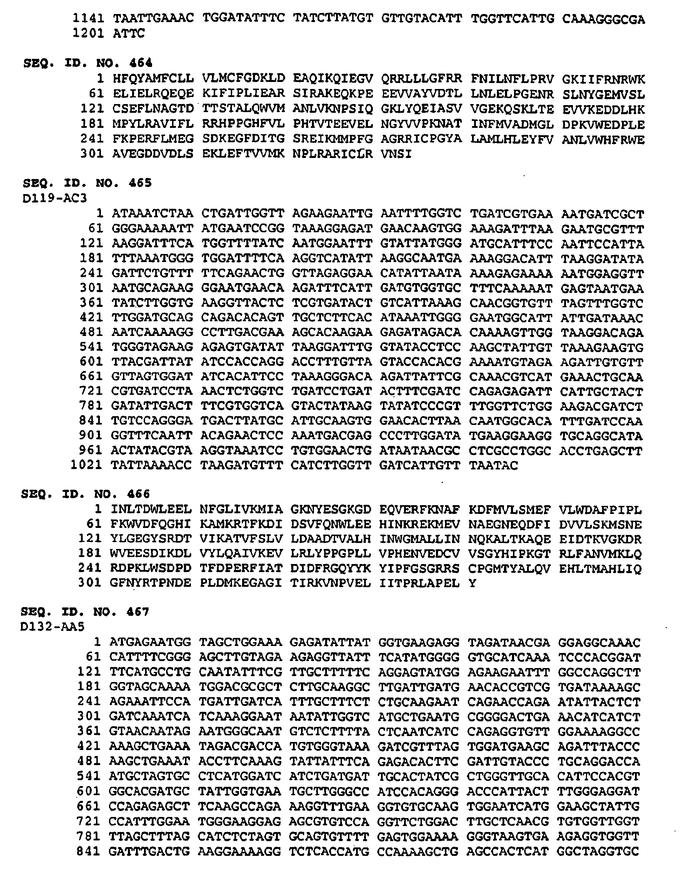

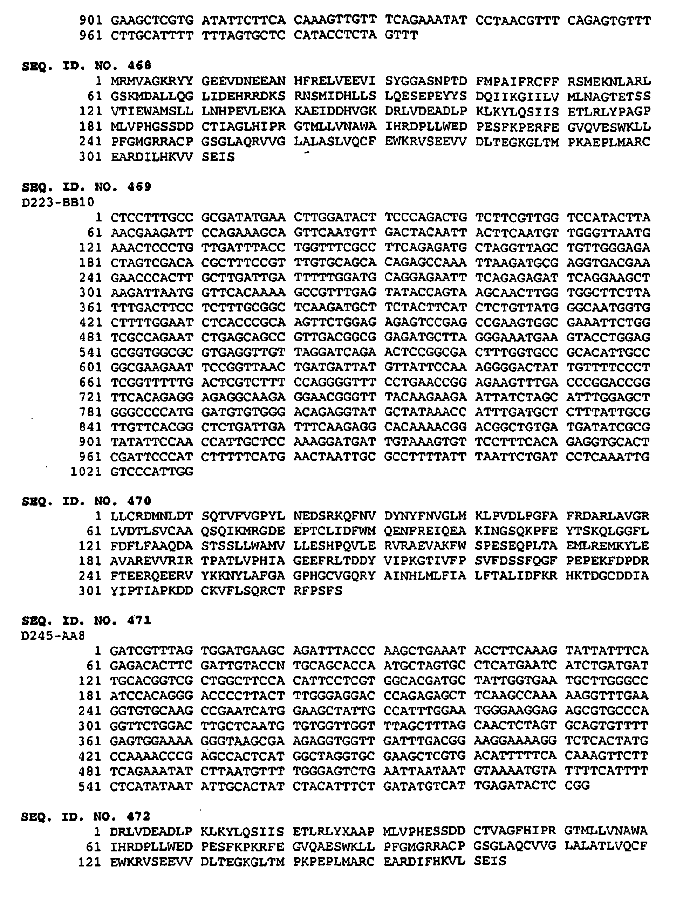

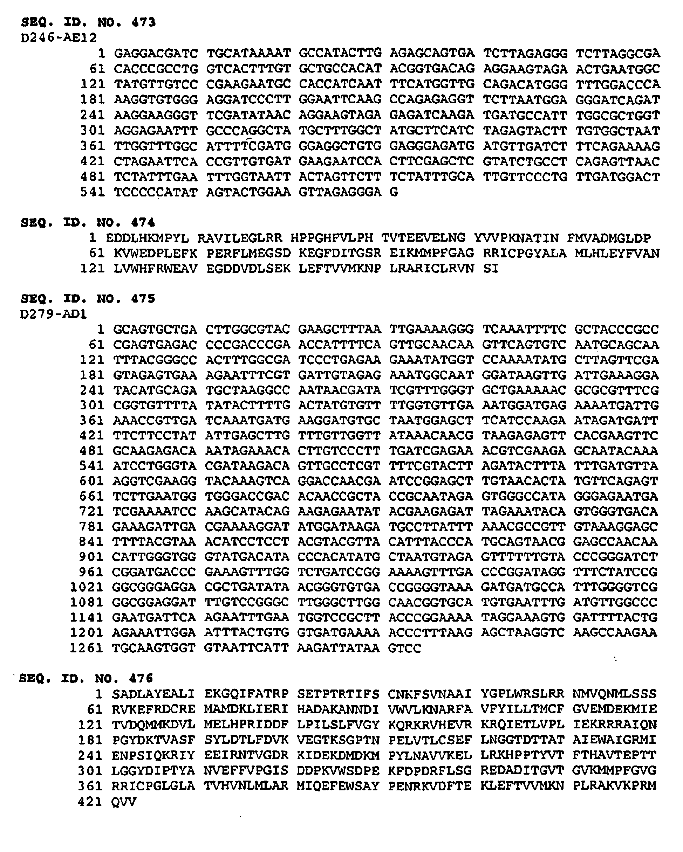

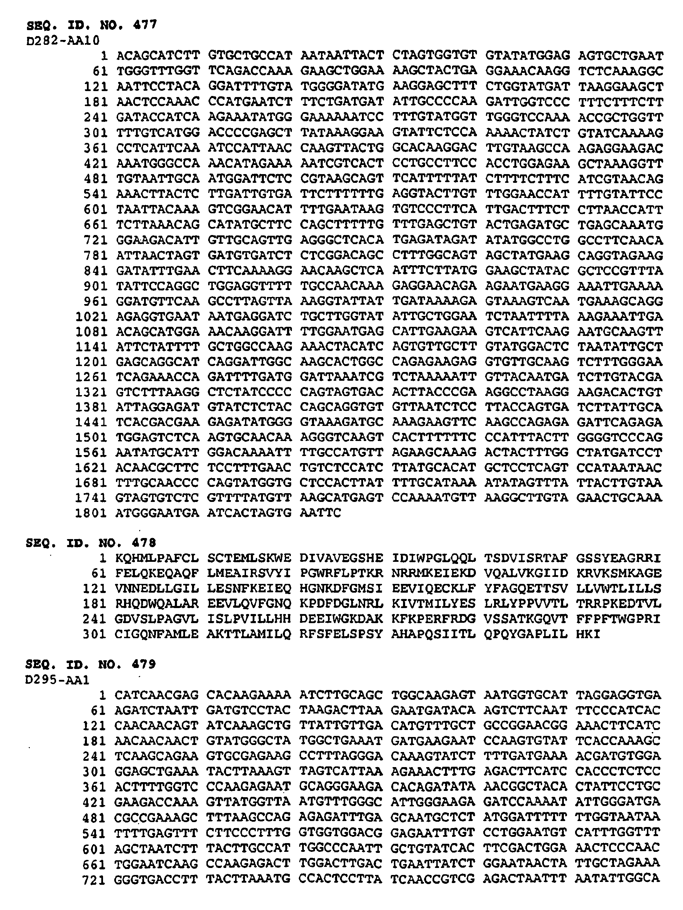

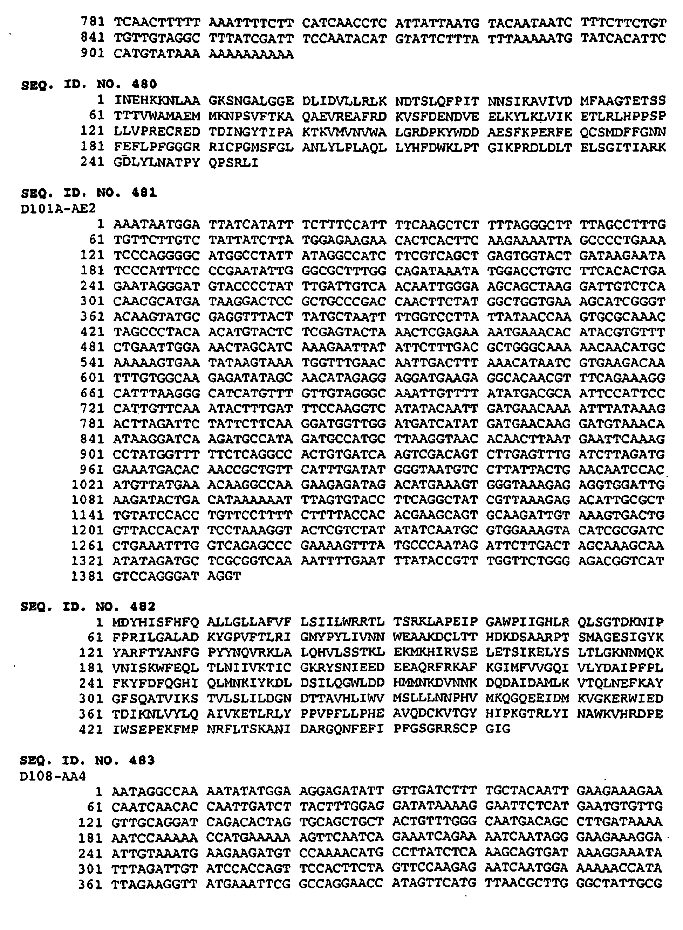

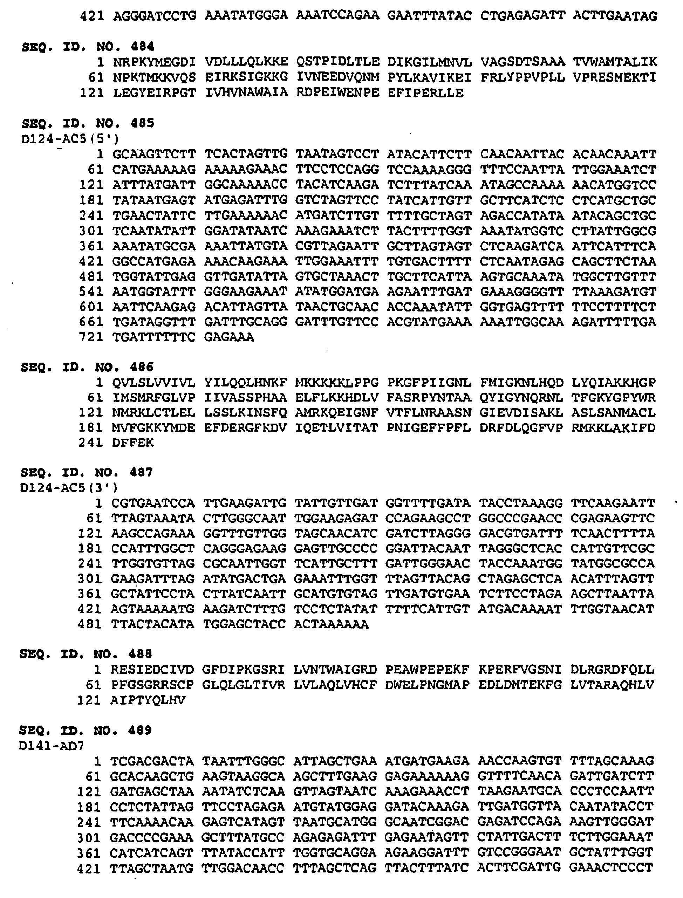

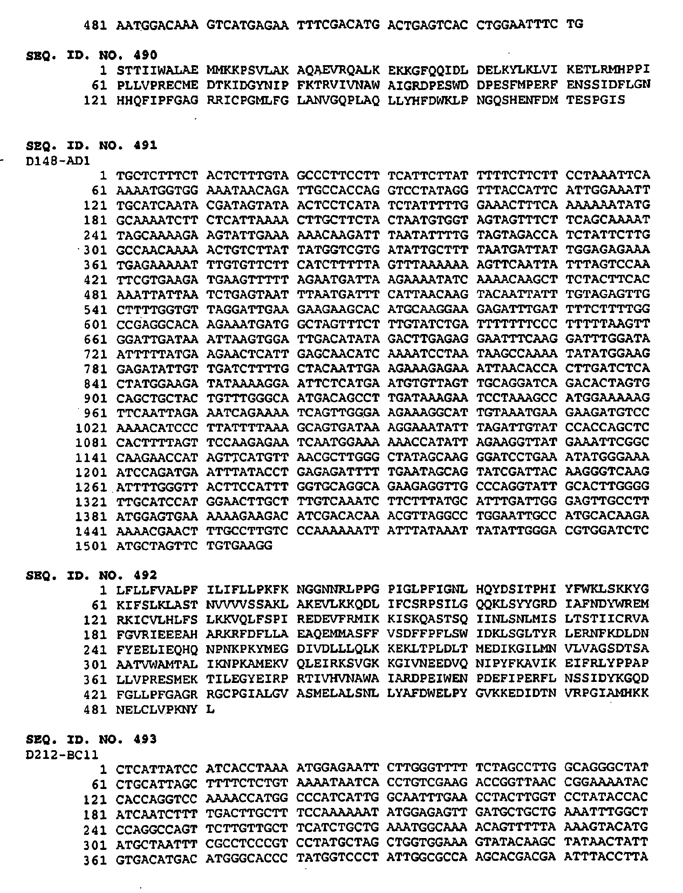

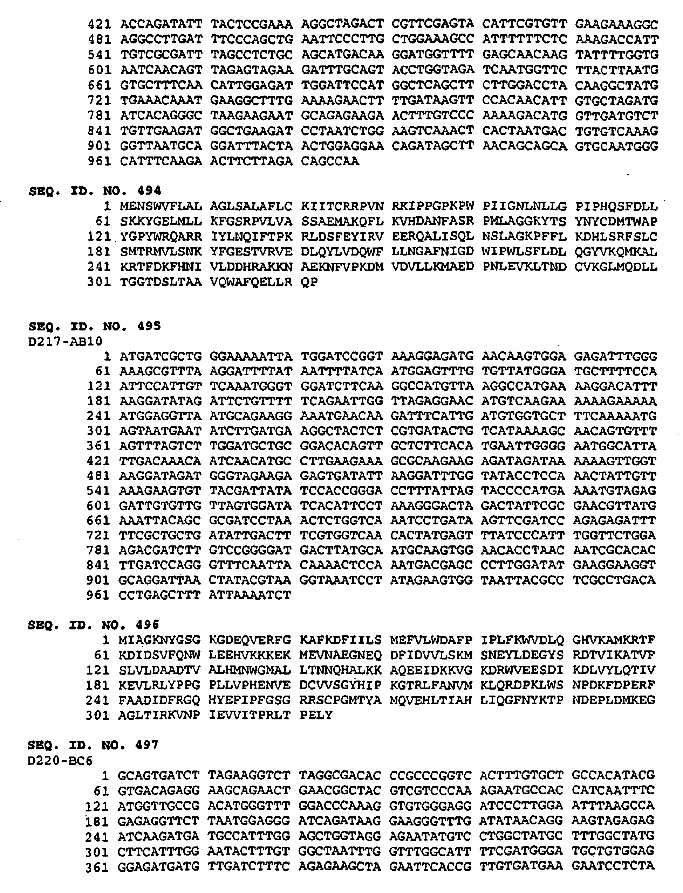

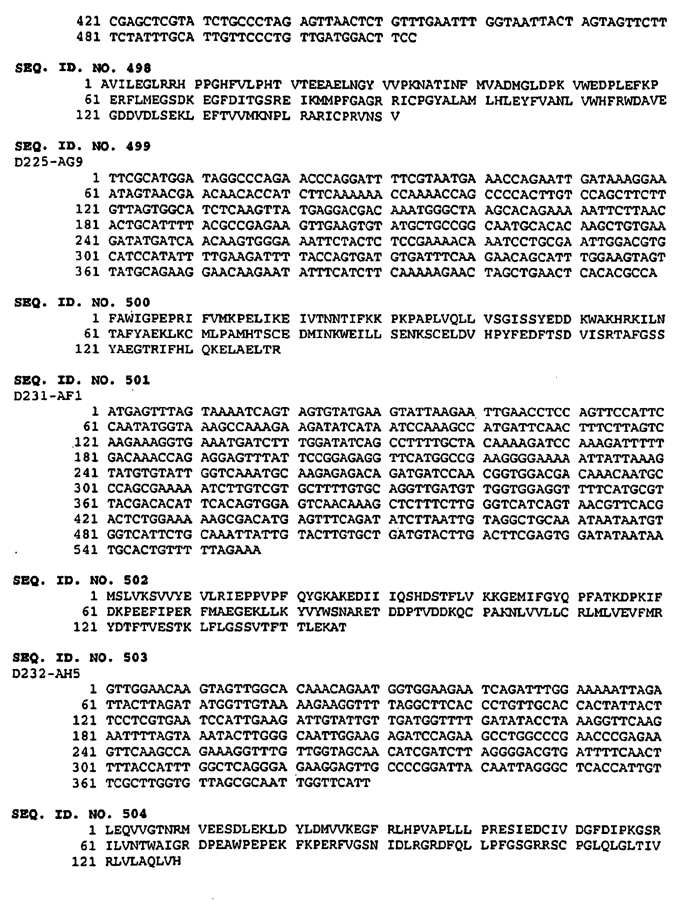

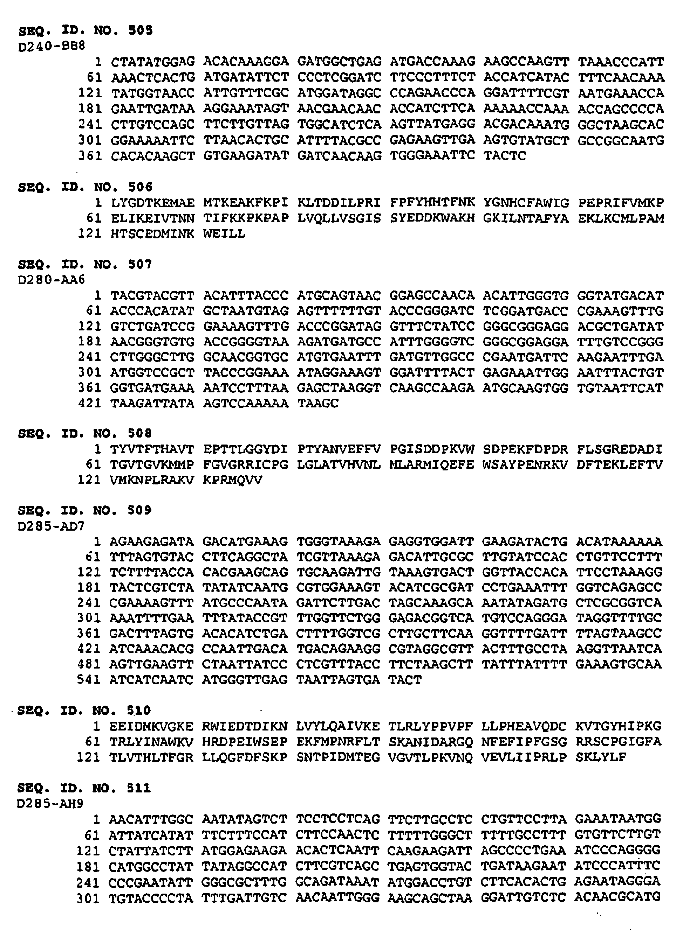

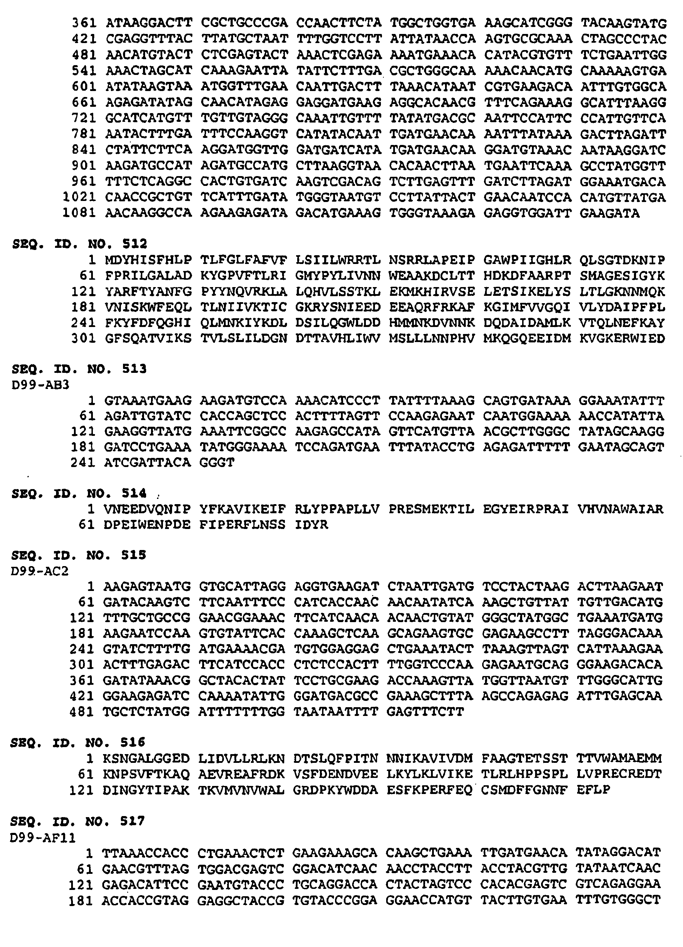

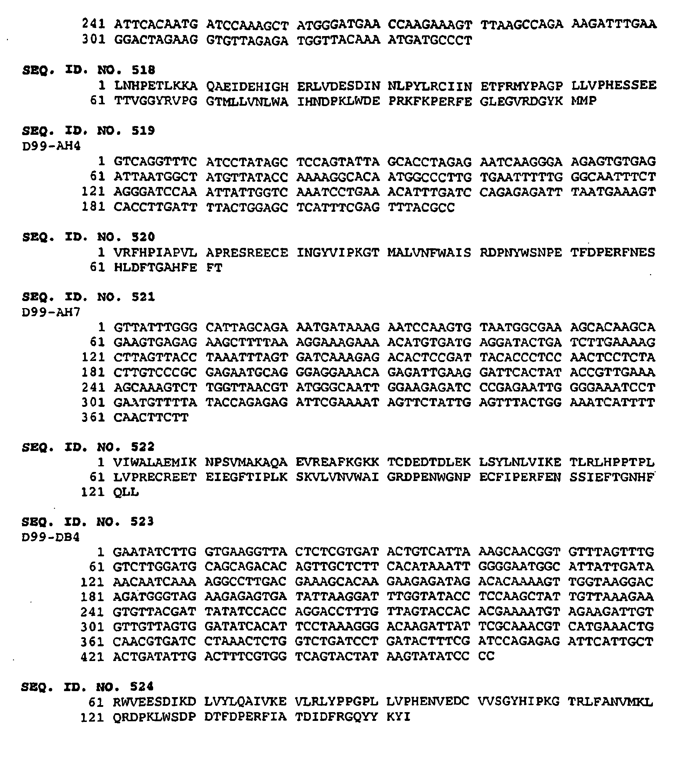

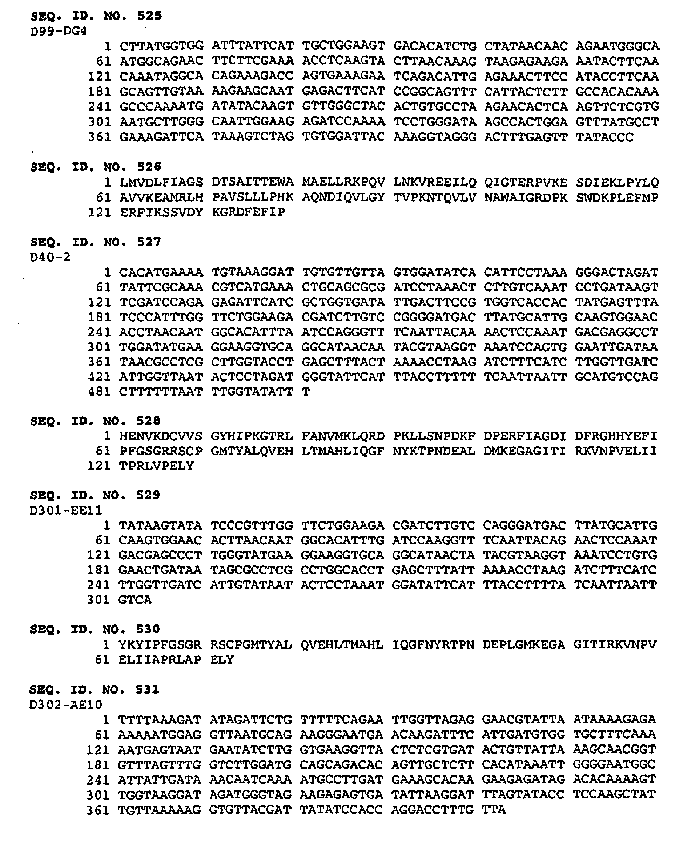

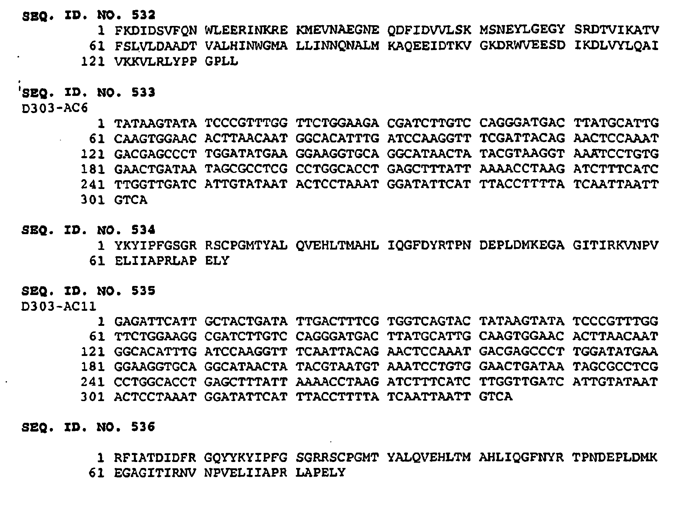

Figure 163 shows probe set sequences of all clones on GeneChip.

### DETAILED DESCRIPTION

### DEFINITIONS

Unless defined otherwise, all technical and scientific terms used herein have the same meaning as commonly understood by one of ordinary skill in the art to which this invention belongs. Singleton et al. (1994) Dictionary of Microbiology and Molecular Biology, second edition, John Wiley and Sons (New York) provides one of skill with a general dictionary of many of the terms used in this invention.

For purposes of the present invention, the following terms are defined below.

"Enzymatic activity" is meant to include demethylation, hydroxylation, epoxidation, N-oxidation, sulfooxidation, N-, S-, and O- dealkylations, desulfation, deamination, and reduction of azo, nitro, and N-oxide groups. The term "nucleic acid" refers to a deoxyribonucleotide or ribonucleotide polymer in either single- or double-stranded form, or sense or anti-sense, and unless otherwise limited, encompasses known analogues of natural nucleotides that hybridize to nucleic acids in a manner similar to naturally occurring nucleotides. Unless otherwise indicated, a particular nucleic acid sequence includes the complementary sequence thereof. The terms "operably linked", "in operable combination", and "in operable order" refer to functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence.

The term "recombinant" when used with reference to a cell indicates that the cell replicates a heterologous nucleic acid, expresses said nucleic acid or expresses a peptide, heterologous peptide, or protein encoded by a heterologous nucleic acid. Recombinant cells can express genes or gene fragments in either the sense or antisense form that are not found within the native (non- recombinant) form of the cell. Recombinant cells can also express genes that are found in the native form of the cell, but wherein the genes are modified and re- introduced into the cell by artificial means.

A "structural gene" is that portion of a gene comprising a DNA segment encoding a protein, polypeptide or a portion thereof, and excluding the 5' sequence which drives the initiation of transcription. The structural gene may alternatively encode a nontranslatable product. The structural gene may be one which is normally found in the cell or one which is not normally found in the cell or cellular location wherein it is introduced, in which case it is termed a "heterologous gene". A heterologous gene may be derived in whole or in part from any source known to the art, including a bacterial genome or episome, eukaryotic, nuclear or plasmid DNA, cDNA, viral DNA or chemically synthesized DNA. A structural gene may contain one or more modifications that could effect biological activity or its characteristics, the biological activity or the chemical structure of the expression product, the rate of expression or the manner of expression control. Such modifications include, but are not limited to, mutations, insertions, deletions and substitutions of one or more nucleotides. The structural gene may constitute an uninterrupted coding sequence or it may include one or more introns, bounded by the appropriate splice junctions. The structural gene may be translatable or nontranslatable, including in an anti-sense orientation. The structural gene may be a composite of segments derived from a plurality of sources and from a plurality of gene sequences (naturally occurring or synthetic, where synthetic refers to DNA that is chemically synthesized).

"Derived from" is used to mean taken, obtained, received, traced, replicated or descended from a source (chemical and/or biological). A derivative may be produced by chemical or biological manipulation (including, but not limited to, substitution, addition, insertion, deletion, extraction, isolation, mutation and replication) of the original source.

"Chemically synthesized", as related to a sequence of DNA, means that portions of the component nucleotides were assembled in vitro. Manual chemical synthesis of DNA may be accomplished using well established procedures (Caruthers, Methodology of DNA and RNA Sequencing, (1983), Weissman (ed.), Praeger Publishers, New York, Chapter 1); automated chemical synthesis can be performed using one of a number of commercially available machines.

Optimal alignment of sequences for comparison may be conducted by the local homology algorithm of Smith and Waterman, Adv. Appl. Math. 2:482 (1981), by the homology alignment algorithm of Needleman and Wunsch, J. Mol. Biol. 48:443 (1970), by the search for similarity method of Pearson and Lipman Proc. Natl. Acad. Sci. (U.S.A.) 85: 2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by inspection.

The NCBI Basic Local Alignment Search Tool (BLAST) (Altschul et al., 1990) is available from several sources, including the National Center for Biological Information (NCBI, Bethesda, Md.) and on the Internet, for use in connection with the sequence analysis programs blastp, blastn, blastx, tblastn and tblastx. It can be accessed at htp://www.ncbi.nlm.nih.gov/BLAST/. A description of how to determine sequence identity using this program is available at http: //www.ncbi.nlm.nih.gov/BLAST/blast help.html.

The terms "substantial amino acid identity" or "substantial amino acid sequence identity" as applied to amino acid sequences and as used herein denote a characteristic of a polypeptide, wherein the peptide comprises a sequence that has at least 70 percent sequence identity, preferably 80 percent amino acid sequence identity, more preferably 90 percent amino acid sequence identity, and most preferably at least 99 to 100 percent sequence identity as compared to a reference group over region corresponding to the first amino acid following the cytochrome p450 motif GXRXCX(G/A) to the stop codon of the translated peptide.

The terms "substantial nucleic acid identity" or "substantial nucleic acid sequence identity" as applied to nucleic acid sequences and as used herein denote a characteristic of a polynucleotide sequence, wherein the polynucleotide comprises a sequence that has at least 75 percent sequence identity, preferably 81 percent amino acid sequence identity, more preferably at least 91 percent sequence identity, and most preferably at least 99 to 100 percent sequence identity as compared to a reference group over region corresponding to the first nucleic acid following the cytochrome p450 motif GXRXCX(G/A) to the stop codon of the translated peptide.

Another indication that nucleotide sequences are substantially identical is if two molecules hybridize to each other under stringent conditions. Stringent conditions are sequence-dependent and will be different in different circumstances. Generally, stringent conditions are selected to be about 5°C to about 20°C, usually about 10°C to about 15°C, lower than the thermal melting point (Tm) for the specific sequence at a defined ionic strength and pH. The Tm is the temperature (under defined ionic strength and pH) at which 50% of the target sequence hybridizes to a matched probe. Typically, stringent conditions will be those in which the salt concentration is about 0.02 molar at pH 7 and the temperature is at least about 60°C. For instance in a standard Southern hybridization procedure, stringent conditions will include an initial wash in 6xSSC at 42 °C followed by one or more additional washes in 0.2xSSC at a temperature of at least about 55°C, typically about 60°C and often about 65°C.

Nucleotide sequences are also substantially identical for purposes of this invention when the polypeptides and/or proteins which they encode are substantially identical. Thus, where one nucleic acid sequence encodes essentially the same polypeptide as a second nucleic acid sequence, the two nucleic acid sequences are substantially identical, even if they would not hybridize under stringent conditions due to degeneracy permitted by the genetic code (see, Darnell et al. (1990) Molecular Cell Biology, Second Edition Scientific American Books W. H. Freeman and Company New York for an explanation of codon degeneracy and the genetic code). Protein purity or homogeneity can be indicated by a number of means well known in the art, such as polyacrylamide gel electrophoresis of a protein sample, followed by visualization upon staining. For certain purposes high resolution may be needed and HPLC or a similar means for purification may be utilized.

As used herein, the term "vector" is used in reference to nucleic acid molecules that transfer DNA segment(s) into a cell. A vector may act to replicate DNA and may reproduce independently in a host cell. The term "vehicle" is sometimes used interchangeably with "vector." The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eucaryotic cells are known to utilize promoters, enhancers, and termination and polyadenylation signals.

For the purpose of regenerating complete genetically engineered plants with roots, a nucleic acid may be inserted into plant cells, for example, by any technique such as in vivo inoculation or by any of the known in vitro tissue culture techniques to produce transformed plant cells that can be regenerated into complete plants. Thus, for example, the insertion into plant cells may be by in vitro inoculation by pathogenic or non-pathogenic *A. tumefaciens*. Other such tissue culture techniques may also be employed.

"Plant tissue" includes differentiated and undifferentiated tissues of plants, including, but not limited to, roots, shoots, leaves, pollen, seeds, tumor tissue and various forms of cells in culture, such as single cells, protoplasts, embryos and callus tissue. The plant tissue may be in planta or in organ, tissue or cell culture.

"Plant cell" as used herein includes plant cells *in planta* and plant cells and protoplasts in culture. "cDNA" or "complementary DNA" generally refers to a single stranded DNA molecule with a nucleotide sequence that is complementary to an RNA molecule. cDNA is formed by the action of the enzyme reverse transcriptase on an RNA template.

### STRATEGIES FOR OBTAINING NUCLEIC ACID SEQUENCES

In accordance with the present disclosure, RNA was extracted from Nicotiana tissue of converter and non-converter Nicotiana lines. The extracted RNA was then used to create cDNA. Nucleic acid sequences of the present disclosure were then generated using two strategies.

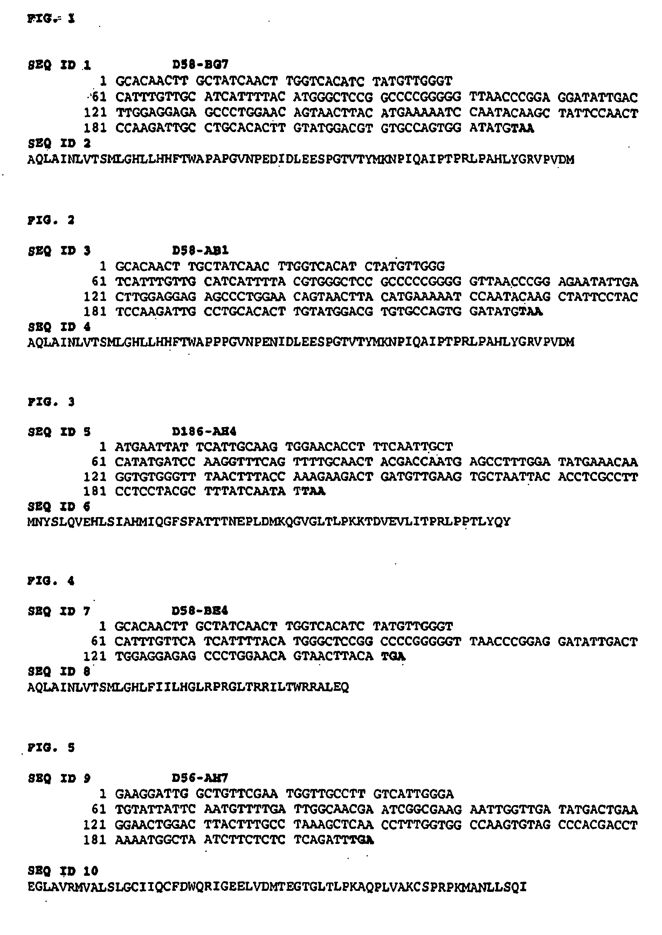

In the first strategy, the poly A enriched RNA was extracted from plant tissue and cDNA was made by reverse transcription PCR. The single strand cDNA was then used to create p450 specific PCR populations using degenerate primers plus a oligo d(T) reverse primer. The primer design was based on the highly conserved motifs of p450. Examples of specific degenerate primers are set forth in Figure 1. Sequence fragments from plasmids containing appropriate size inserts were further analyzed. These size inserts typically ranged from about 300 to about 800 nucleotides depending on which primers were used.

In a second strategy, a cDNA library was initially constructed. The cDNA in the plasmids was used to create p450 specific PCR populations using degenerate primers plus T7 primer on plasmid as reverse primer. As in the first strategy, sequence fragments from plasmids containing appropriate size inserts were further analyzed.

Nicotiana plant lines known to produce high levels of nornicotine (converter) and plant lines having undetectable levels of nornicotine may be used as starting materials.

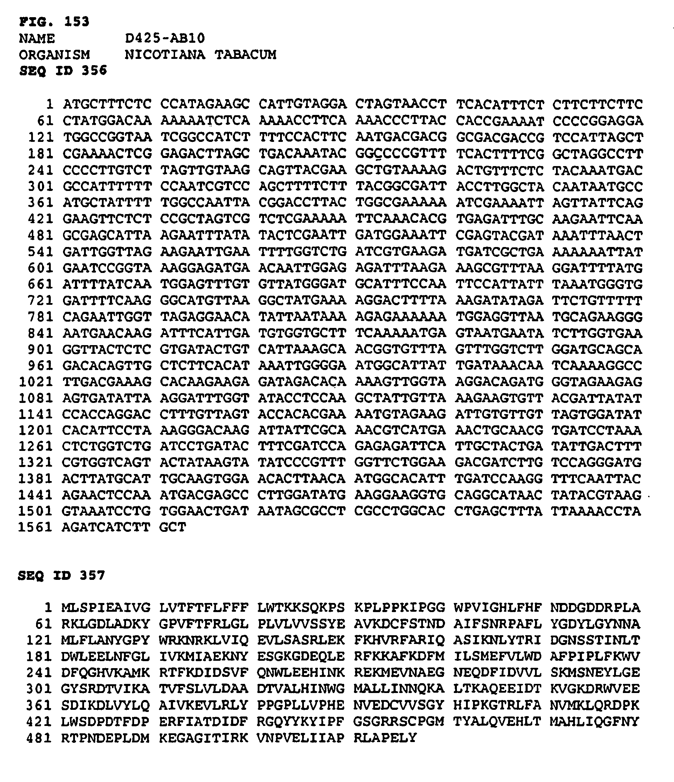

Leaves can then be removed from plants and treated with ethylene to activate p450 enzymatic activities defined herein. Total RNA is extracted using techniques known in the art. cDNA fragments can then be generated using PCR (RT-PCR) with the oligo d(T) primer as described in Figure 153. The cDNA library can then be constructed more fully described in examples herein.

The conserved region of p450 type enzymes can be used as a template for degenerate primers (Figure 75). Using degenerate primers, p450 specific bands can be amplified by PCR. Bands indicative for p450 like enzymes can be identified by DNA sequencing. PCR fragments can be characterized using BLAST search, alignment or other tools to identify appropriate candidates.

Sequence information from identified fragments can be used to develop PCR primers. These primers in combination of plasmid primers in cDNA library were used to clone full length p450 genes. Large-scale Southern reverse analysis was conducted to examine the differential expression for all fragment clones obtained and in some cases full length clones. In this aspect of the invention, these large-scale reverse Southern assays can be conducted using labeled total cDNA's from different tissues as a probe to hybridize with cloned DNA fragments in order to screen all cloned inserts.

Nonradioactive and radioactive (P³²) Northern blotting assays were also used to characterize clones p450 fragments and full length clones.

Peptide specific antibodies were made against several full-length clones by deriving their amino acid sequence and selecting peptide regions that were antigenic and unique relative to other clones. Rabbit antibodies were made to synthetic peptides conjugated to a carrier protein. Western blotting analyses or other immunological methods were performed on plant tissue using these antibodies.

Nucleic acid sequences identified as described above can be examined by using virus induced gene silencing technology (VIGS, Baulcombe, Current Opinions in Plant Biology, 1999, 2:109-113).

Peptide specific antibodies were made for several full-length clones by deriving their amino acid sequence and selecting peptide regions that were potentially antigenic and were unique relative to other clones. Rabbit antibodies were made to synthetic petides conjugated to a carrier protein. Western blotting analyses were perfomed using these antibodies.

In another aspect of the invention, interfering RNA technology (RNAi) is used to further characterize cytochrome p450 enzymatic activities in Nicotiana plants of the present invention. The following references which describe this technology are incorporated by reference herein, Smith et al., Nature, 2000, 407:319-320; Fire et al., Nature, 1998, 391:306-311; Waterhouse et al., PNAS, 1998, 95:13959-13964; Stalberg et al., Plant Molecular Biology, 1993, 23:671- 683; Baulcombe, Current Opinions in Plant Biology, 1999, 2:109-113; and Brigneti et al., EMBO Journal, 1998, 17(22):6739-6746. Plants may be transformed using RNAi techniques, antisense techniques, or a variety of other methods described.

Several techniques exist for introducing foreign genetic material into plant cells, and for obtaining plants that stably maintain and express the introduced gene. Such techniques include acceleration of genetic material coated onto microparticles directly into cells (US Patents 4,945,050 to Cornell and 5,141,131 to DowElanco). Plants may be transformed using Agrobacterium technology, see US Patent 5,177,010 to University of Toledo, 5,104,310 to Texas A&M, European Patent Application 0131624B1, European Patent Applications 120516, 159418B1, European Patent Applications 120516, 15941881 and 176,112 to Schilperoot, US Patents 5,149,645, 5,469,976, 5,464,763 and 4,940,838 and 4,693,976 to Schilperoot, European Patent Applications 116718, 290799, 320500 all to MaxPlanck, European Patent Applications 604662 and 627752 to Japan Nicotiana, European Patent Applications 0267159, and 0292435 and US Patent 5,231,019 all to Ciba Geigy, US Patents 5,463,174 and 4,762,785 both to Calgene, and US Patents 5,004,863 and 5,159,135 both to Agracetus. Other transformation technology includes whiskers technology, see U.S. Patents 5,302,523 and 5,464,765 both to Zeneca. Electroporation technology has also been used to transform plants, see WO 87/06614 to Boyce Thompson Institute, 5,472,869 and 5,384,253 both to Dekalb, WO9209696 and WO9321335 both to PGS. All of these transformation patents and publications are incorporated by reference. In addition to numerous technologies for transforming plants, the type of tissue which is contacted with the foreign genes may vary as well. Such tissue would include but would not be limited to embryogenic tissue, callus tissue type I and II, hypocotyl, meristem, and the like. Almost all plant tissues may be transformed during dedifferentiation using appropriate techniques within the skill of an artisan.

Foreign genetic material introduced into a plant may include a selectable marker. The preference for a particular marker is at the discretion of the artisan, but any of the following selectable markers may be used along with any other gene not listed herein which could function as a selectable marker. Such selectable markers include but are not limited to aminoglycoside phosphotransferase gene of transposon Tn5 (Aph II) which encodes resistance to the antibiotics kanamycin, neomycin and G418, as well as those genes which code for resistance or tolerance to glyphosate; hygromycin; methotrexate; phosphinothricin (bar); imidazolinones, sulfonylureas and triazolopyrimidine herbicides, such as chlorosulfuron; bromoxynil, dalapon and the like.

In addition to a selectable marker, it may be desirous to use a reporter gene. In some instances a reporter gene may be used without a selectable marker. Reporter genes are genes which are typically not present or expressed in the recipient organism or tissue. The reporter gene typically encodes for a protein which provide for some phenotypic change or enzymatic property. Examples of such genes are provided in K. Weising et al. Ann. Rev. Genetics, 22, 421 (1988), which is incorporated herein by reference. Preferred reporter genes include without limitation glucuronidase (GUS) gene and GFP genes.

Once introduced into the plant tissue, the expression of the structural gene may be assayed by any means known to the art, and expression may be measured as mRNA transcribed, protein synthesized, or the amount of gene silencing that occurs (see U.S. Patent No. 5, 583, 021). Techniques are known for the in vitro culture of plant tissue, and in a number of cases, for regeneration into whole plants (EP Appln No. 88810309.0). Procedures for transferring the introduced expression complex to commercially useful cultivars are known to those skilled in the art.

Once plant cells expressing the desired level of p450 enzyme are obtained, plant tissues and whole plants can be regenerated therefrom using methods and techniques well-known in the art. The regenerated plants are then reproduced by conventional means and the introduced genes can be transferred to other strains and cultivars by conventional plant breeding techniques.

The following examples illustrate methods for carrying out the invention and should be understood to be illustrative of, but not limiting upon, the scope of the invention which is defined in the appended claims.

### EXAMPLES

### EXAMPLE I: DEVELOPMENT OF PLANT TISSUE AND ETHYLENE TREATMENT

### Plant Growth

Plants were seeded in pots and grown in a greenhouse for 4 weeks. The 4 week old seedlings were transplanted into individual pots and grown in the greenhouse for 2 months. The plants were watered 2 times a day with water containing 150ppm NPK fertilizer during growth. The expanded green leaves were detached from plants to do the ethylene treatment described below.

### Cell Line 78379

Tobacco line 78379, which is a burley tobacco line released by the University of Kentucky was used as a source of plant material. One hundred plants were cultured as standard in the art of growing tobacco and transplanted and tagged with a distinctive number (1-100). Fertilization and field management were conducted as recommended.

Three quarters of the 100 plants converted between 20 and 100% of the nicotine to nornicotine. One quarter of the 100 plants converted less than 5% of the nicotine to nornicotine. Plant number 87 had the least conversion (2%) while plant number 21 had 100% conversion. Plants converting less than 3% were classified as non-converters. Self-pollinated seed of plant number 87 and plant number 21, as well as crossed (21 x 87 and 87 x 21) seeds were made to study genetic and phenotypic differences. Plants from selfed 21 were converters, and 99% of selfs from 87 were non-converters. The other 1% of the plants from 87 showed low conversion (5-15%). Plants from reciprocal crosses were all converters.

### Cell Line 4407

Nicotiana line 4407, which is a burley line was used as a source of plant material. Uniform and representative plants (100) were selected and tagged. Of the 100 plants 97 were non-converters and three were converters. Plant number 56 had the least amount of conversion (1.2%) and plant number 58 had the highest level of conversion (96%). Self-pollenated seeds and crossed seeds were made with these two plants.

Plants from selfed-58 segregated with 3:1 converter to non-converter ratio. Plants 58-33 and 58-25, were identified as homozygous converter and nonconverter plant lines, respectively. The stable conversion of 58-33 was confirmed by analysis of its progenies of next generation.

### Cell Line PBLB01

PBLB01 is a burley line developed by ProfiGen, Inc. and was used as a source of plant material. The converter plant was selected from foundation seeds of PBLB01.

### Ethylene Treatment Procedures

Green leaves were detached from 2-3 month greenhouse grown plants and sprayed with 0.3% ethylene solution (Prep brand Ethephon (Rhone-Poulenc)). Each sprayed leaf was hung in a curing rack equipped with humidifier and covered with plastic. During the treatment, the sample leaves were periodically sprayed with the ethylene solution. Approximately 24-48 hour post ethylene treatment, leaves were collected for RNA extraction. Another sub-sample was taken for metabolic constituent analysis to determine the concentration of leaf metabolites and more specific constituents of interest such as a variety of alkaloids.

As an example, alkaloids analysis could be performed as follows. Samples (0.1 g) were shaken at 150 rpm with 0.5 ml 2N NaOH, and a 5 ml extraction solution which contained quinoline as an internal standard and methyl t-butyl ether. Samples were analyzed on a HP 6890 GC equipped with a FID detector. A temperature of 250°C was used for the detector and injector. An HP column (30m-0.32nm-1·m) consisting of fused silica crosslinked with 5% phenol and 95% methyl silicon was used at a temperature gradient of 110-185 °C at 10°C per minute. The column was operated at 100°C with a flow rate of 1.7cm³min⁻¹ with a split ratio of 40:1 with a 2·1 injection volume using helium as the carrier gas.

### EXAMPLE 2: RNA ISOLATION

For RNA extractions, middle leaves from 2 month old greenhouse grown plants were treated with ethylene as described. The 0 and 24-48 hours samples were used for RNA extraction. In some cases, leaf samples under the senescence process were taken from the plants 10 days post flower-head removal. These samples were also used for extraction. Total RNA was isolated using Rneasy Plant Mini Kit® (Qiagen, Inc., Valencia, California) following manufacturer's protocol.

The tissue sample was ground under liquid nitrogen to a fine powder using a DEPC treated mortar and pestle. Approximately 100 milligrams of ground tissue were transferred to a sterile 1.5 ml eppendorf tube. This sample tube was placed in liquid nitrogen until all samples were collected. Then, 450µ-l of Buffer RLT as provided in the kit (with the addition of Mercaptoethanol) was added to each individual tube. The sample was vortexed vigorously and incubated at 56° C for 3 minutes. The lysate was then, applied to the QIAshredder™ spin column sitting in a 2-ml collection tube, and centrifuged for 2 minutes at maximum speed. The flow through was collected and 0.5 volume of ethanol was added to the cleared lysate. The sample is mixed well and transferred to an Rneasy® mini spin column sitting in a 2 ml collection tube. The sample was centrifuged for 1 minute at 10,000rpm. Next, 700µl of buffer RW1 was pipetted onto the Rneasy® column and centrifuged for 1 minute at 10,000rpm. Buffer RPE was pipetted onto the Rneasy® column in a new collection tube and centrifuged for 1 minute at 10,000 rpm. Buffer RPE was again, added to the Rneasy® spin column and centrifuged for 2 minutes at maximum speed to dry the membrane. To eliminate any ethanol carry over, the membrane was placed in a separate collection tube and centrifuged for an additional 1 minute at maximum speed. The Rneasy® column was transferred into a new 1.5 ml collection tube, and 40 µl of Rnase-free water was pipetted directly onto the Rneasy® membrane. This final elute tube was centrifuged for 1 minute at 10,000rpm. Quality and quantity of total RNA was analyzed by denatured formaldehyde gel and spectrophotometer.

Poly(A)RNA was isolated using Oligotex™ poly A+ RNA purification kit (Qiagen Inc.) following manufacture's protocol. About 200 µg total RNA in 250 µl maximum volume was used. A volume of 250µl of Buffer OBB and 15 µl of Oligotex™ suspension was added to the 250 µl of total RNA. The contents were mixed thoroughly by pipetting and incubated for 3 minutes at 70°C on a heating block. The sample was then, placed at room temperature for approximately 20 minutes. The oligotex:mRNA complex was pelleted by centrifugation for 2 minutes at maximum speed. All but 50 µl of the supernatant was removed from the microcentrifuge tube. The sample was treated further by OBB buffer. The oligotex:mRNA pellet was resuspended in 400 µl of Buffer OW2 by vortexing. This mix was transferred onto a small spin column placed in a new tube and centrifuged for 1 minute at maximum speed. The spin column was transferred to a new tube and an additional 400 µl of Buffer OW2 was added to the column. The tube was then centrifuged for 1 minute at maximum speed. The spin column was transferred to a final 1.5ml microcentrifuge tube. The sample was eluted with 60 ul of hot (70°C) Buffer OEB. Poly A product was analyzed by denatured formaldehyde gels and spectrophotometric analysis.

### EXAMPLE 3: REVERSE TRANSCRIPTION-PCR

First strand cDNA was produced using SuperScript reverse transcriptase following manufacturer's protocol (Invitrogen, Carlsbad, California). The poly A+ enriched RNA/oligo dT primer mix consisted of less than 5 µg of total RNA, 1 µl of 10mM dNTP mix, 1 µl of Oligo d(T)₁₂₋₁₈ (0.5µg/µl), and up to 10 µl of DEPC-treated water. Each sample was incubated at 65°C for 5 minutes, then placed on ice for at least 1 minute. A reaction mixture was prepared by adding each of the following components in order: 2 µl 10X RT buffer, 4 µl of 25 mM MgCl2, 2µl of 0.1 M DTT, and 1 µl of RNase OUT Recombinant RNase Inhibitor. An addition of 9 µl of reaction mixture was pipetted to each RNA/primer mixture and gently mixed. It was incubated at 42°C for 2 minutes and 1 µl of Super Script II^{™} RT was added to each tube. The tube was incubated for 50 minutes at 42°C. The reaction was terminated at 70° C for 15 minutes and chilled on ice. The sample was collected by centrifugation and 1 µl of RNase H was added to each tube and incubated for 20 minutes at 37°C. The second PCR was carried out with 200 pmoles of forward primer (degenerate primers as in Figure 75, SEQ.ID Nos. 149-156) and 100 pmoles reverse primer (mix of 18nt oligo d(T) followed by 1 random base).

Reaction conditions were 94°C for 2 minutes and then performed 40 cycles of PCR at 94°C for 1 minute, 45° to 60°C for 2 minutes, 72°C for 3 minutes with a 72°C extension for an extra 10 min.

Ten microliters of the amplified sample were analyzed by electrophoresis using a 1% agarose gel. The correct size fragments were purified from agarose gel.

### EXAMPLE 4: GENERATION OF PCR FRAGMENT POPULATIONS

PCR fragments from Example 3 were ligated into a pGEM-T® Easy Vector (Promega, Madison, Wisconsin) following manufacturer's instructions. The ligated product was transformed into JM109 competent cells and plated on LB media plates for blue/white selection. Colonies were selected and grown in a 96 well plate with 1.2 ml of LB media overnight at 37°C. Frozen stock was generated for all selected colonies. Plasmid DNA from plates were purified using Beckman's Biomeck 2000 miniprep robotics with Wizard SV Miniprep® kit (Promega). Plasmid DNA was eluted with 100µl water and stored in a 96 well plate. Plasmids were digested by EcoR1 and were analyzed using 1% agarose gel to confirm the DNA quantity and size of inserts. The plasmids containing a 400-600 bp insert were sequenced using an CEQ 2000 sequencer (Beckman, Fullerton, California). The sequences were aligned with GenBank database by BLAST search. The p450 related fragments were identified and further analyzed. Alternatively, p450 fragments were isolated from substraction libraries. These fragments were also analyzed as described above.

### EXAMPLE 5: CONSTRUCTION OF CDNA LIBRARY

A cDNA library was constructed by preparing total RNA from ethylene treated leaves as follows. First, total RNA was extracted from ethylene treated leaves of tobacco line 58-33 using a modified acid phenol and chloroform extraction protocol. Protocol was modified to use one gram of tissue that was ground and subsequently vortexed in 5 ml of extraction buffer (100 mM Tris-HCl, pH 8.5; 200 mM NaCl; 10mM EDTA; 0.5% SDS) to which 5 ml phenol (pH5.5) and 5 ml chloroform was added. The extracted sample was centrifuged and the supernatant was saved. This extraction step was repeated 2-3 more times until the supernatant appeared clear. Approximately 5 ml of chloroform was added to remove trace amounts of phenol. RNA was precipitated from the combined supernatant fractions by adding a 3-fold volume of ETOH and 1/10 volume of 3M NaOAc (pH5.2) and storing at - 20ºC for 1 hour. After transferring to a Corex glass container the RNA fraction was centrifuged at 9,000 RPM for 45 minutes at 4ºC. The pellet was washed with 70% ethanol and spun for 5 minutes at 9,000 RPM at 4ºC. After drying the pellet, the pelleted RNA was dissolved in 0.5 ml RNase free water. The pelleted RNA was dissolved in 0.5 ml RNase free water. The quality and quantity of total RNA was analyzed by denatured formaldehyde gel and spectrophotometer, respectively.

The resultant total RNA was isolated for poly A+ RNA using an Oligo(dT) cellulose protocol (Invitrogen) and Microcentrifuge spin columns (Invitrogen) by the following protocol. Approximately twenty mg of total RNA was subjected to twice purification to obtain high quality poly A+ RNA. Poly A+ RNA product was analyzed by performing denatured formaldehyde gel and subsequent RT-PCR of known full-length genes to ensure high quality of mRNA.

Next, poly A+ RNA was used as template to produce a cDNA library employing cDNA synthesis kit, ZAP-cDNA® synthesis kit, and ZAP-cDNA® Gigapack® III gold cloning kit (Stratagene, La Jolla, California). The method involved following the manufacture's protocol as specified. Approximately 8 µg of poly A+ RNA was used to construct cDNA library. Analysis of the primary library revealed about 2.5 x 10⁶ - 1x 10⁷ pfu. A quality background test of the library was completed by complementation assays using IPTG and x-gal, where recombinant plaques was expressed at more than 100-fold above the background reaction.

A more quantitative analysis of the library by random PCR showed that average size of insert cDNA was approximately 1.2 kb. The method used a two-step PCR method as followed. For the first step, reverse primers were designed based on the preliminary sequence information obtained from p450 fragments. The designed reverse primers and T3 (forward) primers were used amplify corresponding genes from the cDNA library. PCR reactions were subjected to agarose electrophoresis and the corresponding bands of high molecular weight were excised, purified, cloned and sequenced. In the second step, new primers designed from 5'UTR or the start coding region of p450 as the forward primers together with the reverse primers (designed from 3'UTR of p450) were used in the subsequent PCR to obtain full-length p450 clones.

The p450 fragments were generated by PCR amplification from the constructed cDNA library as described in Example 3 with the exception of the reverse primer. The T7 primer located on the plasmid downstream of cDNA inserts (see Figure 75) was used as a reverse primer. PCR fragments were isolated, cloned and sequenced as described in Example 4.

Full-length p450 genes were isolated by PCR method from constructed cDNA library. Gene specific reverse primers (designed from the downstream sequence of p450 fragments) and a forward primer (T3 on library plasmid) were used to clone the full length genes. PCR fragments were isolated, cloned and sequenced. If necessary, second step PCR was applied. In the second step, new forward primers designed from 5'UTR of cloned p450s together with the reverse primers designed from 3'UTR of p450 clones were used in the subsequent PCR reactions to obtain full-length p450 clones. The clones were subsequently sequenced.

### EXAMPLE 6: CHARACTERIZATION OF CLONED FRAGMENTS - REVERSE SOUTHERN BLOTTING ANALYSIS

Nonradioactive large scale reverse southern blotting assays were performed on all p450 clones identified in above examples to detect the differential expression. It was observed that the level of expression among different p450 clusters was very different. Further real time detection was conducted on those with high expression.

Nonradioactive Southern blotting procedures were conducted as follows.
1) Total RNA was extracted from ethylene treated and nontreated converter (58-33) and nonconverter (58-25) leaves using the Qiagen Rnaeasy kit as described in Example 2.
2) Probe was produced by biotin-tail labeling a single strand cDNA derived from poly A+ enriched RNA generated in above step. This labeled single strand cDNA was generated by RT-PCR of the converter and nonconverter total RNA (Invitrogen) as described in Example 3 with the exception of using biotinalyted oligo dT as a primer (Promega). These were used as a probe to hybridize with cloned DNA.
3) Plasmid DNA was digested with restriction enzyme EcoR1 and run on agarose gels. Gels were simultaneously dried and transferred to two nylon membranes (Biodyne B®). One membrane was hybridized with converter probe and the other with nonconverter probe. Membranes were UV-crosslinked (auto crosslink setting, 254 nm, Stratagene, Stratalinker) before hybridization.
   Alternatively, the inserts were PCR amplified from each plasmid using the sequences located on both arms of p-GEM plasmid, T3 and SP6, as primers. The PCR products were analyzed by running on a 96 well Ready-to-run agarose gels. The confirmed inserts were dotted on two nylon membranes. One membrane was hybridized with converter probe and the other with nonconverter probe.
4) The membranes were hybridized and washed following manufacture's instruction with the modification of washing stringency (Enzo MaxSence™ kit, Enzo Diagnostics, Inc, Farmingdale, NY). The membranes were prehybridized with hybridization buffer (2x SSC buffered formamide, containing detergent and hybridization enhancers) at 42°C for 30 min and hybridized with 10µl denatured probe overnight at 42°C. The membranes then were washed in 1X hybridization wash buffer 1 time at room temperature for 10 min and 4 times at 68°C for 15 min. The membranes were ready for the detection.
5) The washed membranes were detected by alkaline phosphatase labeling followed by NBT/BCIP colometric detection as described in manufacture's detection procedure (Enzo Diagnostics, Inc.). The membranes were blocked for one hour at room temperature with 1x blocking solution, washed 3 times with 1X detection reagents for 10 min, washed 2 times with 1x predevelopment reaction buffer for 5 min and then developed the blots in developing solution for 30-45 min until the dots appear. All reagents were provided by manufacture (Enzo Diagnostics, Inc). In Addition, large scale reverse Southern assay was also performed using KPL southern hybridization and detection kit^{™} following manfacturer's instruction (KPL, Gaithersburg, Maryland).

### EXAMPLE 7: CHARACTERIZATION OF CLONES - NORTHERN BLOT ANALYSIS

Alternative to Southern Blot analysis, some membranes were hybridized and detected as described in the example of Northern blotting assays. Northern Hybridization was used to detect mRNA differentially expressed in Nicotiana as follows.

A random priming method was used to prepare probes from cloned p450 (Megaprime™ DNA Labelling Systems, Amersham Biosciences).

The following components were mixed: 25ng denatured DNA template; 4ul of each unlabeled dTTP, dGTP and dCTP; 5ul of reaction buffer; P³³-labelled dATP and 2ul of Klenow I; and H₂O, to bring the reaction to 50µl. The mixture was incubated in 37°C for 1-4 hours, then stopped with 2µl of 0.5 M EDTA. The probe was denatured by incubating at 95°C for 5 minutes before use.

RNA samples were prepared from ethylene treated and non-treated fresh leaves of several pairs of tobacco lines. In some cases poly A+ enriched RNA was used. Approximately 15µg⁻ total RNA or 1.8µg mRNA (methods of RNA and mRNA extraction as described in Example 5) were brought to equal volume with DEPC H₂O (5-10 µl). The same volume of loading buffer (1 x MOPS; 18.5 % Formaldehyde; 50 % Formamide; 4 % Fico11400; Bromophenolblue) and 0.5 µl EtBr (0.5 µg/µl) were added. The samples were subsequently denatured in preparation for separation of the RNA by electrophoresis.

Samples were subjected to electrophoresis on a formaldehyde gel (1 % Agarose, 1 x MOPS, 0.6 M Formaldehyde) with 1XMOP buffer (0.4 M Morpholinopropanesulfonic acid; 0.1 M Na-acetate-3 x H2O; 10 mM EDTA; adjust to pH 7.2 with NaOH). RNA was transferred to a Hybond-N+ membrane (Nylon, Amersham Pharmacia Biotech) by capillary method in 10 X SSC buffer (1.5 M NaCl; 0.15 M Na-citrate) for 24 hours. Membranes with RNA samples were UV-crosslinked (auto crosslink setting, 254 nm, Stratagene, Stratalinker) before hybridization.

The membrane was prehybridized for 1-4 hours at 42°C with 5-10 ml prehybridization buffer (5 x SSC; 50 % Formamide; 5 x Denhardt's-solution; 1 % SDS; 100µg/ml heat-denatured sheared non- homologous DNA). Old prehybridization buffer was discarded, and new prehybridization buffer and probe were added. The hybridization was carried out over night at 42°C. The membrane was washed for 15 minutes with 2 x SSC at room temperature, followed by a wash with 2 x SSC.

A major focus of the disclosure was the discovery of novel genes that may be induced as a result of ethylene treatment or play a key role in tobacco leaf quality and constituents. As illustrated in the table below, Northern blots and reverse Southern Blot were useful in determining which genes were induced by ethylene treatment relative to non-induced plants. Interestingly, not all fragments were affected similarly in the converter and nonconverter. The cytochrome p450 fragments of interest were partially sequenced to determine their structural relatedness. This information was used to subsequently isolate and characterize full length gene clones of interest.

| Fragments | Induced mRNA Expression Ethylene Treatment |
|---|---|
| | Converter |
| D56-AC7 (SEQ ID No: 35) | + |
| D56-AG11 (SEQ ID No: 31) | + |
| D56-AC12 (SEQ ID No: 45) | + |
| D70A-AB5 (SEQ ID No: 95) | + |
| D73-AC9 (SEQ ID No: 43) | + |
| D70A-AA12 (SEQ ID No: 131) | + |
| D73A-AG3 (SEQ ID No: 129) | + |
| D34-52 (SEQ ID No: 61) | + |
| D56-AG6 (SEQ ID No: 51) | + |

Northern analysis was performed using full length clones on tobacco tissue obtained from converter and nonconverter burley lines that were induced by ethylene treatment. The purpose was to identify those full length clones that showed elevated expression in ethylene induced converter lines relative to ethylene induced converter lines relative to ethylene induced nonconverter burley lines. By so doing, the functionality relationship of full length clones may be determined by comparing biochemical differences in leaf constituents between converter and nonconverter lines. As shown in table below, six clones showed significantly higher expression, as denoted by ++ and +++, in converter ethylene treated tissue than that of nonconverter treated tissue, denoted by +. All of these clones showed little or no expression in converter and nonconverter lines that were not ethylene treated.

| Full Length Clones | Converter | Nonconverter |
|---|---|---|
| D101-BA2 | ++ | + |
| D207-AA5 | ++ | + |
| D208-AC8 | +++ | + |
| D237-AD1 | ++ | + |
| D89-AB1 | ++ | + |
| D90A-BB3 | ++ | + |

### EXAMPLE 8: IMMUNODETECTION OF p450S ENCODED BY THE CLONED GENES

Peptide regions corresponding to 20-22 amino acids in length from three p450 clones were selected for 1) having lower or no homology to other clones and 2) having good hydrophilicity and antigenicity. The amino acid sequences of the peptide regions selected from the respective p450 clones are listed below. The synthesized peptides were conjugated with KHL and then injected into rabbits. Antisera were collected 2 and 4 weeks after the 4^{th} injection (Alpha Diagnostic Intl. Inc. San Antonio, TX).
- D234-AD1: DIDGSKSKLVKAHRKIDEILG
- D90a-BB3: RDAFREKETFDENDVEELNY
- D89-AB1: FKNNGDEDRHFSQKLGDLADKY

Antisera were examined for crossreactivity to target proteins from tobacco plant tissue by Western Blot analysis. Crude protein extracts were obtained from ethylene treated (0 to 40 hours) middle leaves of converter and nonconverter lines. Protein concentrations of the extracts were determined using RC DC Protein Assay Kit (BIO-RAD) following the manufacturer's protocol.

Two micrograms of protein were loaded onto each lane and the proteins separated on 10% - 20% gradient gels using the Laemmli SDS-PAGE system. The proteins were transferred from gels to PROTRAN® Nitrocellulose Transfer Membranes (Schleicher & Schuell) with the Trans-Blot® Semi-Dry cell (BIO-RAD). Target p450 proteins were detected and visualized with the ECL Advance™ Western Blotting Detection Kit (Amersham Biosciences). Primary antibodies against the synthetic-KLH conjugates were made in rabbits. Secondary antibody against rabbit IgG, coupled with peroxidase, was purchased from Sigma. Both primary and secondary antibodies were used at 1:1000 dilutions. Antibodies showed strong reactivity to a single band on the Western Blots indicating that the antisera were monospecific to the target peptide of interest. Antisera were also crossreactive with synthetic peptides conjuated to KLH.

### EXAMPLE 9: NUCLEIC ACID IDENTITY AND STRUCTURE RELATEDNESS OF ISOLATED NUCLEIC ACID FRAGMENTS

Over 100 cloned p450 fragments were sequenced in conjunction with Northern blot analysis to determine their structural relatedness. The approach used utilized forward primers based either of two common p450 motifs located near the carboxyl-terminus of the p450 genes. The forward primers corresponded to cytochrome p450 motifs FXPERF or GRRXCP(A/G) as denoted in Figure 1. The reverse primers used standard primers from either the plasmid, SP6 or T7 located on both arms of pGEM^{™} plasmid, or a poly A tail. The protocol used is described below.

Spectrophotometry was used to estimate the concentration of starting double stranded DNA following the manufacturer's protocol (Beckman Coulter). The template was diluted with water to the appropriate concentration, denatured by heating at 95° C for 2 minutes, and subsequently placed on ice. The sequencing reaction was prepared on ice using 0.5 to 10µl of denatured DNA template, 2 µl of 1.6 pmole of the forward primer, 8 µl of DTCS Quick Start Master Mix and the total volume brought to 20 µl with water. The thermocycling program consisted of 30 cycles of the follow cycle: 96° C for 20 seconds, 50° C for 20 seconds, and 60° C for 4 minutes followed by holding at 4° C.

The sequence was stopped by adding 5 µl of stop buffer (equal volume of 3M NaOAc and 100mM EDTA and 1 µl of 20 mg/ml glycogen). The sample was precipitated with 60 µl of cold 95% ethanol and centrifuged at 6000g for 6 minutes. Ethanol was discarded. The pellet was 2 washes with 200 µl of cold 70% ethanol. After the pellet was dry, 40 µl of SLS solution was added and the pellet was resuspended. A layer of mineral oil was over laid. The sample was then, placed on the CEQ 8000 Automated Sequencer for further analysis.

In order to verify nucleic acid sequences, nucleic acid sequence was re-sequenced in both directions using forward primers to the FXPERF or GRRXCP(A/G) region of the p450 gene or reverse primers to either the plasmid or poly A tail. All sequencing was performed at least twice in both directions.

The nucleic acid sequences of cytochrome p450 fragments were compared to each other from the coding region corresponding to the first nucleic acid after the region encoding the GRRXCP(A/G) motif through to the stop codon. This region was selected as an indicator of genetic diversity among p450 proteins. A large number of genetically distinct p450 genes, in excess of 70 genes, were observed, similar to that of other plant species. Upon comparison of nucleic acid sequences, it was found that the genes could be placed into distinct sequences groups based on their sequence identity. It was found that the best unique grouping of p450 members was determined to be those sequences with 75% nucleic acid identity or greater (shown in Table I). Reducing the percentage identity resulted in significantly larger groups. A preferred grouping was observed for those sequences with 81% nucleic acid identity or greater, a more preferred grouping 91% nucleic acid identity or greater, and a most preferred grouping for those sequences 99% nucleic acid identity of greater. Most of the groups contained at least two members and frequently three or more members. Others were not repeatedly discovered suggesting that approach taken was able to isolated both low and high expressing mRNA in the tissue used.

Based on 75% nucleic acid identity or greater, two cytochrome p450 groups were found to contain nucleic acid sequence identity to previously tobacco cytochrome genes that genetically distinct from that within the group. Group 23, showed nucleic acid identity, within the parameters used for Table I, to prior GenBank sequences of GI:1171579 (CAA64635) and GI:14423327 (or AAK62346) by Czernic et al and Ralston et al, respectively. GI:1171579 had nucleic acid identity to Group 23 members ranging 96.9% to 99.5% identity to members of Group 23 while GI:14423327 ranged 95.4% to 96.9% identity to this group. The members of Group 31 had nucleic acid identity ranging from 76.7% to 97.8% identity to the GenBank reported sequence of GI:14423319 (AAK62342) by Ralston et al. None of the other p450 identity groups of Table 1 contained parameter identity, as used in Table 1, to Nicotiana p450s genes reported by Ralston et al, Czernic et al., Wang et al or LaRosa and Smigocki.

As shown in Figure 76, consensus sequence with appropriate nucleic acid degenerate probes could be derived for group to preferentially identify and isolate additional members of each group from Nicotiana plants.

**Table Nicotiana p450 Nucleic Acid Sequence Identity Groups**

| GROUP | FRAGMENTS |
|---|---|
| 1 | D58-BG7 (SEQ ID No.:1), D58-AB1 (SEQ ID No.:3); D58-BE4 (SEQ ID No.:7) |
| 2 | D56-AH7 (SEQ ID No.:9); D13a-5 (SEQ ID No.:11) |
| 3 | D56-AG10 (SEQ ID No.:13); D35-33 (SEQ ID No.:15); D34-62 (SEQ ID No.:17) |
| 4 | D56-AA7 (SEQ ID No.:19); D56-AE1 (SEQ ID No.:21); 185-BD3 (SEQ ID No.:143) |
| 5 | D35-BB7 (SEQ ID No.:23); D177-BA7 (SEQ ID No.:25); D56A-AB6 (SEQ ID No.:27); D144-AE2 (SEQ ID No.:29) |
| 6 | D56-AG11 (SEQ ID No.:31); D179-AA1 (SEQ ID No.:33) |
| 7 | D56-AC7 (SEQ ID No.:35); D144-AD1 (SEQ ID No.:37) |
| 8 | D144-AB5 (SEQ ID No.:39) |
| 9 | D181-AB5 (SEQ ID No.:41); D73-Ac9 (SEQ ID No.:43) |
| 10 | D56-AC12 (SEQ ID No.:45) |
| 11 | D58-AB9 (SEQ ID No.:47); D56-AG9 (SEQ ID No.:49); D56-AG6 (SEQ ID No.:51); D35-BG11 (SEQ ID No.:53); D35-42 (SEQ ID No.:55); D35-BA3 (SEQ ID No.:57); D34-57 (SEQ ID No.:59); D34-52 (SEQ ID No.:61); D34-25 (SEQ ID No.:63) |
| 12 | D56-AD10 (SEQ ID No.:65) |
| 13 | 56-AA11 (SEQ ID No.:67) |
| 14 | D177-BD5 (SEQ ID No.:69); D177-BD7 (SEQ ID No.:83) |
| 15 | D56A-AG10 (SEQ ID No.:71); D58-BC5 (SEQ ID No.:73); D58-AD12 (SEQ ID No.:75) |
| 16 | D56-AC11 (SEQ ID No.:77); D35-39 (SEQ ID No.:79); D58-BH4 (SEQ ID No.:81); D56-AD6 (SEQ ID No.:87) |
| 17 | D73A-AD6 (SEQ ID No.:89); D70A-BA11 (SEQ ID No.:91) |
| 18 | D70A-AB5 (SEQ ID No.:95); D70A-AA8 (SEQ ID No.:97) |
| 19 | D70A-AB8 (SEQ ID No.:99); D70A-BH2 (SEQ ID No.:101); D70A-AA4 (SEQ ID No.:103) |
| 20 | D70A-BA1 (SEQ ID No.:105); D70A-BA9 (SEQ ID No.:107) |
| 21 | D70A-BD4 (SEQ ID No.:109) |
| 22 | D181-AC5 (SEQ ID No.:111); D144-AH1 (SEQ ID No.:113); D34-65 (SEQ ID No.:115) |
| 23 | D35-BG2 (SEQ ID No.:117) |
| 24 | D73A-AH7 (SEQ ID No.:119) |
| 25 | D58-AA1 (SEQ ID No.:121); D185-BC1 (SEQ ID No.:133); D185-BG2 (SEQ ID No.:135) |
| 26 | D73-AE10 (SEQ ID No.:123) |
| 27 | D56-AC12 (SEQ ID No.:125) |
| 28 | D177-BF7 (SEQ ID No.:127); D185-BE1 (SEQ ID No.:137); D185-BD2 (SEQ ID No.:139) |
| 29 | D73A-AG3 (SEQ ID No.:129) |
| 30 | D70A-AA12 (SEQ ID No.:131); D176-BF2 (SEQ ID No.:85) |
| 31 | D176-BC3 (SEQ ID No.:145) |
| 32 | D176-BB3 (SEQ ID No.: 147) |
| 33 | D186-AH4 (SEQ ID No.:5) |

### EXAMPLE 10: RELATED AMINO ACID SEQUENCE IDENTITY OF ISOLATED NUCLEIC ACID FRAGMENTS

The amino acid sequences of nucleic acid sequences obtained for cytochrome p450 fragments from Example 8 were deduced. The deduced region corresponded to the amino acid immediately after the GXRXCP(A/G) sequence motif to the end of the carboxyl-terminus, or stop codon. Upon comparison of sequence identity of the fragments, a unique grouping was observed for those sequences with 70% amino acid identity or greater. A preferred grouping was observed for those sequences with 80% amino acid identity or greater, more preferred with 90% amino acid identity or greater, and a most preferred grouping for those sequences 99% amino acid identity of greater. The groups and corresponding amino acid sequences of group members are shown in Figure 2. Several of the unique nucleic acid sequences were found to have complete amino acid identity to other fragments and therefore only one member with the identical amino acid was reported.

The amino acid identity for Group 19 of Table II corresponded to three distinct groups based on their nucleic acid sequences. The amino acid sequences of each group member and their identity is shown in Figure. 77. The amino acid differences are appropriated marked.

At least one member of each amino acid identity group was selected for gene cloning and functional studies using plants. In addition, group members that are differentially affected by ethylene treatment or other biological differences as assessed by Northern and Southern analysis were selected for gene cloning and functional studies. To assist in gene cloning, expression studies and whole plant evaluations, peptide specific antibodies will be prepared on sequence identity and differential sequence.

**Table II; Nicotiana p450 Amino Acid Sequence Identity Groups**

| GROUP | FRAGMENTS |
|---|---|
| 1 | D58-BG7 (SEQ ID No.:2), D58-AB1 (SEQ ID No.:4) |
| 2 | D58-BE4 (SEQ ID No.:8) |
| 3 | D56-AH7 (SEQ ID No.:10); D13a-5 (SEQ ID No.:12) |
| 4 | D56-AG10 (SEQ ID No.:14); D34-62 (SEQ ID No.:18) |
| 5 | D56-AA7 (SEQ ID No.:20); D56-AE1 (SEQ ID No.:22); 185-BD3 (SEQ ID No.:144) |
| 6 | D35-BB7 (SEQ ID No.:24); D177-BA7 (SEQ ID No.:26); D56A-AB6 (SEQ ID No.:28); D144-AE2 (SEQ ID No.:30) |
| 7 | D56-AGL1 (SEQ ID No.:32); D179-AA1 (SEQ ID No.:34) |
| 8 | D56-AC7 (SEQ ID No.:36); D144-AD1 (SEQ ID No.:38) |
| 9 | D144-AB5 (SEQ ID No.:40) |
| 10 | D181-AB5 (SEQ ID No.:42); D73-Ac9 (SEQ ID No.:44) |
| 11 | D56-AC12 (SEQ ID No.:46) |
| 12 | D58-AB9 (SEQ ID No.:48); D56-AG9 (SEQ ID No.:50); D56-AG6 (SEQ ID No.:52); D35-BG11 (SEQ ID No.:54); D35-42 (SEQ ID No.:56); D35-BA3 (SEQ ID No.:58); D34-57 (SEQ ID No.:60); D34-52 (SEQ ID No.:62) |
| 13 | D56AD10 (SEQ ID No.:66) |
| 14 | 56-AA11 (SEQ ID No.:68) |
| 15 | D177-BD5 (SEQ ID No.:70); D177-BD7 (SEQ ID No.:84) |
| 16 | D56A-AG10 (SEQ ID No.:72); D58-BC5 (SEQ ID No.:74); D58-AD12 (SEQ ID No.:76) |
| 17 | D56-AC11 (SEQ ID No.:78); D56-AD6 (SEQ ID No.:88) |
| 18 | D73A-AD6 (SEQ ID No.90:) |
| 19 | D70A-AB5 (SEQ ID No.:96); D70A-AB8 (SEQ ID No.:100); D70A-BH2 (SEQ ID No.:102); D70A-AA4 (SEQ ID No.:104); D70A-BA1 (SEQ ID No.:106); D70A-BA9 (SEQ ID No.:108) |
| 20 | D70A-BD4 (SEQ ID No.:110) |
| 21 | D181-AC5 (SEQ ID No.:112); D144-AH1 (SEQ ID No.:114); D34-65 (SEQ ID No.:116) |
| 22 | D35-BG2 (SEQ ID No.:118) |
| 23 | D73A-AH7 (SEQ ID No.:120) |
| 24 | D58-AA1 (SEQ ID No.:122); D185-BC1 (SEQ ID No.:134); D185-BG2 (SEQ ID No.:136) |
| 25 | D73-AE10 (SEQ ID No.:124) |
| 26 | D56-AC12 (SEQ ID No.:126) |
| 27 | D177-BF7 (SEQ ID No.:128); 185-BD2 (SEQ ID No.:140) |
| 28 | D73A-AG3 (SEQ ID No.:130) |
| 29 | D70A-AA12 (SEQ ID No.:132); D176-BF2 (SEQ ID No.:86) |
| 30 | D176-BC3 (SEQ ID No.:146) |
| 31 | D176-BB3 (SEQ ID No.:148) |
| 32 | D186-AH4 (SEQ ID No.:6) |

### EXAMPLE 11: RELATED AMINO ACID SEQUENCE IDENTITY OF FULL LENGTH CLONES

The nucleic acid sequence of full length Nicotiana genes cloned in Example 5 were deduced for their entire amino acid sequence. Cytochrome p450 genes were identified by the presence of three conserved p450 domain motifs, which corresponded to UXXRXXZ, PXRFXF or GXRXC at the carboxyl-terminus where U is E or K, X is any amino acid and Z is P, T, S or M. It was also noted that two of the clones appeared nearly complete but lacked the appropriate stop codon, D130-AA1 and D101-BA2, however but both contained all three p450 cytochrome domains. All p450 genes were characterized for amino acid identity using a BLAST program comparing their full length sequences to each other and to known tobacco genes. The program used the NCBI special BLAST tool (Align two sequences (b12seq), httn://www.ncbi.nlm.nih.gov/blast/b12seq/b12.html). Two sequences were aligned under BLASTN without filter for nucleic acid sequences and BLASTP for amino acid sequences. Based on their percentage amino acid identity, each sequence was grouped into identity groups where the grouping contained members that shared at least 85% identity with another member. A preferred grouping was observed for those sequences with 90% amino acid identity or greater, a more preferred grouping had 95% amino acid identity or greater, and a most preferred grouping had those sequences 99% amino acid identity or greater. Using these criteria, 25 unique groups were identified and are depicted in Table III.

Within the parameters used for Table III for amino acid identity, three groups were found to contain greater than 85% or greater identity to known tobacco genes. Members of Group 5 had up to 96% amino acid identity for full length sequences to prior GenBank sequences of GI:14423327 (or AAK62346) by Ralston et al. Group 23 had up to 93% amino acid identity to GI:14423328 (or AAK62347) by Ralston et al. and Group 24 had 92% identity to GI:14423318 (or AAK62343) by Ralston et al.

**Table III: Amino Acid Sequence Identity Groups of Full Length Nicotiana p450 Genes**

| | |
|---|---|
| 1 | D208-AD9 (SEQ. ID. No. 224); D120-AH4 (SEQ. ID. No. 180); D121-AA8 (SEQ. ID. No. 182), D122-AF10 (SEQ. ID. No. 184); D103-AH3 (SEQ. ID. No. 222); D208-AC8 (SEQ. ID. No. 218); D-235-ABI (SEQ. ID. No. 246) |
| 2 | D244-AD4 (SEQ. ID. No. 250); D244-AB6 (SEQ. ID. No. 274) ; D285-AA8; D285-AB9; D268-AE2 (SEQ. ID. No. 270) |
| 3 | D100A-AC3 (SEQ. ID. No. 168); D100A-BE2 |
| 4 | D205-BE9 (SEQ. ID. No. 276); D205-BG9 (SEQ. ID. No. 202); D205-AH4 (SEQ. ID. No. 294) |
| 5 | D259-AB9 (SEQ. ID. No. 260) ; D257-AE4 (SEQ. ID. No. 268); D147-AD3 (SEQ. ID. No. 194) |
| 6 | D249-AE8 (SEQ. ID. No. 256); D-248-AA6 (SEQ. ID. No. 254) |
| 7 | D233-AG7 (SEQ. ID. No. 266; D224-BD11 (SEQ. ID. No. 240); DAF10 |
| 8 | D105-AD6 (SEQ. ID. No. 172); D215-AB5 (SEQ. ID. No. 220); D135-AE1 (SEQ. ID. No. 190) |
| 9 | D87A-AF3 (SEQ. ID. No. 216), D210-BD4 (SEQ. ID. No. 262) |
| 10 | D89-AB1 (SEQ. ID. No. 150); D89-AD2 (SEQ. ID. No. 152); 163-AG11 (SEQ. ID. No. 198); 163-AF12 (SEQ. ID. No. 196) |
| 11 | D267-AF10 (SEQ. ID. No. 296); D96-AC2 (SEQ. ID. No. 160); D96-AB6 (SEQ. ID. No. 158); D207-AA5 (SEQ. ID. No. 204); D207-AB4 (SEQ. ID. No. 206); D207-AC4 (SEQ. ID. No. 208) |
| 12 | D98-AG1 (SEQ. ID. No. 164); D98-AA1 (SEQ. ID. No. 162) |
| 13 | D209-AA12 (SEQ. ID. No. 212); D209-AA11; D209-AH10 (SEQ. ID. No. 214); D209-AH12 (SEQ. ID. No. 232); D90a-BB3 (SEQ. ID. No. 154) |
| 14 | D129-AD10 (SEQ. ID. No. 188); D104A-AE8 (SEQ. ID. No. 170) |
| 15 | D228-AH8 (SEQ. ID. No. 244); D228-AD7 (SEQ. ID. No. 241), D250-AC11 (SEQ. ID. No. 258); D247-AH1 (SEQ. ID. No. 252) |
| 16 | D128-AB7 (SEQ. ID. No. 186) ; D243-AA2 (SEQ. ID. No. 248); D125-AF11 (SEQ. ID. No. 228) |
| 17 | D284-AH5 (SEQ. ID. No. 298); D110-AF12 (SEQ. ID. No. 176) |
| 18 | D221-BB8 (SEQ. ID. No. 234) |
| 19 | D222-BH4 (SEQ. ID. No. 236) |
| 20 | D134-AE11 (SEQ. ID. No. 230) |
| 21 | D109-AH8 (SEQ. ID. No. 174) |
| 22 | D136-AF4 (SEQ. ID. No. 278) |
| 23 | D237-AD1 (SEQ. ID. No. 226) |
| 24 | D112-AA5 (SEQ. ID. No. 178) |
| 25 | D283-AC1 (SEQ. ID. No. 272) |

The full length genes were further grouped based on the highly conversed amino acid homology between UXXRXXZ p450 domain and GXRXC p450 domain near the end the carboxyl-terminus. As shown in Figure 3, individual clones were aligned for their sequence homology between the conserved domains relative to each other and placed in distinct identity groups. In several cases, although the nucleic acid sequence of the clone was unique, the amino acid sequence for the region was identical. The preferred grouping was observed for those sequences with 90% amino acid identity or greater, a more preferred group had 95% amino acid identity or greater, and a most preferred grouping had those sequences 99% amino acid identity of greater. The final grouping was similar to that based on the percent identity for the entire amino acid sequence of the clones except for Group 17 (of Table III) which was divided into two distinct groups.

Within the parameters used for amino acid identity in Table IV, three groups were found to contain 90% or greater identity to known tobacco genes. Members of Group 5 had up to 93.4% amino acid identity for full length sequences to prior GenBank sequences of GI:14423326 (AAK62346) by Ralston et al. Group 23 had up to 91.8% amino acid identity to GI:14423328 (or AAK62347) by Ralston et al. and Group 24 had 98.8% identity to GI:14423318 (or AAK62342) by Ralston et al.

**Table IV: Amino Acid Sequence Identity groups of Regions between Conserved Domains of Nicotiana p450 Genes**

| | |
|---|---|
| 1 1 | D208-AD9 (SEQ. ID. No. 224); D120-AH4 (SEQ. ID. No. 180); D121-AA8 (SEQ. ID. No. 182), D122-AF10 (SEQ. ID. No. 184); D103-AH3 (SEQ. ID. No. 222); D208-AC8 (SEQ. ID. No. 218); D-235-ABI (SEQ. ID. No. 246) |
| 2 | D244-AD4 (SEQ. ID. No. 250); D244-AB6 (SEQ. ID. No. 274) ; D285-AA8; D285-AB9; D268-AE2 (SEQ. ID. No. 270) |
| 3 | D100A-AC3 (SEQ. ID. No. 168); D100A-BE2 |
| 4 | D205-BE9 (SEQ. ID. No. 276); D205-BG9 (SEQ. ID. No. 202); D205-AH4 (SEQ. ID. No. 294) |
| 5 | D259-AB9 (SEQ. ID. No. 260) ; D257-AE4 (SEQ. ID. No. 268); D147-AD3 (SEQ. ID. No. 194) |
| 6 | D249-AEQ (SEQ. ID. No. 256); D-248-AA6 (SEQ. ID. No. 254) |
| 7 | D233-AG7 (SEQ. ID. No. 266; D224-BD11 (SEQ. ID. No. 240); DAF10 |
| 8 | D105-AD6 (SEQ. ID. No. 172); D215-AB5 (SEQ. ID. No. 220); D135-AE1 (SEQ. ID. No. 190) |
| 9 | D87A-AF3 (SEQ. ID. No. 216), D210-BD4 (SEQ. ID. No. 262) |
| 10 | D89-AB1 (SEQ. ID. No. 150); D89-AD2 (SEQ. ID. No. 152); 163-AG11 (SEQ. ID. No. 198); 163-AF12 (SEQ. ID. No. 196) |
| 11 | D267-AF10 (SEQ. ID. No. 296); D96-AC2 (SEQ. ID. No. 160); D96-AB6 (SEQ. ID. No. 158); D207-AA5 (SEQ. ID. No. 204); D207-AB4 (SEQ. ID. No. 206); D207-AC4 (SEQ. ID. No. 208) |
| 12 | D98-AG1 (SEQ. ID. No. 164); D98-AA1 (SEQ. ID. No. 162) |
| 13 | D209-AA12 (SEQ. ID. No. 212); D209-AA11; D209-AH10 (SEQ. ID. No. 214); D209-AH12 (SEQ. ID. No. 232); D90a-BB3 (SEQ. ID. No. 154) |
| 14 | D129-AD10 (SEQ. ID. No. 188); D104A-AE8 (SEQ. ID. No. 170) |
| 15 | D228-AH8 (SEQ. ID. No. 244); D228-AD7 (SEQ. ID. No. 241), D250-AC11 (SEQ. ID. No. 258); D247-AH1 (SEQ. ID. No. 252) |
| 16 | D128-AB7 (SEQ. ID. No. 186) ; D243-AA2 (SEQ. ID. No. 248); D125-AF11 (SEQ. ID. No. 228) |
| 17 | D284-AH5 (SEQ. ID. No. 298); D110-AF12 (SEQ. ID. No. 176) |
| 18 | D221-BB8 (SEQ. ID. No. 234) |
| 19 | D222-BH4 (SEQ. ID. No. 236) |
| 20 | D134-AE11 (SEQ. ID. No. 230) |
| 21 | D109-AH8 (SEQ. ID. No. 174) |
| 22 | D136-AF4 (SEQ. ID. No. 278) |
| 23 | D237-AD1 (SEQ. ID. No. 226) |
| 24 | D112-AA5 (SEQ. ID. No. 178) |
| 25 | D283-AC1 (SEQ. ID. No. 272) |
| 26 | D110-AF12 (SEQ. ID. No. 176) |

### EXAMPLE 12: NICOTIANA CYTOCHROME P450 CLONES LACKING ONE OR MORE OF THE TOBACCO CYTOCHROME P450 SPECIFIC DOMAINS

Four clones had high nucleic acid homology, ranging 90% to 99% nucleic acid homology, to other tobacco cytochrome genes reported in Table III. The four clones included D136-AD5, D138-AD12, D243-AB3 and D250-AC11. However, due to a nucleotide frameshift these genes did not contain one or more of three C-terminus cytochrome p450 domains and were excluded from identity groups presented in Table III or Table IV.

The amino acid identity of one clone, D95-AG1, did not contain the third domain, GXRXC, used to group p450 tobacco genes in Table III or Table IV. The nucleic acid homology of this clone had low homology to other tobacco cytochrome genes. This clone represents a novel and different group of cytochrome p450 genes in Nicotiana.

### EXAMPLE 13: USE OF NICOTIANA CYTOCHROME P450 FRAGMENTS AND CLONES IN ALTERED REGULATION OF TOBACCO PROPERTIES

The use of tobacco p450 nucleic acid fragments or whole genes are useful in identifying and selecting those plants that have altered tobacco phenotypes or tobacco constituents and, more importantly, altered metabolites. Transgenic tobacco plants are generated by a variety of transformation systems that incorporate nucleic acid fragments or full length genes, selected from those reported herein, in orientations for either down-regulation, for example anti-sense orientation, or over-expression for example, sense orienation. For over-expression to full length genes, any nucleic acid sequence that encodes the entire or a functional part or amino acide sequence of the full-length genes described in this invention are desired that are effective for increasing the expression of a certain enzyme and thus resulting in phenotypic effect within Nicotiana. Nicotiana lines that are homozygous lines are obtained through a series of backcrossing and assessed for phenotypic changes including, but not limited to, analysis of endogenous p450 RNA, transcripts, p450 expressed peptides and concentrations of plant metabolites using techniques commonly avaiable to one having ordinary skill in the art. The changes exhibited in the tobacco plans provide information on the functional role of the selected gene of interest or are of a utility as a preffered Nicotiana plant species.

### EXAMPLE 14. IDENTIFICATION OF GENES INDUCED IN ETHYLENE TREATED CONVERTER LINES

High density oligonucleotide array technology, Affymetrix GeneChip® (Affymetrix Inc., Santa Clara, CA) array, was used for quantitative and highly parallel measurements of gene expression. In using this technology, nucleic acid arrays were fabricated by direct synthesis of oligonucleotides on a solid surface. This solid-phase chemistry is able to produce arrays containing hundreds of thousands of oligonucleotide probes packed at extremely high densities on a chip referred to as GeneChip®. Thousands of genes can be simultaneously screened from a single hybridization. Each gene is typically represented by a set of 11-25 pairs of probes depending upon size. The probes are designed to maximize sensitivity, specificity, and reproducibility, allowing consistent discrimination between specific and background signals, and between closely related target sequences.

Affymetrix GeneChip hybridization experiments involve the following steps: design and production of arrays, preparation of fluorescently labeled target from RNA isolated from the biological specimens, hybridization of the labeled target to the GeneChip, screening the array, and analysis of the scanned image and generation of gene expression profiles.

### A. Designing and Custom making Affymetrix GeneChip

A GeneChip CustomExpress Advantage Array was custom made by Affymetrix Inc. (Santa Clara, CA). Chip size was 18 micron and array format was 100-2187 that can accommodate 528 probe sets (11, 628 probes). Except for GenBank derived nucleic acid sequences, all sequences were selected from our previously identified tobacco clones and all probes were custom designed. A total of 400 tobacco genes or fragments were selected to be included on the GeneChip. The sequences of oligonucleotides selected were based on unique regions of the 3' end of the gene. The selected nucleic acid sequences consisted of 56 full length p450 genes and 71 p450 fragments that were cloned from tobacco, described in (patent applications). Other tobacco sequences included270 tobacco ESTs which were generated from suppression subtraction library using Clontech SSH kit(BD Biosciences, Palo Alto, CA). Among these genes, some oligonucleotide sequences were selected from cytochrome P450 genes listed in GenBank. Up to 25 probes were used for each full length gene and 11 probes for each fragment. A reduced number of probes were used for some clones due to the lack of unique, high quality probes. Appropriate control sequences were also included on the GeneChip®.

The probe Arrays were 25-mer oligonucleotides that were directly synthesized onto a glass wafer by a combination of semiconductor-based photolithography and solid phase chemical synthesis technologies. Each array contained up to 100,000 different oligonucleotide probes. Since oligonucleotide probes are synthesized in known locations on the array, the hybridization patterns and signal intensities can be interpreted in terms of gene identity and relative expression levels by the Affymetrix Microarray Suite® software. Each probe pair consists of a perfect match oligonucleotide and a mismatch oligonucleotide. The perfect match probe has a sequence exactly complimentary to the particular gene and thus measures the expression of the gene. The mismatch probe differs from the perfect match probe by a single base substitution at the center base position, which disturbs the binding of the target gene transcript. The mismatch produces a nonspecific hybridization signal or background signal that was compared to the signal measured for the perfect match oligonucleotide.

### B. Sample preparation

Hybridization experiments were conducted by Genome Explorations, Inc. (Memphis, TN). The RNA samples used in hybridization consisted of six pairs of nonconverter/converter isogenic lines that were induced by ethylene treatments. Samples included one pair of 4407-25/4407-33 non-treated burly tobacco samples, three pairs of ethylene treated 4407-25/4407-33 samples, one pair of ethylene treated dark tobacco NL Madole/181 and one pair of ethylene treated burly variety PBLB01/178. Ethylene treatment was as described in Example 1.

Total RNA was extracted from above mentioned ethylene treated and non-treated leaves using a modified acid phenol and chloroform extraction protocol. Protocol was modified to use one gram of tissue that was ground and subsequently vortexed in 5 ml of extraction buffer (100 mM Tris-HCl, pH 8.5; 200 mM NaCl; 10mM EDTA; 0.5% SDS) to which 5 ml phenol (pH5.5) and 5 ml chloroform was added. The extracted sample was centrifuged and the supernatant was saved. This extraction step was repeated 2-3 more times until the supernatant appeared clear. Approximately 5 ml of chloroform was added to remove trace amounts of phenol. RNA was precipitated from the combined supernatant fractions by adding a 3-fold volume of ETOH and 1/10 volume of 3M NaOAc (pH5.2) and storing at -20ºC for 1 hour. After transferring to a Corex glass container the RNA fraction was centrifuged at 9,000 RPM for 45 minutes at 4ºC. The pellet was washed with 70% ethanol and spun for 5 minutes at 9,000 RPM at 4ºC. After drying the pellet, the pelleted RNA was dissolved in 0.5 ml RNase free water. The pelleted RNA was dissolved in 0.5 ml RNase free water. The quality and quantity of total RNA was analyzed by denatured formaldehyde gel and spectrophotometer, respectively. The total RNA samples with 3-5µg/ul were sent to Genome explorations, inc. to do the hybridization.

### C. Hybridization, detection and data output

The preparation of labeled cRNA material was performed as follows. First and second strand cDNA were synthesized from 5-15 µg of total RNA using the SuperScript Double-Stranded cDNA Synthesis Kit (Gibco Life Technologies) and oligo-dT24-T7 (5'-GGC CAG TGA ATT GTA ATA CGA CTC ACT ATA GGG AGG CGG-3') primer according to the manufacturer's instructions.

The cRNA was concurrently synthesized and labeled with biotinylated UTP and CTP by in vitro transcription using the T7 promoter coupled double stranded cDNA as template and the T7 RNA Transcript Labeling Kit (ENZO Diagnostics Inc.). Briefly, double stranded cDNA synthesized from the previous steps were washed twice with 70% ethanol and resuspended in 22 µl Rnase-free H2O. The cDNA was incubated with 4 µl of 10X each Reaction Buffer, Biotin Labeled Ribonucleotides, DTT, Rnase Inhibitor Mix and 2 µl 20X T7 RNA Polymerase for 5 hr at 37ºC. The labeled RNA was separated from unincorporated ribonucleotides by passing through a CHROMA SPIN-100 column (Clontech) and precipitated at -20ºC for 1 hr to overnight.

Oligonucleotide array hybridization and analysis were performed as follows. The cRNA pellet was resuspended in 10 µl Rnase-free H2O and 10.0 µg was fragmented by heat and ion-mediated hydrolysis at 95ºC for 35 mins in 200 mM Tris-acetate, pH 8.1, 500 mM KOAc, 150 mM MgOAc. The fragmented cRNA was hybridized for 16hr at 45ºC to HG_U95Av2 oligonucleotide arrays (Affymetrix) containing ~12,500 full length annotated genes together with additional probe sets designed to represent EST sequences. Arrays were washed at 25ºC with 6 X SSPE (0.9M NaCl, 60 mMNaH2PO4, 6 mM EDTA + 0.01% Tween 20) followed by a stringent wash at 50ºC with 100 mM MES, 0.1M [Na+], 0.01% Tween 20. The arrays were stained with phycoerythrein conjugated streptavidin (Molecular Probes) and the fluorescence intensities were determined using a laser confocal scanner (Hewlett-Packard). The scanned images were analyzed using Microarray software (Affymetrix). Sample loading and variations in staining were standardized by scaling the average of the fluorescent intensities of all genes on an array to constant target intensity (250) for all arrays used. Data Analysis was conducted using Microarray Suite 5.0 (Affymetrix) following user guidelines. The signal intensity for each gene was calculated as the average intensity difference, represented by [Σ(PM - MM)/(number of probe pairs)], where PM and MM denote perfect-match and mismatch probes.

### D. Data Analysis and results

Twelve sets of hybridizations were successful as evidenced by the Expression Report generated using detection instruments from Genome Explorations. The main parameters on the report included Noise, Scale factor, background, total probe sets, number and percentage of present and absent probe sets, signal intensity of housekeeping controls. The data was subsequently analyzed and presented using software GCOS in combination of other Microsoft software. Signal comparison between treatment pairs was analyzed. Overall data for all respective probes corresponding to genes and fragments of each different treatment including replications were compiled and compiled expression data such as call of the changes and signal log 2 ratio changes were analyzed.

A typical application of GeneChip technology is finding genes that are differentially expressed in different tissues. In the present application, genetic expression variations caused by ethylene treatment were determined for pairs of converter and nonconverter tobacco lines that included a 4407-25/4407-33 burley variety, PBLB01/178 burley variety, and a NL Madole/181 dark variety. These analyses detected only those genes whose expression is significantly altered due to biological variation. These analyses employed the Fold change (signal ratio) as a major criterion to identify induced genes. Other parameters, such as signal intensity, present/absent call, were also taken into consideration.

After analyzing the data for expression differences in converter and nonconverter pairs of samples for approximately 400 genes, the results based on the signal intensities showed that only two genes, D121-AA8 and D120-AH4, had reproducible induction in ethylene treated converter lines versus non-converter lines. To illustrate the differential expression of these genes, the data was represented as follows. As shown in Table V, the signal of a gene in a converter line, for example, burley tobacco variety, 4407-33, was determined as ratio to the signal of a related nonconverter isogenic line, 4407-25. Without ethylene treatment, the ratio of converter to nonconverter signals for all genes approached 1.00. Upon ethylene treatment, two genes, D121-AA8 and D120-AH4, were induced in converter lines relative to non-converter line as determined by three independent analyses using isogenic burley lines. These genes have very high homology to each other, approximately 99.8% or greater nucleic acid sequence homology. As depicted in Table V, their relative hybridization signals in converter varieties ranged from approximately 2 to 12 fold higher in converter lines than the signals in their non-converter counterparts. In comparison, two actin-like control clones, internal controls, were found not to be induced in converter lines based on their normalized ratios. In addition, a fragment (D35-BG11), whose sequence in coding region is entirely contained in both D121-AA8 and D120-AH4 genes, was highly induced in the same samples of paired isogenic converter and nonconverter lines. Another isogenic pair of burley tobacco varieties, PBLB01 and 178, was shown to have the same genes, D121-AA8 and D120-AH4, induced in converter samples under ethylene induction. Furthermore, D121-AA8 and D120-AH4 genes were preferentially induced in converter lines of isogenic dark tobacco pairs, NL Madole and 181, demonstrating that ethylene induction of these genes in converter lines was not limited to burley tobacco varieties. In all cases, the D35-BG11 fragment was the most highly induced in converter relative to nonconverter paired lines.

**Table V: A Comparison of Clone Induction in Ethylene Treated Converter and Non-Converter Lines**

| Clones | No Treatment | Ethylene Treated Burley Exp 1 | | Ethylene Treated Burley Exp 2 | | Ethylene Treated Burley Exp 3 | | Ethylene Treated Dark | |
|---|---|---|---|---|---|---|---|---|---|
| *Induced* | 33:25 Ratio | 33:25 Ratio | Et:No Ratio | 33:25 Ratio | Et:No Ratio | 33:25 Ratio | Et:No Ratio | 181:NL Ratio | Et:No Ratio |
| D121-AA8 | 1.03 | 2.20 | 2.14 | 13.25 | 12.90 | 5.31 | 5.15 | 17.06 | 16.60 |
| D120-AH4 | 1.44 | 2.74 | 1.90 | 18.33 | 12.74 | 4.13 | 2.87 | 11.76 | 8.17 |

| *Control* | | | | | | | | | |
|---|---|---|---|---|---|---|---|---|---|
| Actin-Like I | 1.18 | 1.17 | 0.99 | 0.88 | 0.74 | 0.88 | 0.73 | 1.20 | 1.02 |
| Actin-Like I | 1.09 | 1.23 | 1.12 | 0.89 | 0.81 | 1.18 | 0.11 | 1.02 | 0.93 |

### EXAMPLE 15: CLONING RELATD D35-BG11 FULL LENGTH GENES

GeneChip hybridization was based on 3' reverse transcription (cRNA). The probes were synthesized on GeneChip were chosen from the 3' end of the genes (in the downstream 1000 nucleotide region). Therefore, in order to obtain all the possible variations of D121-AA8 and D120-AH4 clones, additional cloning was performed from the tobacco cDNA library using 5' sequences.

The full length genes were cloned from cDNA library constructed from 4407-33 ethylene treated tissue as described in Example 5. The Polymerase Chain Reaction method was used as follows. The reverse primers were designed based on the 3' sequence (including part of untranslated region) the D121-AA8 gene. The primer of D121-p2 5'-AGC AAG ATG ATC TTA GGT TTT AA-3' and D121-R-2 5'-can GCA AGA TGA TCT TAG GTT TTA ATA AAG CTC AGG T-3'. The T3 primer (5'CAA TTA ACC CTC ACT AAA GGG 3'), located in upstream of the inserts in the plasmid, was used as forward primer The generated PCR products were subjected to agarose electrophoresis and the corresponding bands of high molecular weight were excised, purified, cloned and sequenced. The methods for cloning and sequencing were described in Example 4. Nine novel clones were sequenced and identified as D425-AB10, D425-AB11, D425-AC9, D425-AC10, D425-AC11, D425-AG11, D425-AH7, D425-AH11, and D427-AA5. Each of the clones was observed to have 99% or greater nucleic acid sequence homology with clones D121-AA8 and D120-AH4.

### EXAMPLE 16: ETHYLENE INDUCTION OF MICROSOMAL NICOTINE DEMETHYLASE IN TOBACCO CONVERTER LINES

Biochemical analyses of demethylase enzymatic activity in microsomal enriched fractions of ethylene treated and non-treated pairs of converter and non-converter tobacco lines was performed as follows.

### A. Preparation of Microsomes

Microsomes were isolated at 4°C. Tobacco leaves were extracted in a buffer consisting of 50 mM N-(2-hydrooxyethyl) piperazine-N'-(2-ethanesulfonic acid) (HEPES), pH 7.5, 3 mM DL-Dithiothreitol (DTT) and Protease Inhibitor Cocktail (Roche) at 1 tablet/50 ml. The crude extract was filtered through four layers of cheesecloth to remove undisrupted tissue, and the filtrate was centrifuged for 20 min at 20,000 x g to remove cellular debris. The supernatant was subjected to ultracentrifugation at 100,000 x g for 60 min and the resultant pellet contained the microsomal fraction. The microsomal fraction was suspended in the extraction buffer and applied to an ultracentrifugation step where a discontinuous sucrose gradient of 0.5 M sucrose in the extraction buffer was used. The purified microsomes were resuspended in the extraction buffer supplemented with 10% (w/v) glycerol as cryoprotectant. Microsomal preparations were stored in a liquid nitrogen freezer until use.

### B. Protein Concentration Determination

Microsomal proteins were precipitated with 10% Trichloroacetic Acid (TCA) (w/v) in acetone, and the protein concentrations of microsomes were determined using RC DC Protein Assay Kit (BIO-RAD) following the manufacturer's protocol.

### 3. Nicotine Demethylase Activity Assay

DL-Nicotine (Pyrrolidine-2-¹⁴C) was obtained from Moravek Biochemicals and had a specific activity of 54 mCi/mmol. Chlorpromazine (CPZ) and oxidized cytochrome c (cyt. C), both P450 inhibitors, were purchased from Sigma. Reduced form of nicotinamide adenine dinucleotide phosphate (NADPH) is the typical electron donor for cytochrome P450 via the NADPH:cytochrome P450 reductase. NADPH was omitted for control incubation. Routine enzyme assay consisted of microsomal proteins (around 2 mg/ml), 6 mM NADPH, 55 µM ¹⁴C labeled nicotine. The concentration of CPZ and Cyt. C, when used, was 1 mM and 100 µM, respectively. The reaction was carried at 25°C for 1 hour and was stopped with addition of 300 µl methanol to each 25 µl reaction mixture. After spinning, 20 µl of the methanol extract was separated with a reverse-phase High Performance Liquid Chromatography (HPLC) system (Agilent) using an Inertsil ODS-3 3µ (150 x 4.6 mm) column from Varian. The isocratic mobile phase was the mixture of methanol and 50 mM potassium phosphate buffer, pH 6.25, with ratio of 60:40 (v/v) and the flow rate was 1 ml/min. The nornicotine peak, as determined by comparison with authentic non-labeled nornicotine, was collected and subjected to 2900 tri-carb Liquid Scintillation Counter (LSC) (Perkin Elmer) for quantification. The activity of nicotine demethylase is calculated based on the production of ¹⁴C labeled nornicotine over 1 hour incubation.

Samples were obtained from pairs of Burley converter (line 4407-33) and non-converter (line 4407-25) tobacco lines that were ethylene treated or not. All untreated samples did not have any detectable microsomal nicotine demethylase activity. In contrast, microsomal samples obtained from ethylene treated converter lines were found to contain significant levels of nicotine demethylase activity. The nicotine demethylase activity was shown to be inhibited by P450 specific inhibitors demonstrating the demethylase activity was consistent to a P450 microsomal derived enzyme. A typical set of enzyme assay results obtained for the burley converter tobacco line is shown in the Table VI. In contrast, sample derived from ethylene treated nonconverter tobacco did not contain any nicotine demethylase activity. These results demonstrated that nicotine demethylase activity was induced upon treatment with ethylene in converter lines but not in the corresponding isogenic nonconverter line. Similar results were obtained for an isogenic dark tobacco variety pair, where microsomal nicotine demethylase activity was induced in converter lines and not detectable in nonconverter paired lines. Together these experiments demonstrated that microsomal nicotine demethylase activity is induced upon ethylene treatment in converter lines while not in paired isogenic nonconverter lines. Those genes that are P450 derived genes and are preferentially induced in converter lines relative to paired non-converter lines are candidate genes to encode the nicotine demethylase enzyme.

**Table VI: DEMETHYLASE ACTIVITY IN MICROSOMES OF ETHYLENE INDUCED BURLEY CONVERTER AND NON CONVERTER LINES**

| Sample | microsomes | Microsomes + 1 mM chlor-promazine | Microsomes + with 100 µM cytochrome C | Microsomes -NADPH |
|---|---|---|---|---|
| Converter | 0.6 ± 0.05 pkat / mg | 0.01 ± 0.01 pkat / mg | 0.03 ± 0.05 pkat / mg | 0.03 ± 0.04 pkat / mg |
| Non-Converter | Not Detected | Not Detected | Not Detected | Not Detected |

## Claims

1. An isolated nucleic acid molecule from Nicotiana wherein said nucleic acid molecule comprises the nucleic acid sequence of SEQ ID NO:356.

2. A transgenic tobacco plant, wherein said transgenic tobacco plant comprises the nucleic acid molecule of claim 1.

3. The transgenic tobacco plant of claim 2, wherein said transgenic tobacco plant is a *Nicotiana tabacum* tobacco plant.

4. A method of producing a transgenic tobacco plant, wherein said method comprises the steps of:
(i) operably linking the nucleic acid molecule of claim 1 with a promoter functional in said tobacco plant to create a plant transformation vector;
(ii) transforming said tobacco plant with said plant transformation vector of step (i);
(iii) selecting a plant cell transformed with said transformation vector; and
(iv) regenerating a transgenic tobacco plant from said transformed plant cell.

5. The method of claim 4, wherein said nucleic acid molecule is in an antisense orientation.

6. The method of claim 4, wherein said nucleic acid molecule is in a sense orientation.

7. The method of any of claims 4 to 6, wherein said nucleic acid molecule is expressed as a double stranded RNA molecule.

8. The method of claim 7, wherein said nucleic acid molecule is about 15 to 25 nucleotides in length.

9. The method of any of claims 4 to 8, wherein said transgenic tobacco plant is a *Nicotiana tabacum* tobacco plant.

10. A method of selecting a tobacco plant containing a nucleic acid molecule, wherein said tobacco plant is analyzed for the presence of the nucleic acid sequence of SEQ ID NO:356.

11. The method of selecting a tobacco plant of claim 10, wherein said plant is analyzed by DNA hybridization.

12. The method of selecting a tobacco plant of claim 11, wherein said DNA hybridization is Southern blot analysis.

13. The method of selecting a tobacco plant of claim 11, wherein said DNA hybridization is northern blot analysis.

14. The method of selecting a tobacco plant of claim 10, wherein said tobacco plant is analyzed by PCR detection.

15. The method of any of claims 10 to 14, wherein said tobacco plant is a *Nicotiana tabacum* tobacco plant.

16. A method of decreasing nornicotine levels in a tobacco plant, wherein said method comprises the steps of:
(i) operably linking the nucleic acid molecule of claim 1 with a promoter functional in a tobacco plant to create a plant transformation vector;
(ii) transforming said tobacco plant with said plant transformation vector of step (i);
(iii) selecting a plant cell transformed with said transformation vector; and
(iv) regenerating a transgenic tobacco plant from said transformed plant cell.

17. The method of claim 16, wherein said nucleic acid molecule is in an antisense orientation.

18. The method of claim 16, wherein said nucleic acid molecule is in a sense orientation.

19. The method of any of claims 16 to 18, wherein said nucleic acid molecule is expressed as a double stranded RNA molecule.

20. The method of any of claims 16 to 19, wherein said transgenic tobacco plant is a *Nicotiana tabacum* tobacco plant.

## Patentansprüche

1. Isoliertes Nukleinsäuremolekül aus Nicotiana, wobei das Nukleinsäuremolekül die Nukleinsäuresequenz von SEQ ID NO: 356 umfasst.

2. Transgene Tabakpflanze, wobei die transgene Tabakpflanze das Nukleinsäuremolekül gemäß Anspruch 1 umfasst.

3. Transgene Tabakpflanze nach Anspruch 2, wobei die transgene Tabakpflanze eine *Nicotiana tabacum*-Tabakpflanze ist.

4. Verfahren zum Herstellen einer transgenen Tabakpflanze, wobei das Verfahren die Schritte umfasst:
(i) funktionsfähiges Verknüpfen des Nukleinsäuremoleküls gemäß Anspruch 1 mit einem in der Tabakpflanze funktionsfähigen Promotor, um einen Pflanzen-Transformationsvektor zu erschaffen;
(ii) Transformieren der Tabakpflanze mit dem Pflanzen-Transformationsvektor von Schritt (i);
(iii) Auswählen einer mit dem Transformationsvektor transformierten Pflanzenzelle; und
(iv) Regenerieren einer transgenen Tabakpflanze aus der transformierten Pflanzenzelle.

5. Verfahren nach Anspruch 4, wobei das Nukleinsäuremolekül in einer Antisense-Orientierung ist.

6. Verfahren nach Anspruch 4, wobei das Nukleinsäuremolekül in einer Sense-Orientierung ist.

7. Verfahren nach einem beliebigen der Ansprüche 4 bis 6, wobei das Nukleinsäuremolekül als ein doppelsträngiges RNA-Molekül exprimiert wird.

8. Verfahren nach Anspruch 7, wobei das Nukleinsäuremolekül etwa 15 bis 25 Nukleotide an Länge aufweist.

9. Verfahren nach einem beliebigen der Ansprüche 4 bis 8, wobei die transgene Tabakpflanze eine *Nicotiana tabacum*-Tabakpflanze ist.

10. Verfahren zum Auswählen einer ein Nukleinsäuremolekül enthaltenden Tabakpflanze, wobei die Tabakpflanze auf die Gegenwart der Nukleinsäuresequenz von SEQ ID NO: 356 analysiert wird.

11. Verfahren zum Auswählen einer Tabakpflanze nach Anspruch 10, wobei die Pflanze durch DNA-Hybridisierung analysiert wird.

12. Verfahren zum Auswählen einer Tabakpflanze nach Anspruch 11, wobei die DNA-Hybridisierung Southern-Blot-Analyse ist.

13. Verfahren zum Auswählen einer Tabakpflanze nach Anspruch 11, wobei die DNA-Hybridisierung Northern-Blot-Analyse ist.

14. Verfahren zum Auswählen einer Tabakpflanze nach Anspruch 10, wobei die Tabakpflanze durch PCR-Nachweis analysiert wird.

15. Verfahren nach einem beliebigen der Ansprüche 10 bis 14, wobei die Tabakpflanze eine *Nicotiana tabacum*-Tabakpflanze ist.

16. Verfahren zum Verringern von Nornicotin-Spiegeln in einer Tabakpflanze, wobei das Verfahren die Schritte umfasst:
(i) funktionsfähiges Verknüpfen des Nukleinsäuremoleküls gemäß Anspruch 1 mit einem in einer Tabakpflanze funktionsfähigen Promotor, um einen Pflanzen-Transformationsvektor zu erschaffen;
(ii) Transformieren der Tabakpflanze mit dem Pflanzen-Transformationsvektor von Schritt (i);
(iii) Auswählen einer mit dem Transformationsvektor transformierten Pflanzenzelle; und
(iv) Regenerieren einer transgenen Tabakpflanze aus der transformierten Pflanzenzelle.

17. Verfahren nach Anspruch 16, wobei das Nukleinsäuremolekül in einer Antisense-Orientierung ist.

18. Verfahren nach Anspruch 16, wobei das Nukleinsäuremolekül in einer Sense-Orientierung ist.

19. Verfahren nach einem beliebigen der Ansprüche 16 bis 18, wobei das Nukleinsäuremolekül als ein doppelsträngiges RNA-Molekül exprimiert wird.

20. Verfahren nach einem beliebigen der Ansprüche 16 bis 19, wobei die transgene Tabakpflanze eine *Nicotiana tabacum*-Tabakpflanze ist.

## Revendications

1. Molécule d'acide nucléique isolée de Nicotiana dans laquelle ladite molécule d'acide nucléique comprend la séquence d'acide nucléique de SEQ ID NO: 356.

2. Plant de tabac transgénique, dans lequel ledit plant de tabac transgénique comprend la molécule d'acide nucléique selon la revendication 1.

3. Plant de tabac transgénique selon la revendication 2, dans lequel ledit plant de tabac transgénique est un plant de tabac *Nicotiana tabacum..*

4. Procédé de production d'un plant de tabac transgénique, dans lequel ledit procédé comprend les étapes de :
(i) liaison de manière fonctionnelle de la molécule d'acide nucléique selon la revendication 1 avec un promoteur fonctionnel dans ledit plant de tabac pour créer un vecteur de transformation végétale ;
(ii) transformation dudit plant du tabac avec ledit vecteur de transformation végétale de l'étape (i) ;
(iii) sélection d'une cellule végétale transformée avec ledit vecteur de transformation ; et
(iv) régénération d'un plant de tabac transgénique à partir de ladite cellule végétale transformée.

5. Procédé selon la revendication 4, dans lequel ladite molécule d'acide nucléique est dans une orientation antisens.

6. Procédé selon la revendication 4, dans lequel ladite molécule d'acide nucléique est dans une orientation sens.

7. Procédé selon l'une quelconque des revendications 4 à 6, dans lequel ladite molécule d'acide nucléique est exprimée sous forme d'une molécule d'ARN double brin.

8. Procédé selon la revendication 7, dans lequel ladite molécule d'acide nucléique est d'environ 15 à 25 nucléotides de long.

9. Procédé selon l'une quelconque des revendications 4 à 8, dans lequel ledit plant de tabac transgénique est un plant de tabac *Nicotiana tabacum.*

10. Procédé de sélection d'un plant de tabac contenant une molécule d'acide nucléique, dans lequel ledit plant de tabac est analysé pour la présence de la séquence d'acide nucléique de SEQ ID NO: 356..

11. Procédé de sélection d'un plant de tabac selon la revendication 10, dans lequel ledit plant est analysé par hybridation d'ADN.

12. Procédé de sélection d'un plant de tabac selon la revendication 11, dans lequel ladite hybridation d'ADN est une analyse par transfert de southern.

13. Procédé de sélection d'un plant de tabac selon la revendication 11, dans lequel ladite hybridation d'ADN est une analyse par transfert de northem.

14. Procédé de sélection d'un plant de tabac selon la revendication 10, dans lequel le plant de tabac est analysé par détection par PCR.

15. Procédé selon l'une quelconque des revendications 10 à 14, dans lequel ledit plant de tabac est un plant de tabac *Nicotiana tabacum.*

16. Procédé de réduction des taux de nornicotine dans un plant de tabac, dans lequel ledit procédé comprend les étapes de :
(i) liaison de manière fonctionnelle de la molécule d'acide nucléique selon la revendication 1 avec un promoteur fonctionnel dans un plant de tabac pour créer un vecteur de transformation végétale ;
(ii) transformation dudit plant de tabac avec ledit vecteur de transformation végétale de l'étape (i) ;
(iii) sélection d'une cellule végétale transformée avec ledit vecteur de transformation ; et
(iv) régénération d'un plant de tabac transgénique à partir de ladite cellule végétale transformée.

17. Procédé selon la revendication 16, dans lequel ladite molécule d'acide nucléique est dans une orientation antisens.

18. Procédé selon la revendication 16, dans lequel ladite molécule d'acide nucléique est dans une orientation sens.

19. Procédé selon l'une quelconque des revendications 16 à 18, dans lequel ladite molécule d'acide nucléique est exprimée sous forme d'une molécule d'ARN double brin.

20. Procédé selon l'une quelconque des revendications 16 à 19, dans lequel le plant de tabac transgénique est un plant de tabac *Nicotiana tabacum.*
